(19) Europäisches Patentamt
European Patent Office
Office européen des brevets

(11) EP 1 350 507 A1

# (12) EUROPEAN PATENT APPLICATION

(43) Date of publication:
**08.10.2003 Bulletin 2003/41**

(21) Application number: **02076316.5**

(22) Date of filing: **04.04.2002**

(51) Int Cl.[7]: **A61K 9/127**, A61K 9/107, A61K 9/50, A61K 47/18, A61K 47/22

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Applied NanoSystems B.V.**
**9711 BC Groningen (NL)**

(72) Inventors:
- **Friesen, Robert Heinz Edward**
  **9753 CB Haren (NL)**
- **Meijberg, Jan Willem**
  **9742 KB Groningen (NL)**
- **Leenhouts, Cornelis Johannes**
  **9753 KX Haren (NL)**
- **Hektor, Harm Jan**
  **9725 LD Groningen (NL)**
- **Moll, Gert Nikolaas**
  **9725 LJ Groningen (NL)**
- **Hulst, Anthony Jacques Ronald Lambert**
  **9722 JK Groningen (NL)**
- **van Esch, Johannes Henricus**
  **9728 XC Groningen (NL)**
- **Heeres, André**
  **9723 CG Groningen (NL)**
- **Robillard, George Thomas**
  **9801 BB Zuidhorn (NL)**

(74) Representative: **Prins, Adrianus Willem et al**
**Vereenigde,**
**Nieuwe Parklaan 97**
**2587 BN Den Haag (NL)**

## (54) Delivery of a substance to a pre-determinated site

(57) The invention is concerned with delivery vehicles for delivering a substance of interest to a predetermined site, said vehicle comprising said substance and a means for inducing availability of at least one compartment of said vehicle toward the exterior, thereby allowing access of said substance to the exterior of said vehicle at said predetermined site. The invention is further concerned with uses of said vehicle and methods for preparing it.

EP 1 350 507 A1

## Description

The invention relates to the field of delivery of substances. The invention is useful in several areas among which some of the more important ones are the field of health, medicine, agriculture and cosmetic.

Many substances are delivered to sites of interest to allow their action at said site. Applications of substance delivery vary widely. Examples of such applications range from pharmacological delivery of drugs, to the delivery of substances for pest control and plant growth, to cosmetic reasons. Delivery of substances to sites of interest is so entangled in our daily practice that it is almost impossible to give a complete listing of all possible applications.

A lot of substances have properties that are far from ideal. Substances are therefore typically not delivered in an essentially pure form. Typically substances are formulated in compositions. Compositions are formulated, for instance, to allow for appropriate dosage, delivery or application of the substance of interest.

Various problems with the use of substances can be overcome by appropriately formulating the composition containing the substance of interest. However, with the advent of technology more, and more sophisticated applications of drug delivery come into focus.

The present invention provides means and methods for broadening the use of substances even further. The present invention allows control over the availability of the substance at the site of interest. Through this control another level of accuracy and predictability is introduced which can be utilized to increase the utility of substances to be delivered and to increase the number of substances that can be used for a certain application. The problems and solutions provided by the present invention will be exemplified predominantly using medical and general health examples. However, the invention is by no means limited to these fields.

In the medical field, delivery is typically used to provide an individual with a drug or the pre-cursor thereof. Many drugs, either in the clinic or in development, have properties which limit their applicability. They may have poor solubility, rapid metabolism, instability under physiological conditions or unfavorable biodistribution leading to, for instance, toxicities. One attempt at achieving a solution to these problems has been to associate the drugs with a variety of drug carriers such as liposomes. Liposomes, i.e. phospholipid membrane vesicles, have the ability to function as carriers for either water-soluble or lipid-soluble drugs. Liposomal formulation of the drug is typically slowly released from the liposome over several hours to several days (Allen *et.al.* Cancer Research 1992; 52: 2431-9). In these cases the formulation typically is used as a means for obtaining slow release of the substance. Though this may be of great help in some instances, it is still not optimal. Though slow release formulations may help to provide a more or less continuous source of substance to be made bioavailable, they do nothing with respect to making the substance available at the site where the action of the drug is desired.

On the other hand there are many methods for delivering drugs of interest to a predetermined site. However, a problem with current methods is that it is difficult to control the availability of a compound once delivered. Control over availability is desired for various reasons. For instance where the compound is toxic, degraded or otherwise removed rapidly once available. Thus a control over availability of the substance of interest at the predetermined site is desired. The present invention provides a solution to problems encountered with delivering substances in that it allows control of availability of the substance at the pre-determined site. To this end the invention provides a delivery vehicle for delivering a substance of interest to a predetermined site, said vehicle comprising said substance and a means for inducing availability of at least one compartment of said vehicle toward the exterior, thereby allowing access of said substance to the exterior of said vehicle at said predetermined site. Through the capability of inducing availability of at least one compartment of said vehicle at the predetermined site it is possible to control the availability of the substance at the site where activity of the substance is desired. With a method of the invention it is possible to at least concentrate the substance in the area of the predetermined site. The substance may be transported from one predetermined site to another site, for instance by a means of fluid transport in the body. Such transport will generally also encompass dilution of the substance in at least said transport fluid thereby lowering the effective concentration of said substance, thereby rendering the substance less effective at said other sites. For instance in the case of substances that are toxic, the toxic effects can be restricted to the predetermined site, thereby at least limiting the overall toxicity of the substance. However, a vehicle of the invention not only allows availability of the substance at the predetermined site. It also allows for control over the availability. Through the means for inducing availability of at least one compartment of said vehicle toward the exterior of said vehicle it is possible to at least change the availability of the substance at the predetermined site after said vehicle has been delivered to said predetermined site.

*Induction of availability*

Induction of availability of at least one compartment at the predetermined site can be achieved in various ways depending on the nature of the vehicle and the nature of the induction means. Various vehicle formulations are detailed below. Non-limiting examples of vehicles and induction means that can advantageously be used for the present invention are for instance various gel formulations, wherein the gel comprises a means for at least in part inducing

fluidisation of the gel at the predetermined site. Fluidisation can be induced for instance under the influence of a specific pH, salt concentration, temperature and/or radiation at the predetermined site. The means for inducing availability of said compartment in this case being incorporated in specific formulation of the gel composition, for instance in the choice of specific pH, salt concentration or temperature sensitive gels and/or side chain. Gels of various nature can be used in this respect. "Organogelators are small organic compounds that already at low concentrations can from gels with solvents ranging from hexane to water (F.M. Menger, K.L. Caran, *J. Am. Chem. Soc, 2000, 122, 11679;* J.H. Jung, M. Amaike, K. Nakashima, S. Shinkai, *J. Chem. Soc, Perkin Trans. 2, 2001, 1938),* via self-assembly of the organo/hydrogelator through highly specific *non-covalent* interactions (J. van Esch, F. Schoonbeek, M. de Loos, M. Veen, R.M. Kellogg, B.L. Feringa, NATO ASI Series, Kluwer Academic Publishers, 527 (1998) 223; P. Terech, R.G. Weiss, Chem. Rev., 97 (1997) 3133)"

In a preferred embodiment said means for inducing availability of said compartment responds to conditions that can be controlled. For instance, control over the specific pH, salt concentration or temperature at the site of interest allows one to control availability of the substance of interest. In another example said inducing means comprises a light sensitive compound that upon exposure to light undergoes a change in conformation thereby allowing availability of the compartment, for instance by enhancing fluidisation of the gel. The control over the specific light at the predetermined site allows control over the availability of the substance at the site of interest. In yet another embodiment the availability of the compartment is induced by means of the application of an electrical field at the predetermined site. In yet another embodiment said signal-receptor combination comprises radiation in combination with a radiation sensitive receptor. Examples of receptor in this embodiment are, iron-oxide or cobalt alloys. These receptors are sensitive to various kinds of radiation and utilize the energy contained in the radiation to induce availability of the substance. Iron-oxide and cobalt alloys are particularly suited to raise the temperature in the vehicle of the invention as a result of the adsorption of radiation. Increase in heat can be used to for instance fluidize at least part of the vehicle at the predetermined site. The signal for inducing availability of said compartment in this embodiment is radiation. Radiation can be provided to predetermined site in a sufficiently specific fashion to allow preferential induction of availability of said compartment at or near said predetermined site. The mentioned examples are far from exhaustive and serve only to illustrate some preferred embodiments of vehicles of the invention.

For the present invention, induction of availability of said compartment is achieved by inducing opening of at least one compartment toward the exterior of said vehicle. Inducing opening can be achieved using various means for instance by inducing the generation of a physical opening in a compartment of the vehicle. This embodiment will be discussed in more detail elsewhere in this document.

Induction of availability and/or opening of a compartment toward the exterior is useful for allowing the entrapped substance to passage out of the vehicle. However, it is also useful for allowing compounds to enter the vehicle and associate with the substance. In this way vehicles of the invention can be used to take up desired compound at the predetermined site and discontinue their availability at the predetermined site. Thus vehicles of the invention may make the substance specifically available to the predetermined site by allowing passage of said substance outside the vehicle or it may specifically allow uptake of compounds at the predetermined site by inducing availability of the substance at said site. Preferably, said induction allows passage of said substance to the exterior of said vehicle.

An inducing means of the invention comprises both an effector, capable of making the compartment available and a signal with which the effector can be switched (activated) into a situation wherein said compartment is available to the exterior of the vehicle. It is clear that an effector must be able to respond directly or indirectly to the provision of a signal. The response can be any type of response that makes the compartment available. For instance, it may be that indeed a physical opening of a compartment of the vehicle is induced. However, it is also possible that the effector induces availability in a different way, for instance by fluidisation of (a compartment of) the vehicle.

*Suitable effectors.*

Suitable effectors allow for induction of availability of the compartment in response to a signal. In a preferred embodiment an effector of the invention comprises a radiation responsive molecule. Preferably said radiation sensitive molecule comprises a light responsive molecule. A light responsive molecule of the invention is a molecule that can assume a different conformation upon exposure to light. The difference in conformation is utilized to allow a physical change in the vehicle of the invention wherein said physical change induces the availability of at least one compartment of said vehicle toward the exterior. Preferred radiation sensitive effectors are light switchable gelling or thickening molecules and light switchable molecules that are part of a film. Non-limiting examples of the latter are light-switchable lipids and light-switchable channel proteins.

In another embodiment of the invention an effector comprises a binding molecule that upon binding induces the vehicle to induce availability of said compartment toward the exterior of said vehicle. Preferably to induce release of the substance from said vehicle (for instance by fluidisation). The signal in this case can be the binding event. A binding molecule as an effector may also induce the prolonged presence of the vehicle at the predetermined site

thereby induce availability by conditions at the predetermined site, for instance a lower pH at the site of a solid tumor. A non-limiting example of such a binding molecule is a binding molecule that undergoes a change in conformation under the influence of conditions at the predetermined site wherein said conformation change allows preferential binding of said binding molecule to its binding partner at the predetermined site. An example of such a binding molecule is a pH-sensitive binding molecule. In a preferred embodiment said pH-sensitive binding molecule comprises a pH-sensitive AcmA and/or AcmD protein. The specific pH at which a vehicle of the invention is specifically retained at the site of interest can be tailored. One way of tailoring said vehicle is by manipulating the ratio of AcmA and AcmD in the vehicle. For examples of suitable AcmA and AcmD proteins reference is made to the examples and to WO 99/25836.

In a preferred embodiment of the invention a compartment is induced to become available by inducing opening of at least one compartment in said vehicle thereby allowing access of said substance to the exterior of said vehicle. In this preferred embodiment of the invention said vehicle comprises a film wherein the continuity of the film can be controlled by providing a signal. In a preferred embodiment said opening is achieved by inducing opening of a pore in said film. In a preferred embodiment said film comprises an effector molecule capable of forming a pore. To this end it is preferred that said effector molecule comprises a proteinaceous channel that allows availability by forming a pore through which compartment is made available toward the exterior of said vehicle. The proteinaceous channel can be any proteinaceous channel that allows induced opening of at least one compartment of said vehicle. Preferably, said proteinaceous channel comprises a solute channel. A solute channel is capable of allowing passage of ions and small hydrophilic molecules. Preferably, said proteinaceous channel comprises an ion channel. More preferably, said proteinaceous channel comprises a mechanosensitive channel. Preferably, a mechanosensitive channel of large conductance (MscL) or a functional equivalent thereof. In nature MscL allows bacteria to rapidly adapt to a sudden change in environmental conditions such as osmolarity. The MscL channel opens in response to increases in membrane tension, which allows for the efflux of cytoplasmic constituents. By allowing passage of said constituents to the outside of the prokaryote, it is able to reduce the damage that the sudden change in environmental conditions would have otherwise inflicted. The genes encoding MscL homologues from various prokaryotes are cloned (Moe, P.C., Blount, P. and Kung, C. (1998) *Mol. Microbiol*. **28**, 583-592). Nucleic acid and amino acid sequences are available and have been used to obtain heterologous (over)-expression of several MscL (Moe, P.C., Blount, P. and Kung, C. (1998) *Mol. Microbiol*. **28**, 583-592). In the present invention vehicles, preferably liposomes, comprising MscL or a functional equivalent thereof are loaded with small hydrophilic molecules whereupon loaded small molecules can be released from said vehicle under activation or opening of the channel. Loading of the lipid vesicle can be accomplished in many ways as long as the small molecules are dissolved in a hydrophilic solvent which is separated from the surrounding hydrophilic solvent by a lipid bilayer. Activation of MscL has been found to be controllable. It is possible to tune the type and relative amount of lipids in the vehicle such that the amount of membrane tension required to activate the channel is altered. Thus depending on the circumstances near cells of selected tissue, the lipid vehicle can be tuned to allow preferential activation of the channel and thus preferential release of said small molecule in the vicinity of said cells of said tissue.

Compositions comprising lipid vehicles have been used in vivo, for instance to enable delivery of nucleic acid or anti-tumor drugs to cells. It has been observed that blood stream administration of such vehicles often leads to uptake of vehicles by cells. Uptake by cells seems to correlate with the charge of the lipid in the vehicle. Uptake is particularly a problem with negatively charged lipid vehicles, these vehicles are very quickly removed from the blood stream by the mononuclear phagocytic system in the liver and the spleen. Although the present invention may be used to facilitate uptake of small molecules by cells, it is preferred that the small molecules are delivered to the outside of cells. In the present invention it has been found that MscL is also active in lipid vehicles that consist of positively and/or neutrally charged lipids. Lipid vehicles comprising said positively and/or neutrally charged lipids are more resistant to uptake by cells of the mononuclear phagocytic system. Lipid vehicles of the invention therefore preferably comprise positively and/or neutrally charged lipids. Such vehicles exhibit improved half-lifes in the bloodstream. Such vehicles demonstrate improved targeting to non-mononuclear phagocytic system cells. The lipid part directed toward the exterior of a lipid vehicle of the invention preferably consists predominantly of positively and/or neutrally charged lipids, thereby nearly completely avoiding cellular uptake through negatively charged lipid and thereby further increasing the bloodstream half life of lipid vehicles of the invention. Apart from increasing the half life of the vehicle in the blood stream, positively and/or neutrally charged lipids can also be used to alter the amount of added pressure needed to activate the channel in the vehicle. Vehicles of the invention wherein the outwardly directed lipid part of a lipid vehicle consists predominantly of positively and/or neutrally charged lipids, postpone the rapid cellular uptake as seen for vehicles wherein the outwardly directed part consists of negatively charged lipid. Postponed uptake through the mononuclear phagocytic system leads to increased circulation times. Apart from this, positively and/or negatively charged lipids can also be used to alter the amount of membrane tension needed to activate the channel.

The signal or event leading to activation of a channel of the invention can also be changed by altering the MscL in the vehicle. Besides the pH sensitive mutants, other mutants are available that have a higher open probability as compared to the wild type MscL from Escherichia coli (Blount, P., Sukharev, S.I., Schroeder, M.J., Nagle, S.K., and Kung, C. (1996) *Proc. Natl. Acad. Sci USA* **93**, 11652-11657; Ou, X., Blount, P., Hoffman, R.J., and Kung C. (1998)

*Proc. Natl. Acad. Sci. USA* **95**, 11471-11475). This property can be used to tune the activation potential of the channel in a method or vehicle of the invention. For instance, it is known that in tumors the pH is very often considerably lower than in the normal tissue surrounding the tumor. Other areas in the body that have a lowered pH are the liver, area's of inflammation and ischemic area's. A lower pH can be used as a trigger for activation of the MscL in a vehicle of the invention. Mutant MscL's are available that activate (open) in response to a pH that is frequently encountered in tumors. One non-limiting example of such a pH-sensitive mutant is the G22H mutant. This mutant exhibits a higher open probability at (low) pH values that are frequently encountered in tumors, as compared to normal pH values of circulating blood (Yoshimura, K., Batiza, A., Schroeder, M., Blount P., and Kung, C. (1999) *Biophys. J.* **77**, 1960-1972). Thus in a preferred embodiment of a method or composition of the invention, said mutant allows preferred release of said small molecule in said target tissue.

A small molecule can be any hydrophilic molecule small enough to pass through the pore of a channel of the invention. Preferably said small molecule comprises a diameter of no more than 60 Å, more preferably no more than 50 Å and still more preferably no more than 40 Å. Particularly peptides are preferred for the present invention. Peptides typically have very poor pharmacodynamic properties when injected into the bloodstream. With the present invention it is possible to significantly increase the half life of peptides in the circulation. Moreover, by enabling controlled release of a small molecule with a vehicle of the invention it is also possible to have a relatively high bioavailability of the peptide at the predetermined site, whereas systemically the bioavailability is low or even absent. This also allows for the therapeutic use of molecules that are otherwise too toxic when bioavailable systemically.

Induction of availability of a small molecule by means of a proteinaceous channel in a vehicle of the invention can be achieved in many ways. For instance, by tuning of the composition of the lipid vehicle and/or the use of a mutant MscL it is possible to control how and where release of the small molecule will occur. In a preferred embodiment, activation of said channel is triggered upon the availability of a signal. The signal for activation can for instance be exposure of the vehicle to a certain pH, to light or to a certain temperature. Exposure to the signal can directly or indirectly (through an intermediary signal) lead to the activation of the channel. Preferably, said signal comprises light. There are hydrophobic compounds such as azobenzene phospholipids and related compounds available (Song, X., Perlstein, J., and Whitten, D.G. (1997) *J. Am. Chem. Soc.* **119**, 9144-9159), that mix with the lipids in the vehicle, and that upon exposure to light undergo a structural change such that the gating of the MscL channel can be controlled. It is also possible to insert a photosenstive mutant MscL in the lipid vehicle. Upon exposure to light, a photoreactive molecule conjugated to a specific site of the MscL protein alters conformation thereby controlling the gating of the MscL channel. Activation through light is just one example of an embodiment wherein opening/activation of the channel can be induced by another signal than membrane tension. An alteration in the redox-potential is another non-limiting example. MscL can be made sensitive to the local redox-potential after conjugation of a redox-sensitive molecule, such as a nicotinamide adenine dinucleotide derivative, to a specific site of the MscL protein. Such a redox-sensitive MscL can be (de)activated by changing the redox-potential of the environment. In yet another embodiment said signal comprises an altered pH, preferably said pH is equal or less then 6.5. Recognition of only the open conformation of MscL by a binding molecule is another non-limiting example of an embodiment that gating of the channel can be induced by another signal than membrane tension. Such a binding molecule is preferably an antibody. The binding molecule can for instance be used to preferentially increase the open probability of the channel near target cells. A binding molecule capable of binding MscL in the open state is a preferred embodiment of an effector molecule that upon binding induces the vehicle to the substance available for the exterior at the predetermined site.

A vehicle comprising a proteinaceous channel can also in another way make the substance of interest available at the predetermined site. In one embodiment said vehicle further comprises a targeting means. In combination with a targeting means a vehicle of the invention can be used to make the substance of interest available at the predetermined site. The means for inducing availability can in these cases comprise the targeting means. Targeting is preferably achieved using a binding molecule capable of binding to a target cell at said predetermined site. The binding of the targeting molecule holds the vehicle in place so that with gradual availability of the substance, the substance is still induced to become preferably available at the predetermined site. In one embodiment said targeting means comprises PrtP or a functional part, derivative and/or analogue thereof. Preferably, said targeting means is capable of binding preferably to said target at the predetermined site. This embodiment is probably better explained with an example. A preferred targeting means of the invention comprises AcmA or AcmD type protein anchors. AcmA type protein anchors can be made to bind its target preferentially in a pH dependent fashion. A vehicle of the invention provided with AcmA , is capable of inducible binding as a result of the signal pH. For examples of suitable AcmA and AcmD proteins reference is made to the examples and to WO 99/25836. In cases where the predetermined site comprises a telltale pH, substance can be made preferentially available to the predetermined site. Preferably availability is induced upon providing the binding molecule capable of binding MscL in the open state. Preferably said means for inducing availability comprises both said AcmA protein and said binding molecule capable of binding MscL in the open state. In another embodiment a bi-specific antibody comprising the above mentioned specificity for the open state and a specificity for a target cell can be used to accumulate open vehicles near target cells.

Another example of a signal that triggers activation of a MscL is local anesthetics (Martinac, B., Adler, J., and Kung, C. (1990) Nature 348, 261-263). Local anesthetics most probably work to activate the channel through their incorporation in the lipid bilayer, which changes the bilayer properties.

There are very likely many substances that can cause activation of the channels. One example to note in this context is [2-(trimethylammonium)ethyl]methanethiosulfonate bromide (MTSES). This compound is capable of associating with MscL mutant G22C. Whereas a hydrophobic moiety at this position makes the channel harder to open, a hydrophilic addition at this position helps to overcome the mechanical work required to open the channel. The compound MTSES helps to lower the amount of signal required to activate the channel (Yoshimura, K., Batiza, A. and Kung, C., 2001, Biophys.J. 80: 2198-2206). Various polar and non-polar variants of MTSET exist that can be used depending on whether the channel should be easier or more difficult to activate. It is also possible and for some applications even preferred to change the signal needed for activation of the channel from membrane pressure to another signal. Signals such as light, local anesthetics, pH, temperature, etc., can be used. For instance, through local illumination a circulating lipid vehicle can be triggered to release incorporated molecules only in the illuminated area of the body. This is an important additional advantage of having another signal or an intermediate signal than pressure for activation of the channel. In a preferred embodiment a vehicle of the invention comprises an asymmetrical bilayer. An asymmetrical bilayer is yet another example of a method to tune the lipid vehicle such that the activation of the channel is altered. It seems that the force gating MscL is from the lipid bilayer and amphipaths probably generate this force by differential insertion into the two leaflets (Martinac, B., Adler, J., and Kung, C. (1990) Nature 348, 261-263). In a preferred embodiment, a signal required for activation is provided through an intermediate. The intermediate is here capable of transforming the given signal into a pressure signal thereby allowing, if sufficient, the opening of the channel.

In one aspect the invention provides a composition comprising a lipid vehicle comprising a proteinaceous channel and a small hydrophilic molecule, wherein said lipid vehicle and/or said proteinaceous channel is formulated such that said proteinaceous channel is in the open state in the vicinity of a target cell. Preferably, said proteinaceous channel comprises an MscL or functional part, derivative and/or analogue thereof. In a preferred aspect the invention provides a composition comprising a lipid vehicle comprising an MscL or functional part, derivative and/or analogue thereof, wherein said composition is formulated and prepared for use in a human. Preferably said lipid vehicle comprises a small hydrophilic molecule capable of passing through an activated MscL. Preferably, said composition is used in the preparation of a medicament. Preferably said small molecule is intended to be delivered to the outside of a cell in said tissue. A composition as described is of course ideally suited to be used in a method of the invention. Preferably, said MscL is a mutant MscL or a functional part, derivative and/or analogue thereof. A functional part of MscL comprises at least the region that in E.coli comprises residue 4 to 110 (Blount, P., Sukharev, S.I., Schroeder, M.J., Nagle, S.K., and Kung, C. (1996) *Proc. Natl. Acad. Sci USA* **93**, 11652-11657). It is possible to generate MscL proteins that comprise amino-acid substitution(s), insertion(s) and/or deletion(s) compared to the protein found in bacteria. Such derivatives can of course also be used for the present invention provided that the derivative is functional, i.e. comprises the channel activity in kind, not necessarily in amount. The channel activity may, as will be apparent from the description, be inducible by means other than pressure. With activity in kind is meant, the capability of the channel protein to allow passage of a hydrophilic substance from one side of the lipid obstruction to the other. The amount of activity, both in the amount of small molecules that may pass per time unit, or the size of the pore through which the small molecule can pass, may differ. A derivative of MscL is also an MscL that comprise more or less or different (post-translational) modifications as compared to the native protein. Other options with mutant or derivative channels would be using MscL with genetically engineered changes in the outside loop, like receptor recognizing domains (e.g. RGD) that upon binding with the receptor undergo conformation changes that induce opening of the channel. One may also add (part of) an antibody to that loop that induces channel opening after ligand binding. An MscL analogue is a molecule comprising the same activity in kind to allow passage of hydrophilic molecules through a lipid obstruction than MscL itself, not necessarily in amount.

In another aspect the invention provides a method for generating a vehicle for delivery of a small hydrophilic molecule to a cell, said method comprising generating in an aqueous fluid, a lipid vehicle comprising a proteinaceous channel, said vehicle formulated such that said proteinaceous channel is in the open state in the vicinity of said cell. Preferably, said proteinaceous channel assumes said open state upon the presence of a signal in the vicinity of said cell. More preferably, said lipid vehicle further comprises said small molecule. A method for the generation of a vehicle as described above can be advantageously used to generate a composition of the invention.

In a preferred embodiment a lipid vehicle of the invention further comprises a non-channel protein. Preferably, said non-channel protein is a binding molecule capable of binding to a binding partner in said tissue thereby enabling at least a prolonged stay of said vehicle in said tissue and/or near a target cell.

In another aspect the invention provides the use of a lipid vehicle comprising an MscL for controlling delivery of a small hydrophilic molecule to a target tissue in a body.

A lipid vehicle of the invention may be used to deliver a small molecule to any part of the body. However, preferably it is used to deliver to tissue with permeable endothelium such as the liver, the spleen, area's of inflammation

or tumor bearing tissues.

A lipid vehicle of the invention can comprise lipid but may also comprise other molecules. Glycolipids or lipids modified in other ways, that maintain the classical bipolarity of a lipid molecule in kind, not necessarily in amount are also called lipids in the present invention. In a preferred embodiment of the invention said lipid vehicle comprises a liposome, more preferably a long circulating liposome. Long circulating liposomes are typically small (150 nm or smaller). Preferably said long circulating liposome comprises neutral lipid. Said long circulation liposome preferably comprises cholesterol with either phosphatidylcholine and PEG or sphingomyelin).

Considering that a vehicle of the invention may comprise a molecule that is regarded as foreign to the human body it is preferred in these cases that said vehicle further comprises a masking group. A masking group at least in part prevents that the immune system of the individual to which said vehicle is administered, to respond to the vehicle. MscL is typically a protein foreign to the human body, it is therefore conceivable that the immune system of a human administered with a vehicle comprising MscL, responds to said MscL either upon first administration or upon repeated administration. To allow at least a partly evasion of the immune system of the host so called masking groups can be attached to the outside of the vehicle of the invention. Preferably, said masking groups comprise PEG.

In a preferred embodiment a vehicle comprising MscL comprises a gel or thickening agent. In a preferred embodiment said gel or thickening agent comprises an anionic polymer that under the influence of an electrical field decondensates and thus results in swelling of the polymer matrix. Preferably said anionic polymer comprises ..... (Qiu, Y. and Park, K., 2001, Advanced Drug Delivery Reviews 53: 321-339) Swelling can be achieved at field strenghts of 2.5V accross a 5µm vehicle which corresponds to a field strength of $5000Vcm^{-1}$. This is a factor of 10 less than the field strength needed to induce leakage of ions through the ion channels. In a confined volume of a vehicle of the invention the swelling is translated into a membrane stress or internal pressure sufficient to induce opening of the MscL or functional equivalent thereof, thereby allowing availability of the compartment toward the exterior of the vehicle at the predetermined site.

*The vehicle*

The vehicle can be generated using various means. For instance, the vehicle may comprise a film that forms a barrier between the interior of the vehicle and the exterior. Alternatively the vehicle can comprise a gel which more or less traps the substance of interest in the interior. Such gels may be continuous or discontinuous. In a preferred embodiment a vehicle of the invention comprises a gel and a film. In this way unintended leakage of the substance from continuous and discontinuous gels can at least in part be reduced.

In a preferred embodiment of the invention said film comprises a membrane. Said membrane preferably generates at least one compartment in said vehicle. In this embodiment said inducing means comprise means to brake up or dissolve or otherwise interrupt the membrane. Creating a discontinuity in the membrane makes the compartment comprising the substance in the interior available to the exterior of the vehicle. The membrane may consist of many different substances. Preferably said membrane comprises lipid. Preferably, said membrane comprises a lipid bilayer.

*Compositions for films*

*Amphiphiles*

In a preferred embodiment the membrane comprises an amphiphile. Amphiphile are capable of self-assembly to form vehicles in a predominantly polar or predominantly apolar environment. Amphiphiles are widely used for generation of membranes. In a preferred embodiment said amphiphile comprises a lipid, in particular a phospholipid. Vehicles comprising such films are generically called liposomes. Thus in a preferred embodiment a vehicle of the invention comprises a liposome or a functional equivalent thereof. Liposomes typically have a size between 50 and 2000nm. For use in human the size is preferable between 50 and 200 nm. A functional equivalent of a liposome comprises a film comprising lipid, in particular phospholipid but with a smaller size, for instance the so-called nanosomes. Nanosomes typically have a size not exceeding 100 nm and are considered to be a functional equivalent of a liposome in the present invention. Typical liposome formulations comprise (Banerjee, R.J., 2001, Biomat. Appli. 16: 3-21).

In one embodiment of the invention the membrane comprises a cationic amphiphile. Since the introduction of the quaternary ammonium containing amphiphile dioleoyloxypropyl trimethyl ammonium choride by Felgner et al (Proc. Natl. Acad. Sci USA, 1987, Vol 84:7413-7417), which in combination with the phospholipid dioleoylphosphatidylethanolamine (DOPE) is commercially available as Lipofectamine™, many more cationic amphiphiles have been developed and marketed. For example, cationic amphiphiles having a pyridinium group, which is an aromatic ring comprising a nitrogen atom, as cationic part, for introducing biologically active compounds into eukaryotic cells have been developed and are disclosed in EP 0755924. (Miller, A.D., 1998, Angew. Chem.Int.Ed.Engl. 37: 1768-1785). A

film comprising a cationic amphiphile is therefore also part of the invention. In a particularly preferred embodiment said cationic amphiphile comprises a cationic amphiphile having an aromatic ring comprising a nitrogen atom according to the following formula (I):

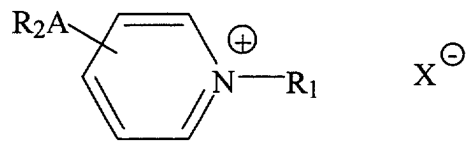

in which:

R1 is selected from the group consisting of:

a branched or linear (C6-C24) carbon chain optionally interrupted by one or more heteroatoms, optionally containing one or more functional groups, optionally containing one or more double or triple carbon-carbon bonds or combinations of double and triple carbon-carbon bonds, optionally being substituted or combinations thereof

and
R3-Ar-A-R2 in which R3 is a C2-C10 carbon chain, Ar is an aromatic or a heteroaromatic ring optionally to which relative to R3 in the ortho-, meta-, or para-position A-R2 is attached,
A is attached to the aromatic ring in the ortho-, meta- or para-position relative to the nitrogen atom in the aromatic ring,
A is selected from the group consisting of $CH_2$, O, S, ,S-S, NH, OC=O, O=CO, SC=O, O=CS, NHC=O, O=CNH, CH=N, N=CH, phosphate, alkylphosphate and phosphonate
R2 represents a branched or linear (C6-C24) carbon chain optionally interrupted by one or more heteroatoms, optionally containing one or more functional groups, optionally containing one or more double or triple carbon-carbon bonds or combinations of double and triple carbon-carbon bonds, optionally being substituted or combinations thereof
$X^-$ is a physiologically acceptable anion.

Cationic amphiphiles according to this embodiment comprise of an aromatic ring to which two carbon chains (R1 and R2) are attached. The aromatic ring comprises a nitrogen atom. One of the two carbon chains (R1) is attached to the nitrogen atom in the ring. The second carbon chain (R2) is attached to the ortho-, meta- or para-position relative to this nitrogen. Both groups R1 and R2 in the formula can be identical but this is not necessary. Preferably A is $CH_2$ and is attached in the para-position relative to the nitrogen atom in the aromatic ring. Preferably, A is OC=O and is attached in the para-position relative to the nitrogen atom in the aromatic ring. Preferred is also a cationic amphiphile wherein A is OC=O and is attached in the meta-position relative to the nitrogen atom in the aromatic ring. In a particularly preferred embodiment R1 is a carbon chain selected from the group consisting of C16, C18, C20 and C22 carbon atoms, optionally containing one or more double or triple carbon-carbon bonds or combinations thereof and R2 is selected from the group consisting of C14, C16 and C18 carbon atoms, optionally containing one or more double or triple carbon-carbon bonds or combinations thereof. In yet another embodiment A is $CH_2$ and is attached in the para-position relative to the nitrogen atom in the aromatic ring and R2 is R4C=OO in which R4 is a C5-C23 carbon chain. Preferably, R1 is a carbon chain selected from the group consisting of C16, C18, C20 and C22 carbon atoms, optionally containing one or more double or triple carbon-carbon bonds or combinations thereof and R2 is selected from the group consisting of C11, C13, C15 and C17 carbon atoms, optionally containing one or more double or triple carbon-carbon bonds or combinations thereof. In a particular preferred embodiment R1 is longer than R2.
In their most elementary form the carbon chains R1 and R2 are linear alkyl chains of 6-24 carbon atoms. Optionally one of the two or both carbon chains comprise one or more unsaturations in the form of double or triple carbon-carbon bonds. Also it is possible that the carbon chains comprise, optionally in combination with one or more unsaturations, one or more heteroatoms in the chain and/or one or more functional groups in the chain and/or substitutions on the chain. It is also possible that the carbon chain is branched. Preferably such branching does not occur on the first six carbon atoms calculated starting from the aromatic ring. Such branching can occur in combination with the presence of unsaturated carbon-carbon bonds and also in combination with the presence of heteroatoms in the

chain and/or with the presence of functional groups and/or in the presence of substitutions.

In one embodiment the carbon chain R1 on the aromatic nitrogen atom comprises an aromatic group. In particular the aromatic group can be a phenyl group. The aromatic group, represented by Ar, in R1 is positioned near the nitrogen atom containing the aromatic ring. Near in this respect means that the carbon chain connecting the nitrogen containing aromatic ring and the aromatic group in R1 is of such a length that "backfolding" of R1 towards the nitrogen atom containing aromatic ring allows alignment of the aromatic group in R1 with the nitrogen atom containing aromatic ring. With the aid of models the skilled person will be able to determine an appropriate length of the chain connecting Ar and the nitrogen containing aromatic ring for backfolding to occur. Backfolding resulting in sufficient alignment is achieved with R3 which is a C2-C10 carbon chain. Optionally attached to the aromatic group in R1 is A-R2 in the ortho-, meta- or para-position relative to R3, in which R2 is defined as above and A as will follow. Another particular embodiment is a cationic amphiphile in which Ar is a heteroaromatic ring. A heteroaromatic ring is an aromatic ring comprising one or more heteroatoms such as O, N and S. In particular Ar is a heteroaromtaic ring which comprises a nitrogen atom. R3 is attached to the nitrogen in this aromatic ring Ar. In the ortho-, meta- or para-position in the aromatic ring A-R2 is attached.

R1 is directly attached to the nitrogen atom in the aromatic ring. R2 is attached to a carbon atom in the aromatic ring via group A. Various ways exist for attaching R2 to an aromatic carbon atom. The skilled person will be able to determine what groups are available for A. Particularly suited as group A for attachment is a group chosen from $CH_2$, O, S, S-S, NH, OC=O, O=CO, SC=O, O=CS, NHC=O, O=CNH, CH=N, N=CH, phosphate, alkylphosphate and phosphonate.

X- represents a physiologically acceptable anion. The cationic amphiphiles of the invention can be used for *in vitro* as well as *in vivo* purposes. In this respect it may vary what anions are physiologically acceptable. The skilled person will be able to determine for what purpose which anion may be suitable. Examples of suitable anions are $Cl^-$, $Br^-$, $I^-$, $HSO_4^-$, $H_2PO_4^-$, $ClO_4^-$ and organic anions such as $CH_3CO_2^-$, $^-O_2CCO_2^-$ and the like.

As mentioned above the R1 and R2 groups in preferred cationic amphiphiles may contain one or more unsaturated carbon-carbon bonds. Also the R1 and R2 groups may contain, in any position, one or more heteroatoms such as O, N and S. Such a heteroatom can be part of a functional group. Thus R1 and R2 may contain, in any position one or more functional groups such as ethers, disulphides, esters, amides, phosphates, imines, amidines and the like. For research or diagnostic or therapeutic purposes R1 or R2 or both may also comprise fluorescent groups, such as fluorescein rhodamine, acridine, diphenylhexatrienepropionic acid and the like in the chain or as substituent attached to the chain or, R1 and/or R2 may comprise or be substituted with radioactive labels. Of particular interest are substituents that can be involved in targeting of cells. For instance ligands for particular receptors on cells or antibodies or parts of antibodies comprising binding domains for a particular epitope at or in the neighbourhood of the site where the incorporated biologically active compound has to exert its activity can be attached to R1 and/or R2. Such targeting substituents can be attached directly or for instance through a spacer. Also functional groups and/or substituents that can be involved in the release from endosomes in cells such as pH labile groups or substituents can be of interest.

Optionally the carbon chains in the vicinity of the nitrogen containing aromatic ring are substituted with groups introducing additional positive charge such as for instance amino groups that are protonated under physiological conditions and trialkylammonium groups. In this respect also substituents which can be involved in hydrogen bonding are considered.

Cationic amphiphiles can be synthesised following known procedures such as described in EP 0755924 and as will be further illustrated in the examples. In short 4-methylpyridine is treated with base and subsequently monoalkylated on the 4-methyl group to introduce R2. Next the nitrogen in the pyridine ring is quaternized with an alkyl halide to introduce R1 followed by ion-exchange to obtain the desired $X^-$ as counterion. However, in order to obtain the desired compounds in acceptable yield and purity the general known method described above is not always satisfactory. Surprisingly, it has been found that cationic amphiphiles that would not be available in an economic acceptable manner using known procedures can be synthesised by applying the microwave technique in the procedure. In particular the attachment of R1 to the nitrogen in the aromatic ring is carried out under microwave conditions. In general this means that the step of attaching a group on the nitrogen in the nitrogen atom containing aromatic ring is carried out in a microwave oven. The attachment reaction is particularly substitution of a halide in the group to be attached. Depending on the specific reaction that is carried out the skilled person will be able to determine the optimal reaction time and settings for the microwave oven. For several cationic amphiphiles according to the invention the reaction time and settings are given in detail in the examples.

*Hydrophobin or functional equivalent thereof.*

In one embodiment said film comprises a hydrophobin. Hydrophobins are capable of forming tight membranous structures that are impermeable to fluid or dissolved molecules. The membranes can be very thin. Hydrophobin membranes are particularly suited in vehicles that also comprise a viscous material and/or a lipid membrane. In these

cases undesired leakage of the substance from the vehicle can be prevented to a large extent. Particularly for vehicles comprising viscous material and/or lipid membranes leakage is a problem. By providing an extra layer comprising of hydrophin or a functional equivalent thereof (the rodlins) such vehicles can be made essentially non-leaky.
In a preferred aspect of this embodiment said film comprises an amphiphile and a hydrophobin and/or rodlin. Vehicles of this preferred embodiment are less prone to release of the substance of interest in the absence of induction of availability. These vehicles are less leaky, thereby allowing more control over the availability of the substance of interest at the predetermined site.

Hydrophobins are a well-defined class of proteins (described in Wessels, 1997 Adv. Microb. Physiol. 38, pp 1-45) capable of self-assembly at a hydrophobic-hydrophilic interface, and having a conserved sequence

$$X_n\text{-}C\text{-}X_{5\text{-}9}\text{-}C\text{-}C\text{-}X_{11\text{-}39}\text{-}C\text{-}X_{8\text{-}23}\text{-}C\text{-}X_{5\text{-}9}\text{-}C\text{-}C\text{-}X_{6\text{-}18}\text{-}C\text{-}X_m$$

wherein X represents any amino acid, and n and m represent an integer. In general, a hydrophobin has a length of up to 125 amino acids. The cysteine residues (C) in the conserved sequence are part of disulfide bridges. In the present invention, the term hydrophobin has a wider meaning to include functionally equivalent proteins, and encompass a group of proteins comprising the sequence or functional parts thereof

$$X_n\text{-}C\text{-}X_{1\text{-}50}\text{-}C\text{-}X_{0\text{-}5}\text{-}C\text{-}X_{1\text{-}100}\text{-}C\text{-}X_{1\text{-}100}\text{-}C\text{-}X_{1\text{-}50}\text{-}C\text{-}X_{0\text{-}5}\text{-}C\text{-}X_{1\text{-}50}\text{-}C\text{-}X_m$$

still displaying the characteristics of self-assembly at a hydrophobic-hydrophilic interface resulting in a protein film. Means and methods for the manipulation, purification and the formation of films comprising hydrophobin are described or can be derived from (Lugones, L.G. et al., 1998, Microbiology 144 (Pt8):2345-2353; Martin, G.G. et al., 2000, Biomacromolecules 1:49-60; Wessels, J.G., 1997, Adv.Microb.Physiol 38:1-45; Wösten, H.A., 2001, Annu.Rev.Microbiol. 55:625-646; Wösten, H.A.B. et al., 1993, The Plant Cell 5:1567-1574). In accordance with the definition of the present invention, self-assembly can be detected by absorbing the protein to Teflon and use Cicular Dichroism to establish the presence of secondary structure (in general α-helix and β-sheet). A functional equivalent of a hydrophobin is a rodlin. Rodlin are proteins with similar properties to hydrophobin but that do not share the same consensus sequence. A typical class of rodlins is described in WO 0174864.

*Compositions for gels*

A vehicle of the invention may comprise a gel, either alone or in combination with a film. Any type of gel may be used for the vehicle of the present invention as long as said vehicle comprises a means for inducing availability of a compartment formed by the gel at the predetermined site. The gel may comprise a so-called organogel, a hydrogel or a combination of both. Preferred examples of such gels are organogels such as (a) amino acid type,b) carbohydrate derived, c) bis urea derivatives, d) steroid derivatives, e) fatty acid derivatives (J. van Esch, F. Schoonbeek, M. de Loos, M. Veen, R.M. Kellogg, B.L. Feringa, NATO ASI Series, Kluwer Academic Publishers, 527 (1998) 223 and P. Terech, R.G. Weiss, Chem.Rev., 97 (1997) 3133) or hydrogels such as a) amino acid based (F.M. Menger, K.L. Caran, *J. Am. Chem. Soc*, 2000, 122, 11679), b) carbohydrate derived (J.H. Jung, M. Amaike, K. Nakashima, S. Shinkai, *J. Chem. Soc, Perkin Trans.* 2, 2001, 1938), c) bis urea derivatives, d) cis,cis 1,3,5-trisubstituted cyclohexane derivatives. Preferably said gel comprises a so-called thermally reversible gelling or thickening agent. In a preferred embodiment a gel is formed from reversible gelling in solvents (typically organic solvents) of low molecular weight compounds, These gelators are of particular interest for many technical applications. The self assembly of these gelator molecules often occurs by means of non-covalent interactions such as hydrophobic interaction, π-π interactions, electronic interactions, hydrogen bonding or combinations thereof. A suitable gel for the present invention can be found in Kiser et al, (Nature 394, 1998, 459-462).

Surprisingly, it has been found that gelling agents or thickeners can be prepared from low molecular weight carbohydrates. A preferred gelling or thickening agent of the invention therefore relates to a gelling agent in the form of a N,N'-disubstituted aldaramides and N,N'-disubstituted pentaramides and derivatives thereof. Specifically, the gelling or thickening agent relates to a gelling agent having the following structure

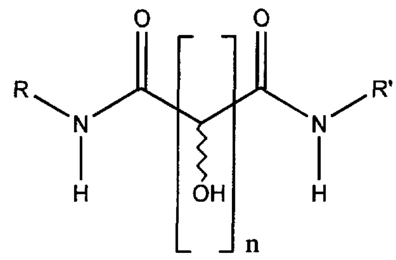

(II),

wherein n is 3 or 4, and wherein R and R' represent the same or different substituents chosen from the group of substituted or unsubstituted, branched, possibly aromatic groups containing, cyclic or linear alkyl, alkenyl, alkynyl groups having from 1 to 40 carbon atoms. In a preferred embodiment, R and R' each represent independently a linear, branched, or cyclic alkyl group having 4-20 carbon atoms. More preferred is that R and R' each are independently selected from the group of cycloalkyl groups having 4-16 carbon atoms. In a preferred embodiment, R and R' represent the same substituent.

It is one of the advantages of the present gelling agents or thickeners can be based on naturally occurring products, such as carbohydrates. Thus, the starting materials for producing them are from a renewable source.

A gelling agent or thickener according to this embodiment of the invention may be prepared by converting an aldose or pentose to its corresponding aldaric or pentaric acid, or a salt thereof, such as an alkali metal salt or an (alkyl) ammonium salt. It is preferred to use an aldose or pentose chosen from the group of allose, altrose, glucose, mannose, gulose, idose, galactose, talose, ribose, arabinose, xylose, lyxose and derivatives thereof, as these lead to products having particularly favorable gelling and/or thickening properties. It is to be noted that both the L and the D isomers of the aldose or pentose, as well as mixtures thereof, can be used. Suitable derivatives of the mentioned aldoses and pentoses include deoxy aldoses or pentoses, ethers, esters and the like. In a more preferred embodiment, D-glucose is chosen as aldose.

The conversion of the aldose or pentose to its corresponding aldaric or pentaric acid is generally achieved by oxidation. The oxidation can suitably be carried out using $Pt/O_2$,TEMPO/NaOCl/(NaBr) or $HNO_3/(NaNO_2)$ as an oxidizing agent. Further details for the manner in which the oxidation may be carried out can be found in US patents 5,831,043, 5,599,977 and 6,049,004, and in J. Org. Chem., 1977, 42, 3562-3567; J-F. Thaburet *et al*., Carbohydr. Res. 330 (2001), 21-29, all of which are incorporated herein by reference.

The thus obtained aldaric or pentaric acid or salt thereof is subsequently condensed with a primary amine to obtain the objective gelling agent or thickener.

The aldaric or pentaric acid can be condensed with an amount of at least 200 mole%, with respect to the aldaric or pentaric acid, of a primary amine. It is preferred to activate the aldaric or pentaric acid first by means of lactonization and/or esterification, depending on the stereochemistry of the carbohydrate. Further details may be found in Kurtz et al., J. Biol. Chem., 1939, 693-699; Hoagland, Carbohydrate Res., 1981, 98, 203-208, and US patent 5,312,967, which are incorporated herein by reference.

In an alternative embodiment, non-symmetrical N,N'-dialkylaldaramides or N,N'-dialkylpentaramides may be prepared, wherein R and R' represent different substituents. In accordance with this embodiment, the aldaric or pentaric acid may be converted into an N-alkyl-1-aldar/pentaramid-6-ate or N-alkyl-6-aldar/pentaramid-1-ate (as disclosed in US patent 5,239,044; L. Chen *et al*., J. Org. Chem., 61 (1996) 5847-5851; R. Lee *et al*., Carbohydr. Res. 64 (1978) 302-308; and K. Hashimoto *et al*., J. Polym. Sci. Part A, Polym. Chem., 37 (1999) 303-312), activated, and subsequently condensed with, preferably 100 mole% with respect to the N-alkyl aldar/pentar-ate, of a second primary amine.

In general, the thus obtained gelling agent or thickener precipitates from the reaction mixture in which it is formed and can be easily isolated by filtration. Further purification can be performed by conventional techniques like crystallization or, in the case of products based on galactaric acid derivatives, by thoroughly washing with ethanol, water, acetone or hexane.

It will be understood that the use of the present gelling agents or thickeners to prepare a gel or to thicken a composition is also encompassed by the invention. As is well-known, gelling behavior of compounds or compositions is highly unpredictable. In principle, a solution of a specific compound in a solvent, e.g. an organic solvent, is considered a gel when a homogeneous substance is obtained which exhibits essentially no gravitational flow. Preferably, the gelling phenomenon is thermoreversible. However, in as far as the present compounds do not provide a gel in a composition, they may be used as a thickener or rheology controlling agent as their addition to a composition may give rise to an increase in viscosity of the composition.

Compositions in which the present compound have been found to produce a gel include compositions in numerous organic solvents. Preferred examples include aromatic and non-aromatic hydrocarbons, alcohols, ethers,

esters, aldehydes, alkanoic acids, epoxides, amines, halogenated hydrocarbons, silicon oils, vegetable oils, phosphoric esters, sulfoxides and mixtures thereof. The compound also produces a gel in hydrophilic solvents such as water. The choice of composition for gelling can be tuned to the invented use. For instance in situations where clinical application of a vehicle of the invention is intended biocompatibility of the composition is preferred. In order to obtain a gel, the gelling agent or thickener is preferably mixed with the composition to be transformed to a gel in an amount of between 0.01 and 50 wt.%, based on the weight of the composition. In a preferred embodiment the mixture of the gelling agent or thickener and the composition is heated to allow for an even better gel formation or thickening. Typically, the heating will involve raising the temperature of the mixture to about 30 - 175 °C until a clear solution is obtained. In an alternative embodiment, the gelling agent is first dissolved in a polar or apolar solvent and then added to or sprayed into a composition or solvent to be converted into a gel.

Once a gel is formed in a gelling composition, the conditions may be changed such that those favorable for gelling are used to prepare the gel, whereupon the gelforming conditions are altered, removed or replaced by conditions favoring other aspects, such as the formulation of a biocompatible gel. Preferably, the substance to be made available in an induced way at the predetermined site, is incorporated in the gel at the time of gelformation. However, this need not always be true. Substance may also be allowed to enter a preformed gel under the appropriate conditions.

In a preferred embodiment the substance to be made available, i.e. the substance of interest, is in some way prevented from unintended leakage out of the gel. This is preferably achieved by allowing for an interaction of the substance with the gel. Said interaction can be achieved using a covalent bond or a non-covalent bond. release of the substance from the gel can be achieved in a number of ways known to the person skilled in the art and depending on the type of gel, substance and environment.

In a preferred aspect of the invention said gelling and or thickener agent comprises a light switchable gelator. Photo-controlled gelation has been reported by Murata et al., J. Am. Chem. Soc., (1994), 116, 6664-6676. They disclose certain cholesterol-azobenzene derivatives which isomerize from the trans-state into the cis-state upon irradiation with light. Gelators comprising a light switch resulting in altered gelling behaviour between the ground state and the light-activated state are very suited for a vehicle of the invention. A further suitable light-switchable gelator is provided by the invention.in the from of a light-switchable gelator is provided having the formula (III):

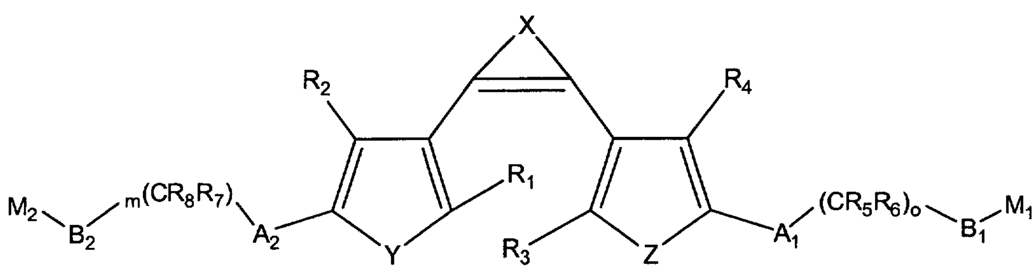

(III)

wherein

- X is chosen from the group of the moieties $-(CH_2)_n-$, $-(CF_2)_n-$, -C(=O)-C(=O)- and -C(=O)-NR-C(=O)-, wherein n is 3 or 4 and wherein R is hydrogen, a (cyclo)alkyl group or an aryl group;
- Y and Z each are nitrogen or sulfur;
- $R_1$ and $R_3$ each are an alkyl group;
- $R_2$ and $R_4$ each are hydrogen or an alkyl group;
- $A_1$ and $A_2$ each are absent or are an aryl group;
- $R_5$, $R_6$, $R_7$, and $R_8$ each are hydrogen, an alkyl group or an aryl group;
- m and o each are integers chosen from the group of 0, 1, 2, 3, and 4;
- $B_1$ and $B_2$ are hydrogen bonding moieties; and
- $M_1$ and $M_2$ each are an aryl group, a (cyclo)alkyl group, or $-CR_9R_{10}R_{11}$,

wherein $R_9$, $R_{10}$ and $R_{11}$ each are hydrogen, a (cyclo)alkyl group, an aralkyl group or an aryl group. It is to be noted that all symbols defined above may be chosen to have a meaning as defined, independent on the meaning of any of the other symbols, unless otherwise indicated herein.

A compound according to the invention can be used to form a stable gel. The gellation phenomenon can be induced by light and has been found to be reversible. This opens a wide range of possible applications of a compound

according to the invention, including the generation of a delivery vehicle for delivering a substance of interest to a predetermined site. An additional advantage of use of a light-switchable gelator with said characteristics is that induction of availability of said substance can be achieved by providing the correct light at the pre-determined site. In this embodiment of the invention the means for inducing availabiltiy include the signal comprising of the correct type of light and the receptor comprising the light-switchable gelator.

As used for the compound of formula III, the alkyl group refers to a straight-chain or a branched-chain alkyl radical containing from 1 to 10, preferably from 1 to 8, carbon atoms. The term (cyclo)alkyl group refers to an alkyl group or a cyclic alkyl radical. The latter includes saturated or partially saturated monocyclic, bicyclic or tricyclic alkyl radicals wherein each cyclic moiety contains 3 to 8 carbon atoms. Examples of such radicals include methyl, ethyl, n-propyl, isopropyl, n-butyl, isobutyl, sec-butyl, tert-butyl, pentyl, iso-amyl, hexyl, octyl, cyclopentyl, cyclopentenyl, cyclohexenyl and cyclohexyl.

The term aryl group for the compound of the formula (III) refers to an aromatic or hetero-aromatic ring system, such as a phenyl, naphtyl or anthracene group, preferably a phenyl, radical which optionally carries one or more substituents chosen from the group of alkyl, methoxy, halogen, hydroxy, amino, nitro, and cyano. Examples of such radicals include phenyl, p-tolyl, 4-methoxyphenyl, 4-(tert-butoxy) phenyl, 4-chlorophenyl, 4-hydroxyphenyl, 1-naphtyl, and 2-naphtyl. It is to be noted that fused and connected rings, as well as 5, 6, 7 or 8-membered rings, such as cyclopentadienyl, imidazolyl, thiophenyl, thienyl, etc., are included.

The term aralkyl group means an alkyl radical as defined above in which one hydrogen atom is replaced by an aryl radical as defined above, such as benzyl, or 2-phenylethyl.

In a preferred embodiment, the invention relates to a light-switchable gelator as defined above having the formula (IV):

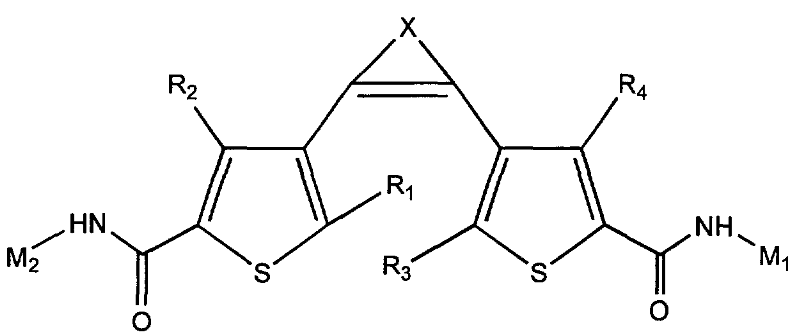

(IV)

wherein $R_1$ and $R_3$ are both methyl, and the other symbols having the same meanings as defined above. It is further preferred that $R_2$ and $R_4$ each are hydrogen or a methyl group. Preferably, $R_2$ and $R_4$ have the same meaning.

In another preferred embodiment of the invention, $M_1$ and $M_2$ have the same meaning. Preferably, $M_1$ and $M_2$ are phenyl or $-CR_9R_{10}R_{11}$, wherein $R_9$ is hydrogen, $R_{10}$ is cyclohexyl, cyclopentyl, or an aryl group, and $R_{11}$ is an alkyl group. Even more preferred is an embodiment wherein $M_1$ and $M_2$ are phenyl, $-CH(CH_3)(C_6H_5)$, or $-CH(CH_3)(C_6H_{11})$.

The invention further relates to a process for preparing a light-switchable gelator as described above. In accordance with this process, suitable starting materials are materials having formula (I) wherein X is $(CH_2)_3$, Y and Z are sulfur, $R_1$ and $R_3$ represent a $CH_3$ group, $R_2$ and $R_4$ are hydrogen, and $A_1$ and $A_2$ are each chosen from the group of hydrogen, chloride, bromide and iodide. These materials may be prepared using any known method. Suitable ways of preparing them have been disclosed in Lucas et al., Chem. Commun., 2001, 759, and Lucas et al., Chem. Commun., 1998, 2313, the contents of which are incorporated herein by reference.

The starting materials may be converted to produce a gelator according to the invention according to the reaction scheme shown in Figure 18, which illustrates a convenient preparation method starting from the above mentioned starting materials wherein $A_1$ and $A_2$ are both chloride (compound **Z**, figure 18).

In a first step, these materials may be converted to their corresponding formyl derivatives (compound **A**, Figure 18), for instance by reaction with n-butyl lithium and dimethylformamide. It will be understood that other lithiating agents can also be used. As a solvent any ether may be employed. Preferred solvents are tetrahydrofuran (THF) and diethyl ether. The temperature at which the lithiation reaction can be performed may range from -80°C to 50°C, but is preferably between -10°C and 10°C, more preferably around 0°C.

In a second step, the formyl group may be converted to a carboxylic acid group to produce a diacid derivative (compound **B**, Figure 18). This is may be done using any oxidizing agent, but is preferably involves silver oxide in water under reflux conditions as described by Gronowitz et al., Heterocycles, 1981, 15, 947.

In an alternative embodiment, the starting material is directly converted to the diacid derivative by quenching the reaction mixture with solid or gaseous carbon dioxide after lithiation has taken place, as described by Norsten et al., J. Am. Chem. Soc., 2001, 123, 1784. It is furthermore possible to perform this step by quenching with a carbamate in order to obtain the corresponding ester, which can be hydrolysed to the diacid.

The final step involves the transformation of the carboxylic acid derivative to the desired amide derivative, by known methods involving first the activation of the carboxylic acid, and secondly reaction of the activated carboxylic acid with the amine (Figure 18) (see e.g. J. March, Advanced Organic Chemistry, Wiley, NY, 1992) Preferably, the diacid derivative (**B**) is treated with N-methylmorpholine and subsequently activated with 2-chloro-4,6-dimethoxytriazine in any chlorinated solvent, such as $CH_2Cl_2$, or ether or dimethylformamide, at a temperature which may vary between -50°C and 30°C, but is preferably between -5°C and 5°C. After the activation N-methylmorpholine is added again and subsequently the desired amine, like phenylethylamine and the reaction is raised to room temperature. Of course, N-methylmorpholine can be substituted for any other suitable base.

Compounds with $A_1$ and $A_2$ groups present can be synthesized via a cross coupling reaction, preferably the Suzuki-coupling. The same compound as described above (**Z**) can be used as starting material. The compound is lithiated first (see above) and subsequently quenched with a boronic ester, preferably n-butyl boronic ester. The formed bis boronic ester derivative is directly used in the cross coupling reaction with a compound of the formula $Q_{1,2}$-$A_{1,2}$-$(CR_{5,7}R_{6,8})_{m,o}$-$P_{1,2}$, in which $Q_{1,2}$ can be Cl, Br or I, but preferably Br or I, and $P_{1,2}$ is an amine or carboxylic acid, or any functional group which can be converted to an amine or carboxylic acid.

The solution of the bis boronic ester may be added, preferably dropwise, to a mixture of $Q_{1,2}$-$A_{1,2}$-$(CR_{5,7}R_{6,8})_{m,o}$-$P_{1,2}$, a catalyst, base and a few drops of ethylene glycol just below reflux temperatures. The solvent is preferably THF, but also other ethers, or aromatic solvents like toluene can be used. The catalyst can be any palladium-, iron- or nickel catalyst, but is preferably palladium tetrakistriphenylphosphine ($Pd(PPh_3)_4$). As a base, a solution of $Na_2CO_3$ in $H_2O$ or $Na_2CO_3$ $xH_2O$ is preferably used, however, any other inorganic base can also be used. After the solution of the bis boronic ester has been added, this mixture is preferably refluxed for a suitable time such as two hours, and subsequently worked-up, to give a compound with $A_1$-$(CR_5R_6)_m$-$P_1$ = $A_2$-$(CR_7R_8)_o$-$P_2$ present.

In order to synthesize compounds in which -$A_1$-$(CR_5R_6)_m$-$B_1$-$M_1$ ≠ -$A_2$-$(CR_7R_8)_o$-$B_2$-$M_2$ a modified Suzuki reaction can be used in which only one side of the molecule undergoes this reaction. This can be accomplished by using the procedure described above for the synthesis of the bis boronic ester derivative wherein only one equivalent of the lithiating agent is used. In this way only one boronic ester per molecule is formed (bis and no substitution less than 5%). This mono boronic ester derivative is then coupled to one equivalent of $Q_1$-$A_1$-$(CR_5R_6)_{m}$-$P_1$. This reaction sequence can be repeated with a different $Q_2$-$A_2$-$(CR_7R_8)_o$-$P_2$ to give a the non-symmetrical substituted precursor for the objective gelator.

Functional group P can be converted to a carboxylic acid group or an amine (Figure 19). For instance if P is a halogen it can be transformed to an azide, which can be reduced to an amine or it can react with succinimide to form a protected amine. Another example is when P is a nitro group, which can be reduced to an amine. Further, P can be an aldehyde which can be oxidized to a carboxylic acid. When P is a nitrile group, it can be hydrolysed to a carboxylic acid. The amine in its turn can be converted to a urea group or an amide by known methods (ref J. March, Advanced Organic Chemistry, Wiley, NY, 1992). (Figure 19). The carboxylic acid can be converted to an amide (Figure 19). The carboxylic acid precursors are then converted to the corresponding carboxylic acid azides, which in an Curtius rearrangement are converted to the corresponding isocyanates by known methods (ref J. March, Advanced Organic Chemistry, chapter 18, Wiley, NY,1992). The isocyanate can be turned into an urea group by means of an amine.

In a preferred embodiment for the symmetric precursor compound $P_{1,2}$ is a methyl ester. After the Suzuki reaction has been carried out, the ester can be hydrolysed to the corresponding carboxylic acid. The hydrolysis can be carried out using any standard saponification conditions, e.g. with a base in water or a water/organic solvent mixture. Preferably, 4M NaOH in a water/THF mixture is used (Figure 20). This compound can thus be treated in the same way as described above in order to obtain the amide derivatives, or used as starting material to prepare a bis-urea derivative or -NHCO- group (see above and Figure 19). Another example is given in which 1,4-dibromobenzene is used in a Suzuki reaction with compound **Z** (Figure 21). In this way a precursor with a halogen is synthesized.

If $P_1 \neq P_2$ and have different reactivities, a sequence of selective reactions can be used to prepare the desired compounds.

The invention further relates to the use of a light-switchable gelator as described herein to prepare a gel. According to the invention, a gel may be prepared by dissolving the gelator in a suitable solvent by heating (if necessary), and subsequently inducing gel formation by cooling and/or irradiating it with light.

Suitable solvents may be chosen from the group of aliphatic hydrocarbons, aromatic hydrocarbons, halogenated hydrocarbons, non-aromatic hydrocarbons, aromatic solvents, alcohols, ethers, esters, aldehydes, alkanoic acids, epoxides, amines, silicon oils, vegetable oils, phosporic esters, sulfoxides, ketones and mixtures thereof. Preferred solvents are hydrocarbons, aromatic hydrocarbons and other aromatic solvents.

A light-switchable gelator of formula (III) or formula (IV) will typically be present in the solution in a concentration of between 0.01 and 10 wt.%, based on the weight of the solution. The temperature needed in order to form a gel will depend on the solvent chosen as well as on the exact structure of the gelator and its concentration. In a preferred embodiment the mixture of the gelling agent and the solvent is heated to dissolve the gelling agent, and subsequent cooling allows the formation of a gel. Typically, the heating will involve raising the temperature of the mixture to about 30 - 175°C. Typically, the minimal temperature needed to achieve gelation will lie in the range of -10 to 100°C, preferably in the range of 30 to 80°C.

The gelation process can be monitored by rheology, microscopic methods, and spectroscopic methods. In the non-restrictive case that $M_1$ and/or $M_2$ are chiral, gelation results in a strong enhancement of the elipticity of the samples as measured by circular dichroism (CD) spectroscopy.

An important property of the gelators according to the present invention is that they can exist as two thermally stable valence isomers, which can be converted into each other by irradiation with light in the range of 200-800 nm (Figure 22). It will be understood that both valence isomers are encompassed by the invention.

Irradiation of the open form of the gelator (see Formula (III) above) with light of a lower wavelength ($\lambda_1$) causes conversion to a photostationary state (PSS) in which the ring closed form is predominant, and irradiation of the PSS with light of higher wavelength ($\lambda_2$) causes conversion to the open form of the gelator. Here, $\lambda_1$ is preferably in the range of 250 to 600 nm, and even more preferably 300 to 450 nm, whereas $\lambda_2$ is preferably in the range of 350 to 900 nm, and even more preferably 450 to 700 nm. The isomerization process can be monitored by spectroscopic methods, and especially UV-VIS spectroscopy, due to the presence of a strong absorption of the closed form with a maximum between 400 and 700 nm, which is absent for the open form.

Most remarkably, photoswitching between the two valence isomers of the gelator has a pronounced effect on thermal stability of the gels, as well as on the kinetics of gel formation. In an preferred embodiment, a gelator with the structure of formula IV can be switched from the open form to the closed form by irradiation with light between 300nm and 450 nm, which is accompanied by an change of the melting point of the gel by 5-50°C, the exact value depending on the structure of the gelator, the solvent used, and the concentration of the gelator. In an even more preferred embodiment the melting point of the closed form of a gelator according to Formula IV is by 5-50°C higher than that of the open form, and gel formation in solutions of the closed form of the gelling agent is faster than gel formation in solutions of the open form.

In accordance with the present invention, the differences in thermal stability and kinetics of gelation between the open and closed form of the gelators may be exploited to induce gel formation by irradiation with light. When the melting point of a gel of the closed form is lower than that of the open form, photoinduced gelation can be achieved at a temperature between the melting point of the open and closed form, by irradiation of such a solution with light of wavelength $\lambda_2$ which causes isomerization from the closed to the open form. In another case, a gel of the open form has a lower melting temperature than that of the closed form, and gelation can be achieved at a temperatures between the melting point of the open and closed form, by irradiation of such a solution with light of wavelength $\lambda_1$ which causes isomerization from the open to the closed form.

It is also possible to make use of the differences in kinetics of gelation between the open and closed form to induce gelation by irradiation with light. In general, gelation at a set temperature below the melting point of the gels, is faster for gels of the valence isomer of the gelator having the higher melting point. Irradiation of a supercooled solution of an isomer of the gelator having the lower melting point with light of a wavelength causing isomerization to the other isomer of the gelator, results in a strong acceleration of the gelation process. In a preferred embodiment, a solution of a gelator according to Formula II is cooled to 10-50°C below the melting point, and irradiation of such a solution with light of wavelength $\lambda_1$ causing isomerization to the PSS (see above) together with gelation within 10 minutes, whereas a similar nonirradiated solution does not turn into a gel within this period.

Another important aspect of the invention is that gelation by a gelator according to the invention is reversible. This reversibility also holds for the photo-induced isomerization processes, and all the photo-induced gelation processes described above can be reversed by performing the back-isomerization by irradiation with light as depicted in Figure 22. It is clear that both the induction of gel-formation and the reverse process by switching between open or closed states can be used to work the present invention. Induction of gel-formation in or of a vehicle of the invention can be used to limit availability at sites that are not the predetermined site, where the induction of fluidization of the gel is typically used to allow for availability of the entrapped substance at the predetermined site. However, the reverse is also true. For instance, when the substance is made available to bind another compound at the predetermined site whereupon inadvertent release of the bound other compound or substance at further sites should be at least in part prevented. In this particular embodiment it is preferred that said gel or components thereof are enclosed in a film. The film in this embodiment at least in part prevents leakage of the loose components and substrate in the fluidized state at the predetermined site. In this condition the substrate is maximally available for binding another compound at the predetermined site. Inadvertent release of substrate or compound bound thereto can then be at least in part prevented by providing light of the wavelength suitable to induce gel-formation thereby effectively trapping the substance and bound compound in the gel at or subsequent to passage from the predetermined site.

*The predetermined site*

The predetermined site can be any site where said compartment should be made available toward the exterior of said vehicle. A predetermined sites preferably comprises a site in a mammalian body. Preferably, a human body. Said site can be, on the outside of said body for instance the skin or eye. Preferably said site is an internal site. An internal site is preferably characterised by a certain molecule that is present at said predetermined site. Preferably, said characterising molecule is not present at other sites in said body. In a preferred embodiment said characterising molecule is present on a cell. Preferably said target molecule is used to target the vehicle of the invention to the predetermined site. It is possible to use a vehicle comprising a targeting means. With a targeting means is meant a means for concentrating the vehicle at the predetermined site. A targeting means is typically provided to the vehicle, though this is not necessarily so. Targeting means typically comprises proteinaceous molecules capable of specifically binding a target molecule. Concentration at the predetermined site can in these situations be achieved by providing a targeting means for target that is specifically present at the predetermined site. It is possible that the target is also present at "a limited number" of other sites. In these cases it is preferred that the means for inducing availability of said compartment are not responsive to conditions at these "limited number" of other sites. As mentioned above, a preferred such means comprises a pH-sensitive AcmA protein. However, also in cases where the inducing means are responsive, and thus induce availability of the said compartment at a number of these other sites, it is still possible to achieve advantageous effects with a delivery vehicle of the invention, depending on the nature of the other sites where the compartment is made available. It can be that at the mentioned other sites, the substance is less toxic, or that limited loss of substance at non-relevant site can be tolerated without affecting the effectivity of a delivery vehicle of the invention. Similarly it is within the scope of the invention that said inducing means is also active at a limited number of other site, independent of the presence or absence of a targeting means. Such activation of the inducing means at other than relevant sites can be tolerated to some extent as long as the reasons for which a delivery vehicle of the invention was used are not negated. This is so if the toxic effects and/or stability problems or accelerated loss of activity can be tolerated. In a preferred embodiment said targeting means acts in synergy with an inducing means of the invention to preferentially make at least one compartment of said vehicle available toward to exterior at the predetermined site. Considering this synergistic effect it is preferred that a vehicle of the invention further comprises a targeting means for directing said vehicle to said predetermined site. A preferred internal predetermined site is the blood stream, where compound should be made available without suffering from rapid clearance or deactivation problems typical for peptidic substances. Other preferred internal sites are the lymph, the gastro-intestinal tract, the urogenital system, the central nervous system, the respiratory system, the peritoneum, organs and tumor. Other preferred sites are sites comprise invading organisms such as bacteria, fungi, yeast and virus, such sites can for instance comprise of a certain tissue or cell type that is otherwise distributed throughout, or over more places in the body. Targeting to invading micro-organisms is preferably achieved using AcmA, AcmD protein anchors. For examples of suitable AcmA and AcmD proteins reference is made to the examples and to WO 99/25836.

*The substance of interest*

In principle any type of substance can be made available using a vehicle of the invention. Substances can range from herbicides, insecticides, cosmetics and drugs. Where the vehicle is used in a mammalian body or for mammalian cells the substance preferably comprises a biologically active substance. A biologically active substance can be any substance. Examples of suitable substances include,

- Interleukins: peptides and proteins that modulate the immune response
- Diphteria toxin (fragment): potent inhibitor of protein synthesis in human cells
- Muramyl dipeptide: activator of immune system; macrophage-mediated destruction of tumor cells
- Cis-4-hydroxyproline: potential treatment for lung fibrosis
- Cisplatin (derivatives): cancer treatment
- Cytosine arabinose: cancer treatment
- Phosphonopeptides: antibacterial agent
- b-Glucuronidase: activator of prodrugs (e.g., epirubicin-glucuronide)
- Cytostatic drugs (doxorubicin,ciplatin etc.)
- Small therapeutic proteins/peptides (interleukins, growth factors, chemokines)

EXAMPLES

EXAMPLE 1. Use of channel-containing liposomes as delivery vehicles for the controlled release of drugs

Example 1-A. MscL-containing liposomes as drug delivery vehicles

Material and Methods

MscL Expression and Purification

*E.coli* PB104 cells containing the plasmid pB104 carrying the MscL-6His construct was grown to mid-logarithmic phase in Luria Bertani medium (10L fermentor) and induced for 4 h with 0.8 mM IPTG [Blount, P. et al., 1996, EMBO J. 15: 4798-4805]. Cells were French-pressed and membranes were isolated by differential centrifugation, as previously described [Arkin, I.T. et al., 1998, Biochim.Biophys.Acta 1369: 131-140]. The membrane pellet (5-8 g wet weight) was solubilized in 100 mL of buffer A (50 mM $Na_2HPO_4$.$NaH_2PO_4$, 300 mM NaCl 10 mM imidazole) containing 3% n-octyl β-glucoside. The extract was cleared by centrifugation at 120 000 x g for 35 min, mixed with 4 mL (bed volume) $Ni^{2+}$-NTA agarose beads (Qiagen, Chatworth, CA) equilibrated with buffer A and gently rotated for 15 min (batch loading). The column material was poured into a Bio-Spin column (Bio-Rad) and washed with 10 column volumes of buffer B (as buffer A, except 1% n-octyl β-glucoside) followed by 5 column volumes of the buffer B but with 100 mM imidazole. The protein was eluted with buffer B but with 300 mM imidazole. Eluted protein samples were analysed by fractionation on a SDS-15 % polyacrylamide gel followed by staining with Coomassie Blue or transferring the fractionated proteins to PVDF membranes by semi-dry electrophoretic blotting for immunodetection with a anti-His antibody (Amerham Pharmacia Biotech). Immunodetection was performed with an alkaline phosphatase conjugated secondary antibody as recommended by the manufacturer (Sigma).

Electrospray Ionization Mass Spectrometry of Detergent Solubilized MscL proteins.

Purified detergent solubilized G22C-MscL-6His was heated to 60 °C for 15' and precipitated protein was spun down at 14 000 rpm in a tabletop centrifuge (Eppendorf) for 5 min. The pellet was dissolved in 50% formic acid and 50% acetonitril just before electrospray ionization mass spectrometry (ESI-MS) analysis. The average molecular masses of the proteins were calculated from the m/z peaks in the charge distribution profiles of the multiple charged ions. Spectral deconvolution was performed on the peaks over the mass range from 800 to 1700 using the computer program MacSpec (Sciex). All molecular masses quoted in this paper are average, chemical atomic masses.

2-Sulfonatoethyl methanethiosulfonate Labeling of G22C-MscL-6His.

The single cysteine mutant, G22C-MscL-6His, was labeled with (2-sulfonatoethyl)methanethiosulfonate (MTSES) . A suspension of 20-30 μM of G22C-MscL-6His in buffer B with 300 mM imidazole ( 0.5 mL final volume), was incubated with 0.6 mM MTSES at 4 °C for 30 min. Conjugation was monitored employing ESI-MS.

Membrane Reconstitution of 6His-MscL

Dry lipid mixtures were prepared by co-dissolving lipids (Avanti Polar Lipids, Alabaster, AL) in chloroform, in weight-fractions as indicated in the experiments, and removing the chloroform by evaporation under vacuum for 4 h. All acyl chains of the synthetic lipids were of the type, dioleoyl, unless indicated otherwise.The dried lipid film was dissolved (20 mg/mL) in 50 mM potassium phosphate, pH 7.0, followed by three freeze/thaw cycles. An aliquot, 200 μL of the rehydrated liposomes and 5% n-octyl β-glucoside, was added to 200 μL purified 6His-MscL. Final protein-to-lipid molar ratio as indicated in the experiments. Subsequent membrane reconstitution was achieved by exhaustive dialysis into a buffer containing 0.1 mM $Na_2HPO_4$.$NaH_2PO_4$ pH 6.8 containing detergent-absorbing Bio-Beads SM-2 (Bio-Rad, Inc.).

Sucrose Gradient Centrifugation.

Discontinuous sucrose gradients were employed to analyze membrane reconstituted 6His-MscL as described elsewere [Knol, J. et al., 1998, Biochemistry 37: 16410-16415].

Freeze-Fracture Electron Microscopy.

Freeze-fracture electron microscopy of membrane-reconstituted 6His-MscL were performed as described elsewere [Friesen, R.H.E. et al., 2000, J.Biol.Chem. 43: 33527-33535].

Electrophysiologic Characterization of Membrane-Reconstituted MscL.

MscL was reconstituted into liposomes of different lipid composition and aliquots of 200 μL were centrifuged at 48 000 rpm in a tabletop ultracentrifuge (Beckmann). Pelleted proteoliposomes were resuspended into 40 μL buffer C (10 mM 4-morpholinepropanesulfonic acid (MOPS)-buffer, 5% ethylene glycol, pH 7.2), and 20 μL droplets were subjected to dehydration-rehydration cycle on glass slides [Delcour, A.H. et al., 1989, Biophys.J. 56: 631-636]. Rehydrated proteoliposomes were analysed employing patch-clamp experiments as described previously [Blount, P. et al., 1996, EMBO J. 15: 4798-4805].

In vitro Release Profiles of a Model Drug from Proteoliposomes

The percentage release of a fluorescent model drug, calcein, from MscL-containing liposomes was calculated from the dequenching of calcein fluorescence according to the following equation:

$$\% \text{ Release} = \frac{F_x - F_0}{F_t - F_0} \times 100$$

Where $F_0$ is the fluorescence intensity at zero time incubation, $F_x$ is the fluorescence at the given incubation timepoints and $F_t$ is the total fluorescence, obtained after Triton X-100 lysis. Fluoresence was monitored with a SLM 500 spectrofluorimeter in a thermostatted cuvette (1 mL) at 37 °C, under constant stirring. Excitation and emission wavelengths were, respectively, 490 (slit 2 nm) and 520 nm (slit 4 nm). The experiments were performed at lipid concentrations of approximately 50 μM. Control, and MscL-containing liposomes were prepared as described above followed by mixing with an equal volume of 200 mM calcein in PBS buffer. Then a freeze-thaw cycle has been repeated three times followed by extrusion through a 100 nm polycarbonate membrane [Mayer, L.D. et al., 1986, Biochim.Biophys. Acta 858: 161-168]. The liposomes were separated from free calcein by using Sephadex 50 column chromatography equilibrated with PBS (160 mM NaCl 3.2 mM KCl, 1.8 mM $KH_2PO_4$, 0.12 mM $Na_2HPO_4$, 1.2 mM EGTA, pH 8.0), which was isotonic to the calcein-containing buffer.

Results

Overexpression and purification of the MscL channel protein.

Since the expression level of His-MscL in *E. coli* was relatively low, based on the absence of a significant IPTG-inducible band on a SDS-PAGE, attention was focused on obtaining a high biomass during fermentation and a high yield after protein purification.
The His-tagged MscL could be purified to apparent homogeneity in a single step using nickel chelate affinity chromatography as shown by SDS-PAGE (Fig. 1, lane B). The yield of this eluted His-tagged MscL was ± 2 mg per Liter of cell-culture with an estimated purity of >98% based on analysis using SDS-PAGE and Coomassie Brilliant Blue staining.
The rate of excretion via MscL of small molecules is > 10 000 nmol/sec. x mg of cell protein, i.e. when the protein is in the open state. Since the expression level of mscL in wild-type bacteria is 4-10 functional units per cell and the MscL channel is a homopentamer of 15 000 Da, it can be concluded that the flux via a functional MscL channel is > $10^6$ x $s^{-1}$. This activity of MscL is such that on average 5 molecules of pentameric MscL per liposome with a diameter of 400 nm should suffice. Such a liposome contains approximately 1.67 x $10^6$ molecules of lipid; the molar ratio of lipid over MscL will thus be 0.67 x $10^5$. Consequently, 2 mg of MscL will yield 6 g of proteoliposomes.

Electrospray Ionization Mass Spectrometry of Detergent Solubilized MscL proteins.

ESI-MS is an accurate and effective method to verify primary sequences of the 6His-MscL protein and the stoichiometry of conjugation reactions. Figure 2 shows the ESI-MS spectra of the G22C-MscL-6His and the MTSES conjugated G22C-MscL-6His samples.
Based on the deduced amino acids, the average molecular weight of G22C-MscL-6His is 15 826 Da. ESI-MS analysis of G22C-MscL-6His resulted in a molecular weight of 15 697 Da, which corresponds to the deduced molecular weight minus a methionine. This observation would be consistent with an excision of the N-terminal methionine as reported for many proteins expressed in *E. coli* [Hirel, A.U. et al., 1989, Proc.Natl.Acad.Sci. U.S.A. 86: 8247-8251]. ESI-MS analysis of the MTSES conjugated G22C-MscL-6His resulted in a molecular weight of 15 837 Da, which corresponds exactly with the calculated mass increase of the MTSES conjugation. ESI-MS analysis is routinely used to verify the average masses of MscL mutants and the products of conjugation reactions.

Membrane reconstitution into liposomes of different lipid compositions. Purified detergent-solubilized MscL was reconstituted into preformed liposomes, which were titrated with low amounts of detergent. After removal of the detergent by adsorption onto polystyrene beads, proteoliposomes were formed. The proteoliposomes were characterized by equilibrium sedimentation on a sucrose gradient as shown in figure 3. All 6His-MscL protein detected by the Western blot (inset in Fig. 3) was associated with the lipid bilayer as detected by octadecylrhodamine-β-chloride ($R_{18}$) fluorescence.
Association of the 6His-MscL protein with the liposomes does not necessarily mean the protein is inserted correctly into the lipd bilayer. Correctly inserted MscL protein should be trans-membrane, and show up as an intra-membrane vesicle (IMP) in a freeze-fracture image as shown in the white boxed area of figure 4.
The equilibrium sedimentation and freeze-fracture electron microscopy experiments provided structural evidence for the correct reconstitution of the 6His-MscL protein into lipid bilayers.

Electrophysiologic characterization of membrane-reconstituted MscL activity.
The purified protein reconstituted into phospholipid liposomes forms functional mechanosensitive channels, as seen from the traces in Fig. 5 at different pipette pressures (mechanical activation). The MscL open probability plotted against pressure can be fitted with a Boltzmann distribution (Fig. 6).
Interestingly, reconstituted MscL is active in the absence of negatively charged lipid headgroups (Fig. 5, PC:PE 70: 30)). This is a very important finding since negatively charged headgroups prevent targeting to most target sites in the human body. Additionally, these experiments have shown for the first time that the pressure treshold is significantly effected by the lipid composition of the membrane reconstituted MscL channels (Fig. 6). This allows tailor making of the drug release profiles to the specific neads.
We have constructed several 6His-MscL mutants with altered gating properties, e.i.; mutants that are hypersensitive to membrane tension, and mutants with increased open probability at lower pH-values.

In vitro release profiles of a model drug.
The fluorescence efflux-assay was developed to monitor the MscL-mediated release profiles.. Liposomes (DOPC:Chol, 60:40, m/m) with and without MscL-6His were subjected to an osmotic downshock, thereby effectively increasing the membrane tension, to monitor the calcein release. As shown in Fig. 7, less calcein remained in the liposomes containing MscL (closed circles) relative to the liposomes without MscL (closed squares) when change in osmolality was larger than 200 mOsm. These data demonstrate that, upon osmotic downshock, liposomes containing reconstituted MscL exhibit a greater efflux of calcein than liposomes without MscL. This MscL-mediated efflux is consistent with the electrophysiologic analysis, showing that the MscL is reconstituted into membranes of synthetic lipids while retaining its functional properties.

For controlled release of drugs at the target site, membrane tension may not be the most promising stimulus since little is known about osmotic differences in the human body. Several alternatives to activate the MscL channel at the target site are described in this patent. Introducing a charge through conjugation of MTSES to cysteine at position 22 serves as an example for channel activation under iso-osmotic conditions. ESI-MS analysis of the MTSES conjugated G22C-MscL-6His protein showed that all MscL monomers were conjugated to a stoichiometry of 1:1. The conjugated G22C-MscL-6His samples was subsequently reconstituted into liposomes as described above and calcein release was measured as shown in Fig. 8. These data demonstrate that, in the absence of an increased membrane tension, MscL exhibits drug release from drug laden synthetic liposomes. This patent includes MscL conjugates that will release drugs at the target site as a function of pH, light activation and specific interactions with target associated molecules.

The integrity of liposomes consisting of phosphatidylcholine is seriously affected at first contact with a biological milieu following intravenous injection [Damen, J. et al., 1981, Biochim.Biophys.Acta 665: 538-545]. The integrity of liposomes (PC:Chol:DGPE-PEG, 55:40:5, m/m/m) was studied as a function of the molecular mass of the PEG group attached to the DGPE lipid in the presence of rat and human plasma at 37 °C. Calcein release from only the liposomes without DGPE-PEG and with 5 mol% DGPE-PEG(2000) are shown in Fig. 9. These data show that addition of 5 mol% of DGPE-PEG(2000) significantly increase liposomal integrity in rat and human plasma up to several hours and will serve to prevent drug leakage during the traveling time to the target cells Example 1-B. Light-switchable opening of the MscL channel

A photo reactive molecule, 4-{2-[5-(2-Bromo-acetyl)-2-methyl-thiophen-3-yl]-cyclopent-1-enyl}-5-methyl-thiophene-2-carboxylic acid (DTCP1), was designed and synthesized to reversibly switch conformation after light absorption of specific wavelengths. Nuclear Magnetic Resonance and spectroscopic analysis indicated DTCP1 was chemically and functionally correct as shown in Fig. 10.
DTCP1 was designed to specifically react with the free sulfhydryl group of a single cysteine at position 22 of MscL (G22C-MscL). Position 22 in the MscL channel was chosen for its involvement in the gating mechanism of the channel. A conjugation protocol was developed and the products were analyzed employing electrospray ionization mass spectrometry (ESI-MS) and absorption spectroscopy. ESI-MS indicated that the mass of all MscL subunits increased with 344 Da, a mass increase expected for a conjugation of DTCP1 to a sulfhydryl group of MscL as shown in Fig. 11. The

two photoisomer states of modified G22C-MscL exhibit different absorption spectra that can be used to monitor the switching of DTCP1 after conjugation to MscL as shown by the absorption spectra in Fig. 12. The detergent-solubilized G22C-MscL-DTCP1 conjugate was subsequently reconstituted into a DOPC:DOPS (90:10 m%) containing lipid bilayer and absorption at 535 nm was used to monitor the switching of DTCP1 in the membrane reconstituted channel (insert Fig. 12). These data show that we have synthesized an organic molecule (DTCP1) and this molecule can be conjugated to a specific site in the MscL channel, known to alter the gating properties of the channel. Electrophysiological characterization of the membrane-reconstituted G22C-MscL-DTCP1 channel showed a direct linkage between DTCP1 switching and the opening and closing of the MscL channel in the membrane.

Example 1-C. pH dependent opening of the MscL channel

The G22H-MscL mutant exhibits a higher open probability at (low) pH values that are frequently encountered in tumours, as compared to normal pH values of circulating blood. The G22H-MscL mutant and a double histidine mutant, G22H-V23H-MscL, were constructed to produce a pH-sensitive channel. Both the single and double histidine mutants exhibited very low overproduction levels in the E.coli expression systems. We have developed a new growth-protocol and significantly increased the overexpression of the single and double histidine mutants. The mutants were overexpressed, purified and membrane-reconstituted. The membrane reconstituted histidine-mutants exhibited pH-sensitive drug-release profiles.
Alternatively, the G22C-MscL is used for conjugation to 2-Bromo-3-(5-imidazolyl) propionic acid (BI) as shown in Fig. 13, effectively introducing a imidazole at the position of the imidazole of the histidine in G22H-MscL. BI conjugated G22C-MscL mimics the pH-dependent properties of the G22H-MscL, except that the G22C-MscL overproduction is relatively high. BI was synthesized and covalently attached to the G22C-MscL using the same protocol as used for DTCP1. Electrophysiological characterization of the membrane-reconstituted conjugate showed a significantly higher MscL channel open probability at pH 6 compared to pH 7, allowing controlled release of drugs at specific tumour sites. The BI is synthesized with different substituents to change the pKa of the imidazole. This allows fine-tuning of the gating properties of the conjugated channel to the specific application.

Example 1-D. Induced opening of the MscL channel by specific recognition

Three peptides, spanning the portion of the channel accessible at the exterior of the liposomes, were synthesized and used to raise antibodies in rabbits. The bleeds from these rabbits all contained antibodies specific for the synthesized peptides and consequently the full length MscL. Single channel electrophysiologic characterization showed that these bleeds contain antibodies that specifically recognize MscL in the open conformation. These antibodies were used to shift the conformational equilibrium to the open state of the channel.

Example 1-E. Controlled and/or localized release of a small molecule by tuning the composition of the lipid vesicle and/or the use of a mutant MscL.

Electrophysiological characterization was performed on MscL, reconstituted in membranes of different lipid compositions. Lipid compositions were chosen to significantly effect lateral pressure profiles of the lipid membranes to gain insight in the gating of the channel. Figure 14 shows that when the percentage of DOPE increases, the membrane tension necessary to open the channel decreases.
These results provide a rational for designing a drug-delivery vehicle with appropriate characteristics for specific applications. For instance, the system allows combinations of a specific MscL-mutant and lipid composition resulting in specific drug release profiles, tunable to specific needs. For example: N-Citraconyl-dioleoylphosphatidyl-ethanolamine (C-DOPE) was used in a lipid bilayer with DOPC (1:3, m%). The C-DOPE undergoes a proton catalyzed elimation reaction at pH 5.0, resulting in an increased fraction of DOPE in the bilayer. This increase in DOPE in the bilayer results in a higher open probability of MscL (see Fig. 14). This pH induced activation of MscL is used to effectively release drugs at the target site.

Example 1-F. Controlled release of molecules at a target site by means of membrane proteins selected from a large library.

Membrane channel proteins allow the exchange of molecules between compartments seperated by the membrane in which they are embedded by forming a channel in the membrane. The channel can be either in a closed (no exchange) or open (exchange) conformation, and the membrane protein can switch between the two conformations. By controlling the switching process via an external signal, release of molecules from a liposome containing the membrane protein embedded in its membrane is achieved. The signal is either pH, temperature, ionic strength or binding.

The membrane proteins that are sensitive to one or more of these signals are selected from a large random library of mutant membrane proteins using appropiate selection methods.

Example 1-G. pH dependent release of antibiotics by mutant OmpF.

A library of genes encoding for mutants of the *E. coli* outer membrane protein F (ompF) was created from the wild type OmpF gene by error prone PCR methods. The wild type OmpF gene in plasmid pGompF-Tet was replaced by the mutant genes using standard restriction and ligation methods. PGompF-Tet is an expression plasmid for OmpF in *E.coli* and was created from plasmid pGompF (Prilipov, A. et al., 1998, FEMS Microbiol Lett. 163: 65) by introducing a tetracycline resistance marker into the ampicillin resistance gene, thereby disrupting the latter. *E. coli* strain BL21 (DE3)omp8 (Prilipov, A. et al, 1998, FEMS Microbiol Lett. 163: 65), a strain that does not express the outer membrane proteins OmpA, OmpC and OmpF, was transformed with the plasmid library to obtain a library of *E. coli* clones expressing mutant OmpF proteins in their outer membrane.
Selection of suitable clones for the delivery of antibiotics was performed by using antibiotic sensitivity of *E. coli* as a tool. Antibiotics of the β-lactam class use the OmpF channel lumen as their pathway to enter the periplasmic space where they interfere with cell wall synthesis, leading to lysis of the cells. OmpF mutants that are mostly in the closed conformation (as opposed to wild type protein that is mostly in the open conformation) have a higher chance of survival when grown in the presence these antibiotics since the rate of access to the periplasm, and consequently the effective concentration of the antibiotic, is impeded (Nikaido, H., 1994, Science 264: 382). *E. coli* clones containing the random OmpF were plated on agar plates at pH 7.4 containing the β-lactam cephalotin at a concentration 10 μg/ml. This concentration is sufficient to inhibit growth of *E. coli* expressing wild type OmpF but allows growth of the bacterium in the absence of OmpF expression. Genes of the surviving clones were subjected to another round of random mutagenesis, ligated into the expression vector, transformed into *E. coli* as described above and plated out on non-selective (i.e. not containing cephalotin) plates. Transformants were transferred to plates at pH 6 containing cephalotin and were rechecked for survival. Clones that did not survive were selected and replated on plates at pH 7 containing cephalotin. The clones that survived the last selection step are permeable for the antibiotic cephalotin at pH 6 but not at pH 7.4 and were selected.
The mutant protein was identified, purified and reconstituted into proteoliposomes. The pH dependency of the OmpF mutant was confirmed by efflux of calcein from mutant containing proteoliposomes at pH 6 but no efflux was observed at pH 7.4. The pH-dependent release of cephalotin was confirmed by culturing *E. coli* containing wild type OmpF in its outer membrane in the presence of these liposomes at pH 6 and pH 7.4. Only in the latter case *E.coli* growth of the bacteria could be observed. Studies using an in vivo model in rats (see example 1A) showed faster release kinetics from the mutant containing liposomes when the pH in the tissue under study was lowered from ca 7.4 to 6.

EXAMPLE 2. Sunfish Amphiphiles as delivery vehicles

A. The introduction of biologically active compounds such as DNA, RNA, proteins and the like into eukaryotic cells is of interest in the light of several applications. The Sunfish Amphiphiles have been developed for delivery of above-mentioned substances. Delivery of DNA/RNA *(transfection)* has been used for therapeutic purposes as such. For example, antisense-oligonucleotides were introduced into a cell, bound to complementary single stranded nucleic acid molecules by appropriate base pairing. In doing so the function of the single or double stranded nucleic acid has been disrupted. In addition transfection has been used in gene therapy. Genes or parts of genes have been introduced to replace or repair defect part of genes or introducing missing genes.

B. Proteins and other macromolecules such as, for instance, carbohydrates have been introduced into cells for therapeutic and screening purposes *(transduction)* using Sunfish Amphiphiles. For example, introducing an immunogenic protein into a cell has enhanced immunisation, so that it has been more efficiently processed and presented on the surface of the cells, thereby enhancing the immunogenic response. Also, negatively charged macromolecules having a function in a cell have been transported past the cell membrane into the cytoplasm. Factors enhancing or hindering transcription of DNA have been used in a screening test to verify the transcription of a gene of interest. Such transcription assays have been exploited for the screening of compounds to determine their effect against a particular cellular macromolecule *e.g.* a cell receptor.

Suitable amphiphiles and a method for preparation thereof are presented in Example 13.

EXAMPLE 3. Use of the Protein Anchor as a targeting means

I. Use of the AcmA-type Protein Anchor as a targeting means

Targeting to microorganisms

The Protein Anchor is the cell-wall binding domain (Anchor) of the major cell-wall hydrolase AcmA of *Lactococcus lactis*, a Generally Recognized As Safe (GRAS) Gram-positive bacterium. The Protein Anchor consists of 3 homologous repeats of 45 amino acids that contain a specific consensus sequence (Table A; patent applications WO99/25836 and EP 01202239.8), separated by intervening sequences of about 30 amino acids that are highly enriched for serine, threonine and asparagines residues. This Protein Anchor has the ability to attach from the outside to a wide variety of Gram-positive (G+) bacteria, also when part of a chimaeric fusion protein. This trait offers the possibility to use this Protein Anchor in therapeutic applications as a device to target pathogenic bacteria in order to inactivate them. Inactivation may be achieved by coupling antibodies, cytokines (signaling molecules for the immune system) or drugs to the Protein Anchor. In another approach the drugs or other compounds may be incorporated in nano- or micro delivery-vehicles to which the Protein Anchor is attached in order to direct these vehicles to a specific target, in the case of the unmodified AcmA Protein Anchor mostly G+ bacteria.
The Protein Anchor has many homologs (domains in other proteins that resemble the Protein Anchor) in a wide variety of microbes and higher organisms (Table A; patent applications WO99/25836 and EP 01202239.8, incorporated herein by reference). These homologs have a different binding spectrum, which is directly applicable. An example of this is given under item 3-A. In addition, new Protein Anchors were obtained by combining the traits of the homologs. This was done by gene-fusion (example 3-B) and mutagenesis by *in vitro* recombination (example 3-C) and this resulted in Anchors that bind to the target at pH 6, but not at pH7.4. This is particularly relevant for targeting tumor cells. Tumors are known to have a lower pH (approximately pH6) than healthy tissues and body fluids (approximately pH7.4).

The AcmA Protein Anchor can be used to target Gram-positive bacteria. Example 3-A demonstrates that homologs of the AcmA anchor can be used to extend the range of micro-organisms that can be targeted. In the examples a reporter molecule is fused to the Anchor. In some applications Anchors are coupled to delivery vehicles that have the ability to make the drugs available upon induction (e.g. liposomes with MscL). Examples 3-B and 3-C demonstrate that modified AcmA-type Anchors can be made in order to obtain pH-dependent binding (induced availability) to the target. Also in this example a reporter molecule is fused to the Anchor and a model target is used. In some applications modified Anchors are coupled to delivery vehicles (e.g. liposomes, hydrophobin particles, etc.).

Targeting means is a Protein Anchor homolog

Example 3-A. Targeting of a reporter molecule through the use of a Protein Anchor homolog to a microorganism other than a Gram-positive bacterium.

The cloning of a Protein Anchor homolog, the anchor of MltD of the Gram-negative ($G^-$) bacterium *E. coli* that has the consensus sequence as described in Table A and patent applications WO99/25836 and EP 01202239.8, is described. The MltD anchor was fused to a reporter and binding of the reporter-anchor fusion to *E. coli* was demonstrated. This example describes targeting G- bacteria.

Materials and Methods

Plasmid pPA9 was used for cloning the MltD anchor (cMD). This plasmid contains the acmA gene (cell-wall hydrolase) devoid of its native Protein Anchor (acmA'). This truncated AcmA has no or little cell wall hydrolase activity. The cell wall hydrolase activity can be restored by cloning Protein Anchor homologs behind the truncated acmA. The cell wall hydrolase activity can be easily detected in plate- and gel assays. For immunological detection of the new fusion protein the c-myc epitope is present in pPA9 in frame downstream the truncated acmA. Downstream the c-myc epitope several unique restriction enzyme recognition sites were present that allow in frame cloning of Protein anchor homologs. The Polymerase Chain Reaction (PCR) method was used to amplify cMD using the primers MltDrepeat.fw (5'-GGA<u>AGACTC</u>AATTGCTGCTGTACAGTCGACG) and MltDrepeat2.rev (5'-TAAT<u>AAGCTT</u>AAAGGTCTCCAATTCCCAACGTCAGCTTATCGCCTGGT TGC) with E. *coli* chromosomal DNA as template. The cMD PCR fragment was digested with *Bgl*II and *Hin*dIII (underlined sequences in the primers) and was cloned into the *Bam*HI and *Hin*dIII sites of pPA9, resulting in plasmid pPA10 (produces protein: AcmA'::myc::cMD). Expression of AcmA'::myc::cMD using pPA10 in *L. lactis* NZ9000ΔacmA was done as described in Kuipers et al. (1997, Tibtech 15: 135-140). Detection of AcmA activity in plate- and SDS-polyacrylamide gel assays was described by Buist et al. (1995, J. Bacteriol. 177: 1554-1563) for use of peptidoglycan from *L. lactis.* Peptidoglycan from *E. coli* was isolated according to the method

described by Rosenthal and Dziarski (1994, Methods in Enzymology 235: 261-263) Western blots and immunological detection using anti-c-myc conjugated horseradish peroxidase (Roche) were performed according to the instructions of the supplier. E. *coli* cells were treated for 10 min at 20 °C with 0.5 mM EDTA in TES buffer (200 mM Tris pH 8.0; 0.5 mM EDTA; 0.5 M sucrose) to destabilize the outer membrane. Prior to binding of the Protein Anchor chimeara the cells were washed with TSM buffer (200 mM Tris pH 8.0; 0.5 M sucrose; 10mM $MgCl_2$) to remove the EDTA and to stabilize the spheroplasts. The *L. lactis* fusion protein AcmA'::myc::cMD was then incubated with the *E. coli* cells. The cells were subsequently washed with buffer TSM in order to remove unbound AcmA'::myc::cMD.

Results

*L. lactis* NZ9000ΔacmA(pPA10) was induced for expression of AcmA'::myc::cMD and culture supernatants were analyzed on SDS-PAA gels containing either peptidoglycan of *L. lactis* or of *E. coli.* The proteins in the gels were renatured and activity was only observed in the gels containing the *E. coli* peptidoglycan. From this result we concluded that the MltD anchor binds to the Gram-negative *E. coli* peptidoglycan, but not to that of the Gram-positive *L. lactis* peptidoglycan and that binding is a prerequisite for activity of the AcmA enzyme. This is in agreement with the observations of Buist et al. (1995, J. Bacteriol. 177: 1554-1563). Binding of AcmA'::myc::cMD to whole cells of *L. lactis* and *E. coli* was also studied by Western blot analysis. No binding was observed to *L. lactis*, whereas some binding to *E. coli* cells could be detected. The binding to *E. coli* could be further improved by pretreating the cells in order to disrupt the outer membrane. These results show that for targeting purposes based on the Protein Anchor principle the range of micro-organisms can be broadened by using AcmA Anchor homologs.

Targeting means is a Protein Anchor chimaera

Example 3-B. pH dependent binding of AcmA protein anchor homologs and hybrids.

The cell wall binding domain of the lactococcal cell wall hydrolase AcmA consists of three repeats of 45 amino acids that show a high degree of homology (Buist, G. et al., 1995, J.Bacteriol. 177: 1554-1563). These repeats belong to a family of domains that meet the consensus criteria (Table A) as defined in patent application WO99/25836 and can be found in various surface located proteins in a wide variety of organisms. Another feature that most of these domains have in common is that their calculated pI values are high: approximately 8 or higher (Table A). At pH lower than 8 these binding domains are positively charged.
The AcmA protein anchor (cA) homolog of the lactococcal cell wall hydrolase AcmD (cD) consists also of three repeats with a calculated pI that is much lower (approximately pI 3.8) than that of the cA domain (Table B).
Consequently, the cD anchor was negatively charged at pH 4 and higher. We have demonstrated that no binding of the MSA2::cD reporter protein occurred under these conditions. Therefore, we investigated here the influence of the pH during binding of a cD fusion protein (MSA2::cD). Furthermore, we demonstrate that a AcmA anchor (cA)/cD hybrid can be made that has showed binding at pH6 (usual in tumor tissues) but not at pH7.4 (usual in other body fluids).

Materials and Methods

Bacterial strains, growth and induction conditions, TCA pretreatment of L. *lactis* cells, incubation of the MSA2 proteins to TCA-pretreated cells, washing conditions, protein gelelectrophoresis, Western blotting and immunodetection were the same as described in patent application EP 01202239.8. Under the conditions used, the cell-free culture supernatants with MSA2::cA or MSA2::cD had a pH of approximately 6.2. To examine the influence of a pH lower than the pI of the cD anchor, the pH of the cultures was lowered with HCl to pH3.2.

Results

*Binding of MSA2::cD at low pH.* Since binding of MSA2::cD was not observed at a pH (the pH of the culture medium after growth and induction is about 6.2) higher than the calculated pI for the cD domain (pI 3.85), we studied the binding when the pH of the medium was adjusted to pH3.2. TCA-pretreated *L. lactis* cells were used as the binding substrate and the relative amounts of bound MSA2::cD were analyzed in Western blots. The amounts of unbound reporter protein left behind in the culture supernatant after binding were also analyzed. Fig. 15 shows that there is a clear increase in bound MSA2::cD when binding is performed at pH3.2 (compare lanes 1 and 3). At the same time less unbound reporter protein remained in the supernatant (compare lanes 2 and 4). This result indicates that positive charges are important for binding of cA-type anchoring domains.
*Binding of cA-type hybrid anchors.* Analysis of the pI values of the AcmA Protein Anchor homologs in Table A learned that two classes of repeats can be distinghuished: a majority (99 out of 148) of homologs that have a high pI value (>

8) and a group (33 out 148), of which AcmD Anchor is a representative, that has pI values lower than 6. Based on our experimental results it is likely to assume that these types of anchoring domains only bind to bacterial cell walls at a pH that is lower than its pI. Notably, most cell wall binding domain homologs consist only of repeats with a pI that are representatives of one of the two groups, i.e. only repeats with a high or low pI.
Interestingly, some proteins with putative cell wall binding domains, e.g. those of DniR of *Trepanoma pallidum* and an amidase of *Borrelia burgdorferi,* consist of repeats with high and low pI. We predict that the binding pH of such 'natural hybrid' cell-wall binding domains is below the intermediate pI value of the total number of repeats present in the domain. Therefore, using the cA and cD repeats that we have designed hybrid cell-wall binding domains that have an intermediate pI value. Table C lists a number of examples of such hybrids. We constructed the hybrid domain consisting of A3D1D2 repeats (for numbering of the repeats see patent application EP 01202239.8) with a total pI value of 7.39. Using a fusion of the reporter MSA2 with A3D1D2 we observed that binding to TCA pretreated *L. lactis* cells occurred at pH6 but not at pH7.4.

This Example demonstrates the induced availability (by pH) of a hybrid Protein Anchor for binding to a target. A model reporter molecule (MSA2) is used and a model target is used (TCA pretreated *L. lactis*). In some applications modified Anchors are coupled to delivery vehicles (e.g. liposomes, hydrophobin particles, etc.) and targeted to microorganisms or specific animal or human organs or tumors.

Example 3-C. pH Dependent binding of mutagenized Protein Anchors.

Mutagenesis of AcmA/AcmD Protein Anchors by *in vitro* recombination of these Protein Anchor homologs and the selection of a variant that binds to a model target (pretreated *L. lactis* 'ghosts') at pH6, but does not bind at pH7.4.

Materials and Methods

*Strains, vectors and Protein Anchor gene fragments. L. lactis* ghost cells were prepared as described in patent application EP 01202239.8. Vector T7Select10-3 and *E. coli* strains BLT5403 and 5615 (T7Select Phage display System, Novagen) were used for cloning of the mutagenized Protein Anchor gene fragments according to the instructions of the supplier. Gene fragments of the AcmA and AcmD Protein Anchor homologs of *L. lactis*, (Table A; Genbank accession numbers U17696, QGC125) were used for *in vitro* recombination. The cloning of the fragments was done such that the 15 amino acid S-tag was included. This tag allows easy immunological detection of the recombinant phages using the S-protein HRP-conjugate (Novagen).

*In vitro recombination.* The Protein Anchor gene fragments of acmA and acmD were amplified by the polymerase chain reaction (PCR). After removal of the free primers, about 2 to 4 μg of the DNA substrates were digested with 0.15 units of DNAseI in 100 μl of Tris-Cl pH 7.5, 1 mM $MgCl_2$, for 5 to 10 minutes at roomtemperature. Fragments of 70 to 200 bp were extracted from 2% low melting point agarose gel. The purified DNA fragments (10 - 30 ng/μl) were resuspended in PCR mix and reassembled in a primerless PCR reaction using *Taq* DNA polymerase (2.5 U) and a program of 94° C for 60 s, 40 cycles of [30 s 94° C, 30 s 50° C, 30 s 72° C] and a final extension of 5 min 72° C. The reassembly mixture was 40-fold diluted into fresh PCR mix with primers. After 15 cycles of PCR, consisting of [30 s 94° C, 30 s 50° C, 30 s 72° C] a single amplification products of the correct size were obtained. The resulting full-length amplification product was digested and ligated into the expression vector.
*Library construction and screening.* Construction, amplification and storage of the phage library was done according to the instructions of the supplier (Novagen). Screening for binding variants to *L. lactis* ghost cells was done by incubation of the phage library in phospate buffered saline (PBS) of pH6. After binding the ghost cells were extensively washed to remove unbound phages.
The phages were released from the ghost cells by incubation with mutanolysin (Sigma). The released phages were amplified and the binding, washing and amplification procedure was repeated two times. Finally single phages were amplified and individual phage lysates were incubated with ghost cells seeded in microtiter plates in duplo. In one set of microtiter plates the binding was done at pH6 and in the other set of plates the binding was done at pH7.4. After multiple washings an immunological detection was done using the S-protein HRP-conjugate (Novagen). Phages that showed binding at pH6 but not at pH7.4 were identified using a microtiter plate reader.

Results

The biopanning of the recombinant phages resulted in a phage population that bound to ghost cells at pH6. The counter screening at pH7.4 identified several putative phages that did not show binding at this specific pH. The mutagenized protein anchor DNA insert in one of these phages was isolated and subsequently cloned into a lactococcal expression vector that allows fusion of protein anchors to the reporter MSA2 (see example 3-B). Expression of the fusion protein, designated MSA2::X1, resulted in its secretion. Binding of MSA2::X1 to lactococcal ghost cells at pH6

and pH7.4 was analyzed by Western blotting. The results clearly showed that the X1 protein anchor binds to ghost cells at pH6 but not at pH7.4. Thus, pH dependent targeting proteins are obtained using AcmA-type Protein Anchor homologs.

This example demonstrates the induced availability (by pH) of a modified Protein Anchor for binding to a target. In the example the mutagenized Anchors are used to target a model substrate (TCA pretreated *L. lactis* 'ghosts'). In some applications modified Anchors are coupled to delivery vehicles (e.g. liposomes, hydrophobin particles, etc.) and are targeted to micro-organisms or specific animal or human organs or tumors.

Targeting to animal or human cells

The Protein Anchor of *L. lactis* AcmA has many homologs (domains in other proteins that resemble the Protein Anchor) in a wide variety of microbes and higher organisms (Table A; patent applications WO99/25836 and EP 01202239.8). These homologs may have a different binding spectrum, including eukaryotic cells. New Protein Anchors can be obtained by combining the traits of the homologs. This was done by *in vitro* recombination of a set of Protein Anchor homologs and this resulted in an Anchor that is able to attach to human intestine tumor cells.
Antibodies or other cell surface binding domains (e.g. lectins) can be used in order to target specific cell lines.

Example 3-D. Selection of mutagenized Protein Anchors to a preselected human tumor cell-line.

Mutagenesis of a preselected set of Protein Anchor homologs by *in vitro* recombination and the selection of a variant that binds to a preselected model tumor cell line (human intestine cancer cells).

Materials and Methods

*Strains, cell-lines, vectors and Protein Anchor gene fragments. L. lactis* ghost cells were prepared as described in patent application EP 01202239.8. Vector T7Select10-3 and *E. coli* strains BLT5403 and 5615 (T7Select Phage display System, Novagen) were used for cloning of the mutagenized Protein Anchor gene fragments according to the instructions of the supplier. Gene fragments of the Protein Anchor homologs of *L. lactis, E. coli, Drosophila melanogaster, Caenorhabditis elegans, Listeria monocytogenes, Enterococcus hirae, Staphylococcus aureus* (Table A; Genbank accession numbers U17696, QGC125, P24204, P43261, AF125384, Z79755, U41109, U64836, U70858, P21171, P39046, A04512) were used for *in vitro* recombination. The cloning of the fragments was done such that the 15 amino acid S-tag was included. This tag allows easy immunological detection of the recombinant phages using the S-protein HRP-conjugate (Novagen). Human intestine cancer cells (Intestine 407 / ATCC CCL6 also designated as Henle cells) were cultivated to confluence in RPMI 1640 medium (Gibco) supplemented with 10% fetal calf serum (Gibco), 1% L-glutamine, 1% non-essential amino acids, and penicillin-streptomycin followed by cultivation in microtiter plates.
*In vitro recombination.* The Protein Anchor gene fragments of the selected homologs were amplified by the polymerase chain reaction (PCR). After removal of the free primers, about 2 to 4 μg of the DNA substrates were digested with 0.15 units of DNAseI in 100 μl of Tris-HCl pH 7.5, 1 mM $MgCl_2$, for 5 to 10 minutes at room temperature. Fragments of 70 to 200 bp were extracted from 2% low melting point agarose gel. The purified DNA fragments (10 - 30 ng/μl) were resuspended in PCR mix and reassembled in a primerless PCR reaction using *Taq* DNA polymerase (2.5 U) and a program of 94° C for 60 s, 40 cycles of [30 s 94° C, 30 s 50° C, 30 s 72° C] and a final extension of 5 min 72° C. The reassembly mixture was 40-fold diluted into fresh PCR mix with primers. After 15 cycles of PCR, consisting of [30 s 94° C, 30 s 50° C, 30 s 72° C] a single amplification products of the correct size were obtained. The resulting full-length amplification product was digested and ligated into the expression vector.
*Library construction and screening.* Construction, amplification and storage of the phage library was done according to the instructions of the supplier (Novagen). Screening for binding variants was done by incubation of the phage library in phosphate buffered saline (PBS) with Henle cells immobilized on tissue culture plates. After binding the Henle cells were extensively washed to remove unbound phages. The phages were released from the Henle cells by using release buffers recommended by the supplier of the display kit (Novagen). The released phages were amplified and the binding, washing and amplification procedure was repeated three times. Finally single phages were amplified and individual phage lysates were incubated with Henle cells seeded in microtiter plates. After multiple washings an immunological detection was done using the S-protein HRP-conjugate (Novagen). Phages that showed binding were identified using a microtiter plate reader.

Results

The biopanning of the recombinant phages resulted in a phage population that bound to Henle cells. The

mutagenized protein anchor DNA insert in one of these phages was isolated and subsequently cloned into a lactococcal expression vector that allows fusion of protein anchors to the reporter MSA2 (see example 3-B). Expression of the fusion protein, designated MSA2::X2, resulted in its secretion. Binding of MSA2::X2 to Henle cells was analyzed by Western blotting. The results demonstrate that the X2 protein anchor binds to human intestine cancer cells. Thus, by using *in vitro* mutagenesis with AcmA-type Protein Anchor homologs as templates, targeting proteins are obtained that are able to bind to eukaryotic cells.
This example demonstrates that mutagenesis on Protein Anchor homologs can be used to obtain new Anchors that bind to tumor cells. In some applications Anchors are coupled to delivery vehicles that have the ability to make the drugs available upon induction (e.g. liposomes with MscL).

II. Use of protein domains other than AcmA-type Protein Anchors

Example 3-E. Multiple cell wall spanning domains of the lactococcal proteinase PrtP cause adherence to eukaryotic cells

A comparative *in silico* analysis of the amino acid sequences of known cell wall-bound extracellular serine proteinase (CEPs) from different lactic acid bacteria showed that the lactococcal PrtP has a modular, multidomain structure (Siezen *et al*. 1999. Antonie van Leeuwenhoek 76: 139-155). The N-terminal part, with the pre-pro domain containing signals for secretion and activation, is followed by the subtilisin-like serine proteinase domain and two large central domains that are thought to have regulatory and stabilizing functions. The C-terminal part of PrtP consists of (i) a helical spacer, followed by (ii) a hydrophobic Gly/Thr/Asp-rich putative cell wall spacer (CWS) domain that can span the peptidoglycan layer and, (iii) a cell wall anchoring domain. Some of the bacterial cell wall-anchored proteins are known to have adhesive properties (Navarre and Schneewind. 1999. Microbiol. Mol. Biol. Rev. 63: 174-229). *L. lactis* cells can adhere to one another via the sex factor CluA, in order to allow conjugal transfer of DNA. The cell-to-cell binding causes a cell aggregation phenotype (Godon *et al.* 1994. Mol. Microbiol. 12: 655-663). Pathogenic Gram-positive bacteria carry cell wall-anchored surface proteins that contribute to virulence (Foster and McDevitt. 1994. FEMS Microbiol. Lett. 118: 199-205). They do so by promoting adherence to the host cells and/or tissue components, and by binding a variety of serum proteins including albumin, collagen, complement regulatory factors, soluble forms of fibronectin and fibrinogen, and the proinflammatory plasminogen and kininogen (Patti *et al*. 1994. Annu. Rev. Microbiol. 48: 585-617). Some streptococcal M proteins as well as fibrinogen binding protein (FgBP) from *Streptococcus equi* can also mediate bacterial autoaggregation, a property shown to be crucial for adherence and resistance to phagocytosis (Frick *et al.* 2000. Mol. Microbiol. 37: 1232-1247, Meehan *et al.* 2001. Microbiology 147: 3311-3322).
Here, we report the direct involvement of the proteinase PrtP duplicated CWS domain in adherence to a human small intestine cancer cell line.

Material and Methods

*Bacterial strains and growth conditions. L. lactis* was grown at 30°C in M17 (Difco, West Molesey, United Kingdom) or ½ M17 broth (containing 0.95% β-glycerophosphate, Sigma Chemicals Co., St. Louis, Mo) as standing cultures or on ½ M17 agar plates containing 1.5% (wt/vol) agar. All media were supplemented with 0.5% (wt/vol) glucose, while 5 μg/ml chloramphenicol (Sigma Chemicals Co), or 5 μg/ml erythromycin (Roche Molecular Biochemicals, Mannheim) were added when appropriate.
*DNA techniques and transformation.* Molecular cloning techniques were performed essentially as described by Sambrook *et al*. (1989, Molecular cloning: a laboratory manual, 2$^{nd}$ ed. Cold Spring Harbor, N.Y.). Restriction enzymes, T4 DNA ligase and Expand™ High Fidelity DNA polymerase were obtained from Roche Molecular Biochemicals and used according the instructions of the supplier. Synthetic oligo deoxynucleotides were obtained from Life Technologies B. V. (Breda, The Netherlands). PCR products were purified using the High pure PCR product purification kit (Roche Molecular Biochemicals). *L. lactis* NZ9000 was transformed by electroporation using a gene pulser (Bio Rad Laboratories, Richmond, Calif.) as described by Leenhouts and Venema (1993, Plasmids a practical, 2$^{nd}$ Ed. Oxford University Press, Oxford). Analytical grade chemicals were obtained from Merck (Darmstadt, Germany), or BDH (Poole, United Kingdom).
*Overexpression of fusion proteins.* The DNA fragment encoding the CWS domain and cell wall anchor domain of the proteinase of *L. lactis* Wg2 was amplified from plasmid pGKV552 by PCR with oligonucleotides CWS1 (5'-ATATAAAGCTTGCAAAGTCTGAA AACGAAGG), and CWS2 (5'-CCGTCTCAAGCTCACTATTCTTCACGTTGTTTCCG). The purified 402-bp PCR product was digested with *Hin*dIII/*Esp*3I (underlined) and ligated into the *Hin*dIII site of pNG300, resulting in pNG301. The merozoite surface antigen of *Plasmodium falciparum* strain 3D7 (MSA2) was chosen as localization reporter protein. As this protein contains a eukaryotic GPI membrane anchor, which could obstruct secretion of the fusion proteins, a truncated version of MSA2 (lacking this membrane-spanning domain) was

used. It was amplified using the oligonucleotides MSA2-1 (5'-ACCATGGCAAAAAATGAAAGT AAATATAGC) and MSA2-4 (5'-CGGTCTCTAGCTTATAAGCTTAGAATTCGGGATG TTGCTGCTCCACAG). The PCR product was digested with *Nco*I/*Hin*dIII (underlined) and ligated into the corresponding restriction enzymes sites of pNG301, resulting in pCWS1a. One or two copies of the 264-bp PCR product obtained with oligonucleotides CWS1 and CWS3 (5'-ATTTAAGCTTTTACCGGATGTAAGTTGACCATTACG), encoding the CWS domain, was/were introduced in the *Hin*dIII site of pCWS1a, resulting in pCWS2a and pCWS3a, respectively. Anchor-less variants of the CWS-containing fusion proteins were obtained using oligonucleotides MSA2-1 and Prt.Myc2 (5'-AAGATCTTCTTTGAAATAAG TTTTTGTTCCGTGCT) with pCWS1a, pCWS2a or pCWS3a as templates, respectively. The PCR products were digested with *Nco*I and *Xho*I and ligated into these sites of pNG300, resulting in pCWS1, pCWS2 and pCWS3, respectively.
Nisin induction of the *nisA* promoter upstream of the fusion protein-encoding fragments was performed as described by de Ruyter *et al.* (1996, Appl. Environ. Microbiol. 62: 3662-3667).

*Adherence to eukaryotic cells.* Human Henle cells (Intestine 407 / ATCC CCL6) were cultivated to confluence in RPMI 1640 medium (Gibco) supplemented with 10% fetal calf serum (Gibco), 1% L-glutamine, 1% non-essential amino acids, and penicillin-streptomycin followed by cultivation for two days on diagnostic glass slides (Danlab Oy, Helsinki, Finland). Before adhesion assays, the cells were washed once with PBS. *L. lactis* NZ9000 carrying either pNG304, pNG3041, pCWS1a, pCWS2a or pCWS3a was induced with nisin and grown overnight. After harvesting the cells by centrifugation (5 min, 12000 x g) and washing with PBS, the cells were resuspended in PBS. Equal amounts of bacteria (10 µl, 2 x $10^8$cells/ml) from each suspension were added to the epithelial cells followed by incubation for 1 h at 37°C in a moist chamber. After five washings with PBS at room temperature the slides were fixed for 10 min with methanol and the cells were stained for 5 min with 10 (v/v) Giemsa stain (Oy Reagena Ltd, Kuopio, Finland) and analyzed by light microscopy. The number of lactococcal cells that were in contact with the epithelial cells was counted from ten randomly chosen fields of view taking into account only whole cells. The mean number of adhering bacteria was quantified.

Results

*Adherence to human intestine 407 cells via multiple CWS domains.* Surface located bacterial proteins can be involved in adherence to eukaryotic cells. Some of these adherence proteins show autoaggregation properties. Since we anticipated that the lactococcal proteinase CWS domain has autoaggregation properties, we investigated whether the expression of the CWS domain on the surface of the lactococcal cells can result in adherence to eukaryotic cells. Cells of the human small intestine cancer cell line 407 (Henle) were used in this study. The adhering ability was tested of *L. lactis* NZ9000 cells expressing MSA2 fusions with 1, 2 or three CWS domains, M::C1a, M::C2a or M::C3a respectively (Fig. 16), covalently anchored to the bacterial cell wall via the LPXTG motif of the PrtP cell wall anchor. As controls, two types of NZ9000 strains were used: (i) one secreting MSA2 and, (ii) one that attaches MSA2 to the bacterial cell wall in a non-covalent way through the AcmA repeats (Leenhouts et al. 1999. Antonie van Leeuwenhoek 76: 367-376, patent applications WO99/25836 and EP01202239.8). All cell types were incubated with the Henle cells and only those *L. lactis* NZ9000 cells expressing the covalent anchored fusion proteins with one, two or three CWS domains showed adherence to these epithelial cancer cells of the human small intestine (Fig. 17). The degree of adherence with Henle cells was positively correlated to the number of CWS domain expressed on the lactococcal cells (Table D). MSA2 does not play any role in this binding since the two control strains did not show any adherence to the Henle cells.
This experiment shows that the CWS domain of the lactococcal PrtP can be used for targeting of bacteria and most likely proteins, drugs, hormones, immune modulating signal, delivery vehicles to eukaryotic tumor cells.

Example 3-F. pH Dependent binding of a mutagenized PrtP cell-wall spanning domain.

Mutagenesis of the 60 aa CWS domain of the lactococcal PrtP by error prone PCR and the selection of variants that bind in a pH-dependent manner to a preselected model tumor cell line (human intestine cancer cells).

Materials and methods

*Strains and vectors.* Human intestine cancer cells (Intestine 407 / ATCC CCL6 also designated as Henle cells) were cultivated to confluence in RPMI 1640 medium (Gibco) supplemented with 10% fetal calf serum (Gibco), 1% L-glutamine, 1% non-essential amino acids, and penicillin-streptomycin followed by cultivation in microtiter plates. Vector T7Select10-3 and *E. coli* strains BLT5403 and 5615 (T7Select Phage display System, Novagen) were used for cloning of the mutagenized PrtP CWS domain gene fragments according to the instructions of the supplier. The cloning of the fragment was done such that the 15 amino acid S-tag was included. This tag allows easy immunological detection of the recombinant phages using the S-protein HRP-conjugate (Novagen).

*Mutagenesis.* Random point mutations were introduced by error-prone PCR. The 100-µl reaction mixture contained 10 µl of 10× reaction buffer (10 mM Tris-HCl, 10 mM KCl, 1.5 mM $(NH_4)SO_4$, 0.1% (v/v) Triton X-100) with 2.5 mM $MgCl_2$, 0.2 mM $MnCl_2$, 200 µM dATP and dGTP, 1 mM dTTP and dCTP, 30 pmol each primer, and 5 units of *Taq* polymerase. The PCR schedule was 2 min at 94 °C, followed by 25 cycles of 94 °C for 30 s, 50 °C for 30 s, and 72 °C for 30 s. The product was purified using Qiaquick (QIAGEN) and after digestion, ligated into an expression vector.
*Library construction and screening.* Construction, amplification and storage of the phage library was done according to the instructions of the supplier (Novagen). Screening for binding variants was done by incubation of the phage library in phospate buffered saline (PBS) of pH6 with Henle cells immobilized on culture plates. After binding the Henle cells were extensively washed to remove unbound phages. The phages were released from the Henle cells by using buffer recommended by the suppliers of the phage display kit (Novagen). The released phages were amplified and the binding, washing and amplification procedure was repeated three times. Finally single phages were amplified and individual phage lysates were incubated with Henle cells seeded in microtiter plates in duplo. In one set of microtiter plates the binding was done at pH6 and in the other set of plates the binding was done at pH7.4. After multiple washings an immunological detection was done using the S-protein HRP-conjugate (Novagen). Phages that showed binding at pH6 but not at pH7.4 were identified using a microtiter plate reader.

Results

The biopanning of the recombinant phages resulted in a phage population that bound to Henle cells at pH6. The counter screening at pH7.4 identified several phages that did not show binding at this specific pH. The mutagenized protein anchor DNA insert in one of these phages was isolated and subsequently cloned into a lactococcal expression vector that allows fusion of protein anchors to the reporter MSA2 (see example 3-B). Expression of the fusion protein, designated MSA2::X3, resulted in its secretion. Binding of MSA2::X3 to Henle cells at pH6 and pH7.4 was analyzed by Western blotting. The results clearly showed that the X3 protein anchor binds to Henle cells at pH6 but not at pH7.4. Thus, pH dependent targeting proteins are obtained using the lactococcal PrtP CWS domain.
This example also demonstrates induced availability (by pH) of a mutagenized PrtP CWS domain for binding to a target. In the example a mutagenized Anchor is used to target a model substrate (human small intestine cancer cells). In some applications mutagenized Anchors are coupled to delivery vehicles (e.g. liposomes, hydrophobin particles, etc.) and are targeted to micro-organisms or specific animal or human organs or tumors.

EXAMPLE 4. Use of hydrophobin vesicles as delivery vehicles

Hydrophobins are self-assembling proteins which are capable of forming an amphipathic film on an interface. By choosing the proper conditions the film can be formed in different shapes and manipulated to have alternative characteristics. Examples of interfaces can be Teflon - water, where the hydrophobic Teflon is coated with a hydrophilic layer, and water - oil. For a recent review see Wösten (1) 2001, Annu.Rev.Microbiol. 55:625-646).
This example describes assembly of hydrophobins in to small vesicles that can be used as delivery vehicles. Hydrophobins are known to have low immunogenicity, which makes vesicles consisting of hydrophobins ideally suitable for drug delivery.

Methods

Hydrophobin SC3 was purified from the culture medium of strain 4-40 of *Schizophylum commune* (CBS 340.81) as described by Wessels and Wösten et al (Wessels, J. G.,1997, Adv.Microb.Physiol 38:1-45; Wösten, H. A. B. et al., 1993, The Plant Cell **5**:1567-1574). Before use, the freeze-dried SC3 was disassembled with pure TFA and dried in a stream of nitrogen. The monomeric protein was then dissolved in 50 mM phosphate buffer or water.
Hydrophobin vesicles can be made with the hydrophobic site inwards, by coating oil droplets, or with the hydrophilic site inwards, by coating water droplets (Wösten, H. A. et al., 1994, EMBO J. 13:5848-5854).
Coating of oil droplets was achieved by emulsifying 10 µl of an oil (kitchen grade olive oil, mineral oil or organic solvents that do not mix with water) in 300 µl water by sonication and adding 300 µl of an aqueous solution of hydrophobin (200 µg $ml^{-1}$). Alternatively, the oil was directly emulsified in the hydrophobin solution and 300 µl water was added.
After overnight incubation at room temperature or 60°C in the presence of 0.02% $NaN_3$, the emulsions were centrifuged at 10,000 g for 30 min and the oil droplets washed four times with water by centrifugation to remove monomeric hydrophobin. Oil droplets coated with assembled hydrophobin were then resuspended in a small volume of water to study stability, size and leakage of encapsulated substances.
For assembly of hydrophobins on oil droplets of a uniform size a micro filtration membrane was used. At the hydrophilic side of the membrane an aqueous solution of hydrophobin (100 µg $ml^{-1}$) was applied and at the hydrophobic side of the membrane the oil with the substance of interest was present. The oil was forced through the filter into the aqueous

solution by pressure, generating an emulsion of precisely sized droplets of as small as 0.1 µm. After overnight incubation at room temperature or 60°C, the emulsion was washed with water and concentrated as described above. Substances to be included in the vesicles are added to the waterphase while generating these vesicles.

For assembly at the water/oil interface of an emulsion of water in oil, 10 µl of an aqueous solution of hydrophobin (1 mg $ml^{-1}$) was emulsified in 600 µl of oil by sonication. After overnight incubation, the mixture was centrifuged at 10,000 g for 30 min in order to collect the aqueous phase. The emulsion was washed with oil by centrifugation and was further characterized by electron microscopy.
Alternatively, 100 µg of freeze-dried monomeric hydrophobin was dispersed in 600 µl oil by sonication or agitation. 10 µl of water was emulsified in the oil/hydrophobin suspension by sonication. After overnight incubation the emulsion was centrifuged at 10,000 g for 30 min, washed four times with oil by centrifugation to remove excess of hydrophobin. The concentrated emulsion was studied for stability, size and leakage.
Furthermore, the water in oil emulsions of uniform droplet size were made with the micro filtration membrane as described above.
Rodlet structures or the so-called β-sheet form of hydrophobins are spontaneously formed at the oil/water interface. Stability of the emulsion and the size of the coated droplets were assessed by electron microscopy and atomic force microscopy according to standard techniques.
Leakage of the vesicles was studied with an efflux assay with a fluorescent model drug, calcein. The percentage release of calcein from the vesicles was calculated from the dequenching of calcein fluorescence according to the following equation:

$$\% \text{ Release} = (F_x - F_0) / (F_t - F_0) \times 100$$

Where $F_0$ is the fluorescence intensity at zero time incubation, $F_x$ is the fluorescence at the given incubation time-points and $F_t$ is the total fluorescence, obtained after lysis by shearing of the vesicles. Fluorescence was monitored with a SLM 500 spectrofluorimeter in a thermostatted cuvette (1 mL) at 37°C, under constant stirring. Excitation and emission wavelengths were, respectively, 490 (slit 2 nm) and 520 nm (slit 4 nm). The experiments were performed at hydrophobin concentrations of approximately 2.5 µM. The hydrophobin vesicles were separated from free calcein by using Sephadex 50 column chromatography equilibrated with PBS (160 mM NaCl, 3.2 mM KCl, 1.8 mM $KH_2PO_4$, 0.12 mM $Na_2HPO_4$, 1.2 mM EGTA, pH 8.0), which was isotonic to the calcein-containing buffer.

EXAMPLE 5. Coating of liposomes with hydrophobins to manipulate stability and/or leakage.

Liposomes are a commonly used vehicle for the delivery of drugs. Their characteristics are quite suitable for this purpose, though there are several features that could be improved. One of those is the stability of liposomes and therefore the leakage of the drug. The fluidity of the liposome membrane makes it susceptible for rupture, which can be avoided by adding a support to the membrane. In the present invention hydrophobins were shown to be ideal support for the lipids, which results in a more stable and/or less leaky liposome.
Hydrophobins are self-assembling molecules which can form a highly amphipathic film on a hydrophobic-hydrophilic interface. In nature this film is used, for example, to cover aerial structures of fungi, which results in a hydrophobic surface, with all the effects on the organism and its environment.
The number of similar proteins is increasing rapidly and therefore also the diversity of specific characteristics.
Introducing hydrophobins to liposomes increases the stability and/or decreases leakage problems of liposomes. The diversity of hydrophobins and liposomes make it possible to adjust the properties to virtually any specific application. This example describes the formation of liposomes stabilized with hydrophobins, the verification of the resulting compartment and the assay used to measure the stability and leakage.

Methods

Hydrophobin SC3 was purified from the culture medium of strain 4-40 of *Schizophylum commune* (CBS 340.81) as described by Wessels and Wösten et al. (Wessels, J. G, 1997, Adv.Microb.Physiol 38:1-45; Wösten, H. A. B. et al., 1993, The Plant Cell 5:1567-1574). Before use, the freeze-dried SC3 was disassembled with pure TFA and dried in a stream of nitrogen. The monomeric protein was, subsequently, dissolved in 50 mM phosphate buffer or water.
Dry lipid mixtures were prepared by co-dissolving lipids (Avanti Polar Lipids, Alabaster, AL) in chloroform, in weight-fractions as indicated in the experiments, and removing the chloroform by evaporation under vacuum for 4 h. All acyl chains of the synthetic lipids were of the type, dioleoyl, unless indicated otherwise. The dried lipid film was dissolved (20 mg/mL) in 50 mM potassium phosphate, pH 7.0, followed by three freeze/thaw cycles and extrusion through polycarbonate filters to form unilamellar liposomes with a diameter of approximately 100 nm.

Formed liposomes were coated with hydrophobins by incubating the liposomes overnight in an aqueous solution of hydrophobins (200 µg $ml^{-1}$) at room temperature or 60 °C in the presence of 0.02 % $NaN_3$.
Alternatively, the hydrophobins were added before the freeze/thaw cycles during the preparation of the liposomes. This method resulted in coated inverted hexagonal liposome structures which were much more stable compared to inverted hexagonal liposome structures in the absence of hydrophobins.
The coating could be manipulated by adding ethanol, propanol or butanol to the hydrophobin solution, decreasing the hydrophilicity. Three parameters could be manipulated in the formation of the hydrophobin liposome structures: the lipid composition, the type of hydrophobin and the hydrophobicity of the buffer.
Another method of obtaining hydrophobin coated liposomes consisted of hydrophobins in a water/oil emulsion (see Example 4), which was dialyzed in a dialysis tube in a lipid containing solution to obtain lipid covered hydrophobin vesicles or internally coated liposomes.

The formed liposomes were analysed with electron microscopy and atomic force microscopy, which visualized the assembly of the rodlet structure of the hydrophobins on the surface. The composition of the hybrid vesicles was analysed with discontinuous sucrose gradients as described (Knol, J. et al.,1998, Biochemistry 37:16410-16415).
The stability of the vesicles was measured with an efflux assay with a fluorescent model drug, calcein. The drug was introduced when the liposomes were being made, by dissolving the calcein in the buffer. The percentage release of calcein was calculated from the dequenching of calcein fluorescence as described in Example 4.

EXAMPLE 6. Use of the Protein Anchor to target liposomes/polymer particles/hydrophobin vesicles complexes to Gram-positive cells

A Protein Anchor is a cell-wall binding domain (Anchor) of, for example, the major cell-wall hydrolase AcmA of *Lactococcus lactis*, a Generally Recognized As Safe (GRAS) Gram-positive bacterium. This Protein Anchor consists of 3 homologous repeats of 45 amino acids that contain a specific consensus sequence (see patent applications WO99/25836 and EP 01202239.8), separated by intervening sequences of about 30 amino acids that are highly enriched for serine, threonine and asparagines residues. This protein Anchor has the ability to attach from the outside to a wide variety of Gram-positive (G+) bacteria, also when part of a chimaeric fusion protein. We use the Protein Anchor in therapeutic applications as a device to target pathogenic bacteria in order to inactivate them. Inactivation may be achieved by coupling antibodies, cytokines (signaling molecules for the immune system) or drugs to the Protein Anchor. In another approach the drugs or other compounds are incorporated in nano- or micro delivery-vehicles to which the Protein Anchor is attached in order to direct these vehicles to a specific target, in the case of the unmodified AcmA Protein Anchor mostly G+ bacteria. Mutagenized Protein Anchors that show pH-dependent binding and/or binding to specific eukaryotic cells are obtained in a similar way as described under Example 3. They can be used in combination with these delivery vehicles to target specific human or animal cells providing induced availability of the substance of interest.

Example 6-A. Targeting of liposomes through the use of the Protein Anchor to Gram-positive bacteria

The coupling of the Protein Anchor to liposomes and sterically stabilized liposomes is described. The liposomes contain calcein as reporter drug. The liposomes with the coupled Protein Anchor displayed on the surface, were then incubated with TCA pretreated *L. lactis* cells (ghost cells). After washing the ghost cells to remove unbound liposomes, binding to the ghost cells was demonstrated by measuring an increase in fluorescence in a fluorometer and microscopically by using a fluorescence microscope.

Materials and Methods

The Protein Anchor (cA) fusion used contained in the N-terminal domain a unique cysteine (cys) that is used as the functional element for the coupling to lipids in the liposome, in addition it contained an epitope than can be used for detection (myc-epitope). This Protein Anchor fusion (cys::myc::cA) was designated PA3. Protein PA3 was produced in *L. lactis* using vector pPA3. Plasmid pPA3 is based on the nisin inducible expression vector pNZ8048 (Kuipers et al. 1997. Tibtech 15: 135-140) and contains a modified multiple cloning site in which the cysteine and c-myc reporter epitope was cloned. An in frame fusion of this reporter was made with at the 5'-end the lactococcal Usp45 signal sequence, and at the 3'-end the AcmA protein anchor sequence. Growth of *L. lactis* and induction for expression was as described before (Kuipers et al. 1997. Tibtech 15: 135-140). Isolation of PA3 from the culture medium was done by using a Sepharose SP cation exchange column (Pharmacia). The culture supernatant was loaded on the column at pH5.8. The column was washed with phosphate buffer pH5.8 containing 25mM NaCl. Elution of the protein was obtained with the same buffer containing 100mM NaCl. Construction of liposomes loaded with calcein and detection of calcein was as described in Example 1. Coupling of PA3 to liposomes containing an activated lipid was done as described

before (Papahajopoulos et al. 1987. Ann NY Acad Sci 507: 64-74, Maruyama et al. 1990. Proc Natl Acad Sci USA 87: 5744-5748). Coupling of PA3 to liposomes sterically stabilized with PEG-PE was according to the method described by Ahmad et al. (1993, Cancer Res 53: 1484-1488). Preparation of the ghost cells and binding of the liposomes with coupled PA3 on the surface to ghost cells was done as described in patent application EP 01202239.8. Fluorescence was measured using a SpectroFluorometer (SLM500) and fluorescence was visualized using a Zeiss fluorescence microscope.

Results

The three types of calcein loaded liposomes, one in which the PA3 anchor was coupled to the lipid, one that has PA3 coupled to the PEG-PE and control liposomes (with or without PEG-GE), were used to analyze targeting to lactococcal ghost cells. After binding, the ghost cells were extensively washed and analyzed in a Spectrofluorometer. Fluorescence, which is an indication for the presence of liposomes, was only observed in the case that PA3 coupled liposomes were used. The type of coupling, to the lipid or to the PEG-PE, had no influence on the binding. The presence of the targeted liposomes on the surface of the ghost cells was confirmed using fluorescence microscopy. These results show that the AcmA-type Protein Anchor can be used to target liposomal delivery vehicles to bacteria. The liposomes may either provide slow release or can be induced to release the drugs upon an external signal (see Example 1). This may have applications in combating pathogenic bacteria. Furthermore, it is likely that mutagenized AcmA-type Protein Anchors similar to those described in Example 3 can be used to target specific human or animal cells. Alternatively, variants of the PrtP CWS domain, also described in Example 3, can be used for this purpose.

Example 6-B. Targeting of polymer particles through the use of the Protein Anchor to Gram-positive bacteria

The coupling of the Protein Anchor to a polymer particle is described. The particles contain an organogel with a reporter drug (calcein). The Protein Anchor was displayed on the particle surface, which were then incubated with TCA pretreated *L. lactis* cells (ghost cells). After washing the ghost cells to remove unbound polymer particles, binding of calcein loaded polymer particles to the ghost cells was demonstrated.

Materials and Methods

Production and isolation of PA3 as described under Example 6-A. Preparation of ghost cells and binding conditions was as described before (patent application EP 01202239.8). Preparation of polymer particles consisting of methyl methacrylate or diethyleneglycol dimethacrylate was described before (Graham NB and Cameron A. 1998. Pure Appl Chem 70: 1271-1275). Crosslinking reagents: N-[*p*-Maleimidophenyl]isocyanate PMPI (couples sulfhydryl groups to hydroxyl groups); N-ε-Maleimidocaproic acid EMCA (couples sulfhydryl groups to amino groups); N-β-Maleimidopropionic acid BMPA (couples sulfhydryl groups to amino groups [peptide bond]); N-[β-Maleimidopropionic acid] hydrazide BMPH (couples sulfhydryl groups to aldehyde groups). Coupling conditions of PA3 to the polymer particles were according to the specifications of the supplier (Pierce, IL, USA). The particles were then loaded with organogel containing calcein (as described in Example 7). Specific binding to the bacteria was demonstrated by detection of calcein as described in Example 6-A.

Results

Two types of polymer particles loaded with organogel and calcein, one to which the PA3 anchor was coupled and one control to which no PA3 had been coupled, were used to analyze targeting to lactococcal ghost cells. After binding, the ghost cells were extensively washed and analyzed in a Spectrofluorometer. Fluorescence, which is an indication for the presence of the polymer particles, was only observed in the case that PA3 coupled polymer particles were used. The presence of the targeted polymer particles on the surface of the ghost cells was confirmed using fluorescence microscopy. These results show that the AcmA-type Protein Anchor can be used to target polymeric delivery vehicles filled with organogels to bacteria. The organogels can provide slow release or can be induced to release the drugs upon an external signal (see Example 7). This has applications in combating pathogenic bacteria. Furthermore, mutagenized AcmA-type Protein Anchors similar to those described in Example 3 can be used to target specific human or animal cells. Alternatively, variants of the PrtP CWS domain, also described in Example 3, can be used for this purpose.

Example 6-C. Targeting of hydrophobin vesicles through the use of the Protein Anchor to Gram-positive bacteria

The coupling of the Protein Anchor PA3 to SC3 vesicles loaded with calcein is described. The SC3 vesicles

with the coupled PA3 displayed on the surface were then incubated with TCA pretreated *L. lactis* cells (ghost cells). After washing the ghost cells to remove unbound vesicles, binding of calcein loaded hydrophobin vesicles to the ghost cells was demonstrated.

Materials and Methods

Production and isolation of PA3 as described in Example 6-A. Preparation of ghost cells was as described before (patent application EP 01202239.8). Preparation of SC3 vesicles loaded with calcein as reporter drug was according to the method as described in Example 4 . Coupling of PA3 to the monomeric form of SC3 or to the SC3 vesicles was done by coupling the carbohydrates of SC3 to the unique cysteine in PA3 using the cross-linking reagent N-β-Maleimidopropionic acid (BMPH), which was used according to the instructions of the supplier (Pierce, IL, USA). The hydrophobin vesicles were then incubated with ghost cells. Specific binding to the bacteria was demonstrated by detection of calcein as described in Example 6-A.

Results

The calcein loaded hydrophobin vesicles to which the PA3 anchor had been coupled and the control to which no PA3 had been coupled, were used to analyze targeting to lactococcal ghost cells. After binding, the ghost cells were extensively washed and analyzed in a Spectrofluorometer. Fluorescence, which is an indication for the presence of the hydrophobin vesicles, was only observed in the case that PA3 coupled hydrophobin vesicles were used. The presence of the targeted hydrophobin vesicles on the surface of the ghost cells was confirmed using fluorescence microscopy. These results show that the AcmA-type Protein Anchor can be used to target hydrophobin vesicles to bacteria. The hydrophobin vesicles either provide slow release or can be induced to release the drugs upon an external signal when they are filled with a responsive organogel (see Example 7). This has applications in combating pathogenic bacteria. Furthermore, mutagenized AcmA-type Protein Anchors similar to those described in Example 3 can be used to target specific human or animal cells. Alternatively, variants of the PrtP CWS domain, also described in Example 3, can be used for this purpose.

EXAMPLE 7. Use of responsive organogels as delivery vehicles

Organogelators are small organic compounds that, already at low concentrations, can form gels with solvents ranging form hexane to water (F.M. Menger, K.L. Caran, J. Am. Chem. Soc, 2000, 122, 11679, J.H. Jung, M. Amaike, K. Nakashima, S. Shinkai, J. Chem. Soc, Perkin Trans. 2, 2001, 1938), via self-assembly of the organo/hydrogelator through highly specific non-covalent interactions (J. van Esch, F. Schoonbeek, M. de Loos, M. Veen, R.M. Kellogg, B. L. Feringa, NATO ASI Series, Kluwer Academic Publishers, 527(1998) 223, P. Terech, R.G. Weiss, Chem. Rev., 97 (1997) 3133). The self-assembly (gel formation) is in equilibrium with the monomer although the equilibrated state lies far to the left (M is monomer, see Scheme below).

$$\text{MMMMMMMMM} \leftrightarrows \text{MMMMMMMM} + \text{M}$$

The gelation process is fully reversible, and dependent on the structure of the organo/hydrogelator, can be modulated by several signals e.g. temperature, light, pH, magnetic fields, electric currents, ultrasound and chemical or biological compounds (N.A. Peppas, P. Bures, W. Leobandung, H. Ichikawa, Eur. J. Pharm. Biopharm. 50 (2000) 27 and references cited therein). We have used organo/hydrogelator molecules as gelling tags to which the substance to be delivered is coupled and, subsequently, immobilized in a gel through self-assembly of the gelling tag.

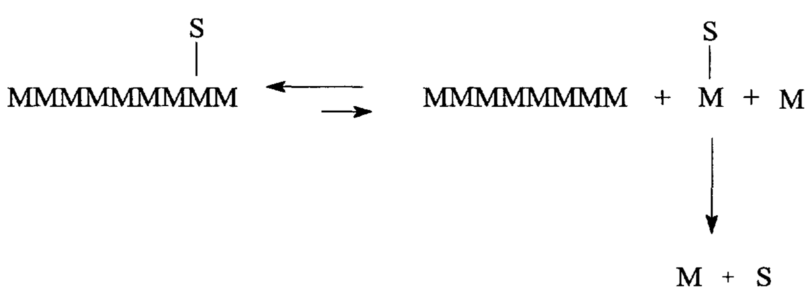

Release of the substance now requires two steps which can be modulated independently: first the disassembly of the organo/hydrogel to a molecular solution and, secondly, the fission of the non-covalent or covalent bond between gelling tag and substance to be delivered.

A. The vehicle is a micro-particle (organo/hydrogel, e.g. composed of a small peptide, carbohydrate, bis-urea derivative, and cis, cis 1,3,5-trisubstituted cyclohexane derivative) or micro-droplet (partially stabilized by a surfactant or emulsifier) forming a separate phase with respect to the aqueous environment to which it is delivered. The micro-phase is stabilized as a gel and the substance of interest is bound within the matrix. The gel has, subsequently, been disrupted by an external signal as described above. If the substance of interest (S) is covalently linked to the matrix the linkage consists of a labile ester or peptide moiety, cleaved readily by hydrolytic enzymes at the target site. In a further example these micro-particles or micro-droplets have been successfully functionalized with a targeting means to deliver the particle/droplet to a site of interest.

B. In a further embodiment, the organo/hydrogel containing the substance of interest has been protected from the environment by encapsulation in a porous micro-capsule or hollow particle, e.g. a liposome, particle, vesicle (Sunfish amphiphiles, see Example 2) or polymer. Alternatively, the organo/hydrogel has been coated with polymers (hydrophobins, nylon, peptides etc). After application or delivery of the above-described vehicle to the target site, a signal (see above) causes dissolution of the gel.
In the case of a porous micro-capsule, the conjugate molecules are now available e.g. to hydrolytic enzymes present at the target site, and subsequent hydrolysis of the linkage liberates the substance of interest. Alternatively, the hydrolytically labile linkage between substances has been replaced by any other linkage which can be easily and selectively cleaved in a (catalytic or enzymatic) chemical or photochemical event. As an example, the gel also retains the catalyst or enzyme necessary to disrupt the linking moiety, coupled either via non-covalent or covalent interactions within the gelling phase. If the substance of interest is not covalently linked to the matrix, elaboration of the substance of interest has been performed by increased mobility due to disruption of the supramolecular system.
In another embodiment, a hollow particle (non-porous) has been disrupted at first by a signal (e.g. enzymatic hydrolysis) elaborating the organo/hydrogel that is disrupted further as described above. Alternatively, the organo/hydrogel has been disrupted within the particle first, after which event disruption of the particle has been achieved. In the preferred embodiment, both occurrences have been triggered by the same signal. The conjugate molecules are now available to liberate the substance of interest.

C. In a further embodiment, the organo/hydrogel containing the substance of interest has been protected from the environment by encapsulation in a porous micro-capsule or (hollow) particle, e.g. a liposome, particle and/or vesicle containing a (responsive) MscL protein channel. Induced opening of the MscL channel leads to the release of small molecules (monomers). As a result the gel shrinks and encapsulated substances of interest are slowly released.

EXAMPLE 8. Method for the rapid release of molecules at a target site with the help of an applied electric field.

Decondensation of anionic polymers under the influence of an electric field results in a swelling of the polymer matrix. Swelling can be achieved at field strengths of 2.5V across a 5 $\mu$m vesicle which corresponds to a field strength of 5000Vcm$^{-1}$. This is a factor of 10 less than the field strength needed to induce leakage of ions through the ion channels. In the confined volume of a liposome the swelling is translated into a membrane stress or internal pressure of approximately 10 bar. This is sufficient to induce the opening of the MscL osmo-regulated channel and the release of soluble small molecular components.

Material and Methods

In vitro release profiles of a model drug were determined. Liposomes, as in Example 1 with and without MscL-6His were prepared in the presence of the dye, calcein and heparin sulfate, a natural polymer found in secretory granules. A liposome was inserted into the tip of a glass pipette with the application of suction. The pipette voltage relative to the bath was controlled by a current-to-voltage converter. The solution in both bath and the pipette was buffered to 6.5 with histamine dichloride and phosphoric acid. We used a Axopatch 200B to apply voltages and to sample the currents at 2.5 ms intervals. Swelling was induced by the application of a negative potential. To measure the swelling we imaged the isolated liposomes and analysed them as described (Fernandez, J.M. et al., 1991, Biophys. J. 59: 1022-1027, and Monck, J.R. et al., 1991, Biophys. J. 59: 39-47).

Results

The heparin sulfate matrix swelled instantaneously and predictably with the application of a negative voltage as reported (Nanavati, C. and Fernandez, J.M., 1993, Science 259: 963-965). The swelling was accompanied by an instantaneous current and a visible diffusion of the calcein dye into the bath. When the voltage was turned off, the current and swelling returned to control levels but the calcein remained in the bath.

Variations on this theme are:

- The gel network was covalently cross-linked to different extend, resulting in different drug release profiles.
- The gelation process is fully reversible, depending on the type of gel, and can be modulated by several signals e.g. temperature, light, pH, magnetic fields, electric currents, ultrasound and chemical or biological compounds.

EXAMPLE 9. Responsive Amphiphiles: Induced availability of substances by means of morphological alterations

This example embodies the enhanced availability of substances of interest following induced morphological changes of the macromolecular vehicle system. A sequential morphology-altering pathway has been implemented in order to direct morphological behaviour e.g. from bilayer vesicular into micellar into none or vice versa. The same sequentially modified vehicle performs all steps. The following example describes the concept: bilayer vesicles (comprising compound 1) are used for transport; after hydrolysis releasing R2, micelles (comprising compound 2) are formed inducing endocytosis and finally the second hydrolysis (yielding compound 3 and releasing R1) induces the actual release by destroying the micellar structure.

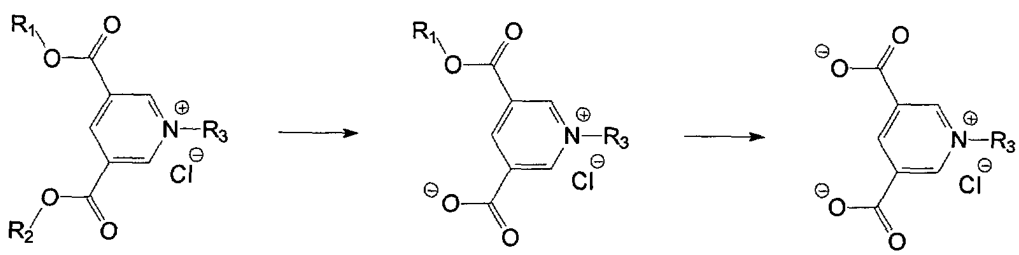

1 2 3

Hydrolysis is used as a signal, although other signals (e.g. reduction potential, light, H-bridges) can be used as well. Morphology changes include vesicular into micellar into none, but changes from lamellar into hexagonal into micellar into none or vice versa as well.

EXAMPLE 10. Carbohydrate-based amphiphiles or alternatives as delivery vehicles

A. Sunfish amphiphiles have been modified with $R_1$ or $R_2$ tails (see Example 13) consisting of a short alkyl chain equipped with (a) carbohydrate(s) providing, amongst others, targeting ability for (specific) cell membranes. The hydrolytically labile acetal functionality has been shown to, upon hydrolysis, improve the delivery efficiency of materials of interest. Also, carbohydrates modified in order "to bridge" tails have been shown active in delivery processes. Two structural examples are given below.

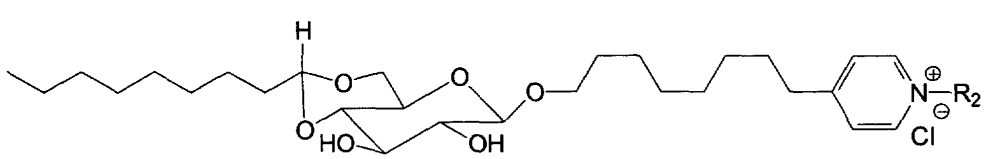

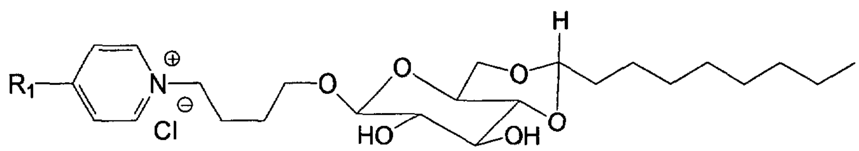

The materials behaved according to the Sunfish protocol in which backfolding takes place and bilayer vesicles are formed. The carbohydrate functionalities provide, amongst others, targeting ability. Subsequent unfolding of "the tails" disturbs the cell membrane leading to endocytosis. In the endosome, lowered pH conditions results in hydrolysis of the acetal moiety, destroying the morphological entity releasing the contents.

Gemini Sunfish amphiphiles equipped with carbohydrate targeting means have been prepared according to the underlying scheme, in which the carbohydrate serves as a targeting means and the bridging moiety consists either of simple alkyl or functionalized bridges.

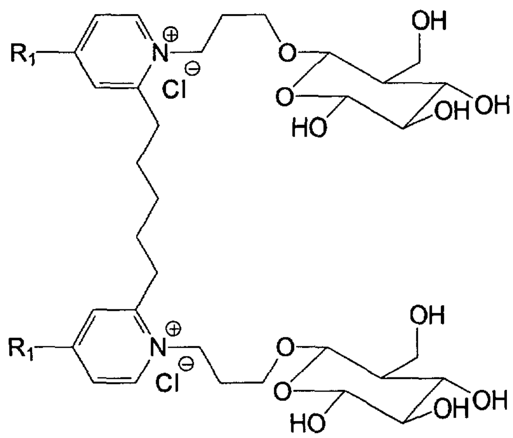

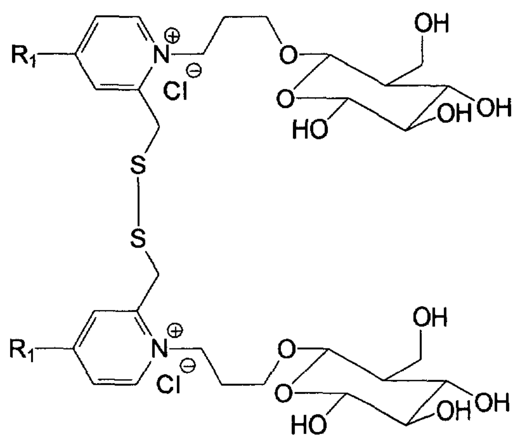

In the example shown above, the disulfide assists in the release of the contents of the vehicle from the endosome after reduction.

B. The Fielden concept [Fielden, M.L., Perrin, C., Kremer, A., Bergsma, M., Stuart, M.C., Camilleri, P., Engberts, J.B.F.N., Eur. J. Biochem. 2001, *268,* 1269] has been modified. Firstly, other carbohydrates such as aldoses and pentoses have been applied successfully increasing transfection efficiency and targeting ability. Secondly, multisaccharides have been applied *e.g.* lactose, maltose and maltotriose. Thirdly, modified carbohydrates have been applied, among this array are ethers, esters and oxidised carbohydrates. A very promising modification is the application of 2-amino-glucose based building blocks.

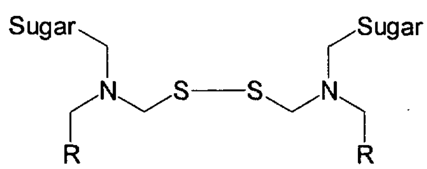

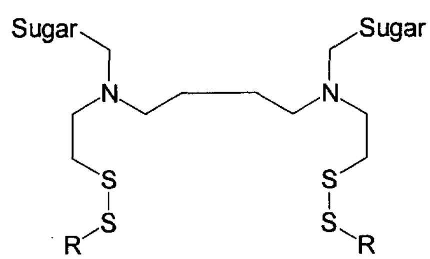

C. For the delivery of drugs (transduction), drugs have been covalently coupled via (several) degradable linking procedures including the use of 3-keto-glucoses and amino acid spacing units (esters, amides, imines etc).

Example 11

Some examples of suitable gelators and thickening agents and a method of preparation thereof.

Synthesis of starting materials (literature procedures)

a) Potassium hydrogen D-glucarate. - Conc. Nitric acid (258 ml, 4 moles) was heated to 50-55 ° C with constant stirring (mechanical stirrer), and $NaNO_2$ (0.1 g, 1.4 mmole) was added. Glucose (180 g, 1 mole) was added in portions and the temperature was maintained at 50-60 ° C by cooling with cold water. The reaction was allowed to continue for 1 h at this temperature and subsequently cooled to room temperature. 45% NaOH potassium hydride was carefully added with stirring to obtain a pH of 10-11. After cooling to room temperature, the solution was adjusted to pH = 3.4 with conc. nitric acid. The mixture was allowed to stand overnight and the precipitate was collected by filtration. The product was washed with cold 25% ethanol and dried in the air to yield potassium hydrogen D-glucarate (98.3 g, 0.42 mole, 42%).

b) D-Glucaric acid (lactone).- Potassium hydrogen D-glucarate (10.00 g, 43 mmole) was added to boiling $H_2O$ (100 ml). Active coal was added and the suspension is stirred for 0.5 h. After filtration the hot solution was added to a column of Dowex H+ (4x8, 200-400 mesh) and washed with $H_2O$ (200 ml) and evaporated. Yield glucaric acid (lactone) 7,35 g.

c) D-Glucaro-6,3-lactone.- The 4.06 g crude glucaric acid (lactone) is seeded with D-glucaro-6,3-lactone (0.10 g). After 3 days the crystalline mass is triturated with acetone, the white solid collected by filtration and air-dried to yield D-glucaro-6,3-lactone (3.30 g, 17.2 mmole).

d) sodium-D-glucarate 6,3-lactone.-NaAc.$_3$.$H_2O$ (2.34 g, 17.2 mmol) was added to a solution of D-glucaro-6,3-lactone (3.30 g, 17.2 mmole) in $H_2O$ (10 ml). White crystals began to form within 1 h. The solution was set aside for 20 h and the crystalline product was collected by filtration and washed with aceton (3 x 5 ml.). Additional product crystallized from the aqueous aceton and the crystals obtained were subsequently washed with aceton (3x5 ml). The crystallization and filtration process was repeated once more and the combined white solids were air-dried to yield sodium-D-glucarate 6,3-lactone (2.68 g, 12.5 mmole, 73%).

e) Diethyl-galacterate.- A mixture of galactaric acid (Aldrich, 30 g, 143 mmole), ethanol (750 ml) and conc. $H_2SO_4$ (6 ml) is refluxed for 24 h. The pale yellow solution is filtered and cooled in the refrigerator. Yield 27.74 g (104 mmole, 73%). $^{13}$C-NMR ($D_2O$, in ppm): d 11.73, 69,24, 68.83, 69.24, 173.23.

Synthesis of dicyclohexyl glucaramide.

Monopotassium D-glucarate (2.50 g, 10 mmol) is added to a solution of acetyl chloride in MeOH (12,5 ml, 1.68 M) and refluxed for 3 hours. After cooling to room temperature salts were removed by filtration, and the filtrate was concentrated under reduced pressure. The syrup obtained was dissolved in MeOH (15 ml) and neutralized with triethylamine until the solution became basic (pH paper. pH = 8)) and then additional triethylamine (0.5 ml) was added to the solution. Cycloheylamine (2.47 g, 25 mmol) was added and after 4 hours stirring the precipitate was filtered off and crystallized from EtOH. The product was recrystallized from ethanol (yield 1.64 g, 4.4 mmol, 44%).

Synthesis of dicyclododecyl glucaramide.

**[0169]** - Monopotassium D-glucarate (2.50 g, 10 mmol) is added to a solution of acetyl chloride in MeOH (12,5 ml, 1.68 M) and refluxed for 3 hours. After cooling to room temperature salts were removed by filtration, and the filtrate was concentrated under reduced pressure. The syrup obtained was dissolved in MeOH (15 ml) and neutralized with triethylamine until the solution became basic (pH paper. pH = 8)) and then additional triethylamine (0.5 ml) was added to the solution. Cyclododecylamine (4.57 g, 25 mmol) was added and after 4 hours stirring the precipitate was filtered off and crystallized from EtOH. The product was recrystallized from 2-propanol (yield 2.26 g, 4.2 mmol, 42%).

Synthesis of dicitronellyl glucaramide.

-Citronellylamine (5.00 g, 32.4 mmole) was added to a solution of D-Glucaric acid (lactone) (Example 2, 2.90 g, about 14.5 mmole) in EtOH (40 ml). After 20 h stirring the precipitate was filtered off and recrystallized from 2-propanol to yield dicitronellyl glucaramide (2.30 g, 4.75 mmole, 33%).

*Synthesis of didodecyl galactaramide.*

-Dodecylamine (3.75 g, 20.5 mmole) was added to a solution of diethyl galactarate (2.66 g, 10 mmole) in EtOH (50 ml). After 72 h stirring the precipitate was filtered and thoroughly washed with ethanol, aceton, and ether to yield didodecyl galactaramide (4.87 g, 8.9 mmole, 89%).

Solvent scope of N,N'-dialkylaldaramides or N,N'-dialkylpentaramides

| Compound | *C*6**Glu**C | *C*12**Glu***C* | *Cit***Glu***Ci* | *12***Gal***1* | *C6***Glu***C1* |
|---|---|---|---|---|---|
| | *6* | *12* | *t* | *2* | *2* |
| Cyclohexane | g. | g. | g. ,c*. | n.s. | -. |
| Toluene | g. | g. | p. | n.s. | -. |
| n-Butylacetate | g. | g. | p. | n.s. | -. |
| 1,2-Dichloro-ethane | g/c. | g. | s. | n.s. | -. |
| Ethanol | g/c. | g/c. | s. | n.s. | -. |
| Dimethylsulfoxide | g/c. | g/c. | s. | g. | -. |
| water | n.s. | n.s. | c. | n.s. | -. |

Concentration range of N,N'-dialkylaldaramides or N,N'-dialkylpentaramides

| Compound | *C*6**Glu**C | *C*12**Glu***C* |
|---|---|---|
| | *6* | *12* |
| Cyclohexane | <2.5%. | <5%. |
| Methyllaurate | <2.5%. | <5%. |
| Dow Corning 702 | <2.5%. | <50% |
| Toluene | <2.5%. | <50%. |
| n-Butylacetate | <2.5%. | <50%. |
| 1,2- Dichloroethane | <5.0%. | <50%. |

Example 12

Examples of suitable light-switchable gelators and a method of preparation thereof.

**1: 1,2-Bis(5'-formyl-2'-methylthien-3'-l)cyclopentene (A)**

n-Butyllithium (7.85 ml of 1.6M solution in hexane, 12.56 mmol) was added to a stirred solution of 1,2-bis(5'-chloro-2'-methylthien-3'-l)cyclopentene (compound Z in Figure 20) (1.97 g, 5.98 mmol) in anhydrous THF (20 ml) under nitrogen at room temperature. One hour after the addition the reaction mixture was quenched with anhydrous dimethylformamide (0.97 ml, 12.56 mmol). The mixture was stirred then for an additional hour at room temperature, before it was poured into HCl (2N, 50 ml). The mixture was extracted with diethyl ether (3 x 25 ml). The combined organic layers were washed with saturated sodium bicarbonate solution (2x 25 ml) and $H_2O$ (1 x 25 ml), and dried ($Na_2SO_4$), filtered and evaporated in vacuo to yield a brown solid (1.89 g, 90%). Chromatography of the solid over silica gel (hexane/ethyl acetate = 9/1) afforded the compound as a brown/orange solid (0.98 g, 52%). In the first step also other lithiating agents can be used, as a solvent all ethers can be used but preferably THF and diethyl ether. The temperature at which the lithiation reaction can be performed ranges from -80°C to 50°C, but preferably at 0°C.

**1,2-bis(5'-carboxylic acid-2'-methylthien-3'-1)cyclopentene (B)**

Silver oxide was used to oxidize dithienylcyclopentene bisaldehyde (compound A)which was prepared as described under **compound A.** This was done in situ by adding $AgNO_3$ (1.64 g, 9.6 mmol) to a solution of NaOH

(0.75g, 18.7 mmol) in $H_2O$ (15 ml). Silver oxide immediately precipitated. This suspension was then added to **compound A** (0.74 g, 2.34 mmol) and refluxed for 1h, subsequently filtered over a glass filter and rinsed with hot water. The filtrate was cooled and acidified with 2M HCl in an ice bath. The compound precipitated and was filtered over a glassfilter (G4). The residual water was azeotropically removed with toluene to yield an off-white solid (0.51g, 62%).

**1,2-Bis(5'-(anilinocarbonyl)-2'-methyl-thien-3'-yl) cyclopentene (C)**

Dicarboxylic acid-thienylcyclopentene derivative (Compound B, 0.5 g, 1.44 mmol), which was prepared as described under **compound B**, was suspended in $CH_2Cl_2$ (5 ml) and placed in an ice bath. Subsequently N-methylmorpholine (0.31 ml, 2.9 mmol) was added and the suspension became a solution. Then 2-chloro-4,6-dimethoxytriazine (0,48 g, 2.9 mmol) was added, and a white precipitate was formed immediately after this addition. The reaction mixture was stirred for 2h at 0°C, and then another two equivalents of N-methylmorpholine (0.31ml, 2.9 mmol) were added followed by aniline (0.28 ml, 2.9 mmol). Stirring was continued for 1h at 0°C, and the reaction mixture was then stirred overnight at room temperature. $CH_2Cl_2$ (50 ml) was added and the solution was washed with, respectively, 1M HCl (2 x 20ml), brine (1x 20 ml), saturated aqueous bicarbonate solution (1 x 20ml) and $H_2O$ (1 x 20ml). The organic phase was dried ($Na_2SO_4$) and after evaporation of the solvent gave a solid product. After purification, refluxing in $CH_2Cl_2$/ diethylether (excess), filtration (G4-glassfilter) and drying under vacuum at 50°C, a white solid was obtained (0.27g, 37%).

**1,2-Bis(2'-methyl-5'-{[((R)-1-phenylethyl) amino]carbonyl}thien-3'-yl)cyclopentene (D)**

This compound was prepared as described above for **C**, starting from **diacid B** (0.5 g, 1.44 mmol) and (R)-phenylethylamine (0.37 ml, 2.9 mmol). After purification by ($CH_2Cl_2$/MeOH = 60:1), stirring in MeOH/ diethyl ether (excess) and filtration (G4-glass filter), a white solid was obtained (0.28g, 35%). molecular formula $C_{33}H_{34}N_2O_2S_2$.

**1,2-Bis(2'-methyl-5'-{[((R)-1-cyclohexylethyl) amino]carbonyl}thien-3'-yl)cyclopentene (E)**

This compound was prepared as described above for **C**, starting from **diacid B** (1.34 g, 3.85 mmol) and (R)-cyclohexylamine (1.1 ml, 7.7 mmol). After purification by stirring in $CH_2Cl_2$/MeOH (60/1), filtration (G4-glass filter) and drying under vacuum at 50°C, a white solid was obtained (0.59g, 44%) molecular formula $C_{33}H_{46}N_2O_2S_2$.

**1,2-Bis(5'-boronyl-2'-methylthien-3'-yl)cyclopentene (F)**

Compound Z (1.0g, 3.04 mmol) was dissolved in anhydrous THF (12 ml) under a nitrogen atmosphere, and n-BuLi (5.0ml of 1.6M solution in hexane, 8 mmol) was added at once using a syringe. This solution was stirred for 30 min at r.t., and B(*n*-OBu)$_3$ (2.25 ml, 8.3 mmol) was added at once. The resulting solution was stirred for 1 h at room temperature and was used directly in the Suzuki cross coupling-reaction.

**1,2-Bis(5'-[methyl 5-(2-thienyl)-acetic acid]-2'-methylthien-3'-yl)cyclopentene (G)**

Methyl 2-(5-bromo-2-thienyl)acetic acid (2.94 g, 12.4 mmol) was dissolved in THF (75 ml) and $Pd(PPh_3)_4$ (0.42 g, 6 mol%) was added, this solution was stirred for 15 minutes at r.t.. Then aqueous $Na_2CO_3$ (36 ml, 2M) and 6 drops of ethylene glycol were added. This two-phase system was heated in an oilbath just below reflux (60°C) and the solution of **compound F** (6.07 mmol) in THF (20 ml) was added dropwise via a syringe in a short time-period. After that the mixture was refluxed for 2 hr. Subsequently cooled down and diethylether (100 ml) and $H_2O$ (50 ml) were added. The organic layer was washed with 1M HCl (2 x 50 ml), brine (2 x 50 ml) and dried ($Na_2SO_4$). After evaporation the compound was purified by column chromatography ($CH_2Cl_2$/ hexane = 4/1) on silica to yield an oil (2.13 g, 62%). ($C_{29}H_{28}O_4S_4$)

**1,2-Bis(5'-[5-(2-thienyl)-acetic acid]-2'-methylthien-3'-yl)cyclopentene (H)**

Compound G (0.81 g, 1.23 mmol) was dissolved in MeOH (20 ml) and THF (20 ml), then 4M NaOH (2.7 ml) was added. This mixture was stirred for 2 hr at r.t.. After evaporation of the solvent $H_2O$ (25 ml) was added and the mixture was acidified in an ice-bath with 2M HCl. A precipitate was formed immediately, which was filtrated over a glasfilter. After removal of the residual water by forming an azeotrope with toluene, an off-white solid was obtained (0.28 g, 76%). ($C_{27}H_{24}O_4S_4$)

**1,2-Bis(5'-{N-dodecyl-N'-[4-(2-thienyl)methyl]urea}-2'-methylthien-3'-yl)cyclopentene (I)**

Dry $CH_2Cl_2$ (20 ml) and $Et_3N$ (0.11 ml, 0.76 mmol) were added under a nitrogen atmosphere to **Compound H** (0.204 g, 0.38 mmol). When a solution was obtained diphenylphosphorylazide (0.16 ml, 0.76 mmol) was added in one time. This mixture was stirred for 2 h at room temperature and subsequently the temperature was raised to 50°C to stir for another 2 h. Then dodecylamine (0.17 ml, 0.76 mmol) was added and the mixture was refluxed for 2 hr and finally stirred at r.t. for 16 hr. The mixture was diluted with diethylether (80 ml) and washed with saturated bicarbonate solution (2 x 25 ml), $H_2O$ (2 x 25 ml), 1M HCl (1 x 25 ml), brine (2 x 25 ml) and dried ($Na_2SO_4$). After evaporation of the solvent in vacuo, the obtained solid This solid was submitted to column chromatography ($CHCl_3$/ MeOH = 20/1) on silica was refluxed in $CHCl_3$ /diethylether (excess) and filtrated (G4) to yield a purple solid (36 mg, 10 %). ($C_{51}H_{76}N_4O_2S_4$).

**1,2-Bis[5'-(4''-bromophenyl)-2'-methylthien-3'-yl] cyclopentene (J)**

1,4-dibromobenzene (3.4 g, 14.4 mmol)was dissolved in THF (12 ml) and after addition of $Pd(PPh_3)_4$ (0.4 g, 0.3 mmol), the solution was stirred for 15 min at r.t.. Then aqueous $Na_2CO_3$ (17 ml, 2M) and 6 drops of ethylene glycol were added, and the resulting two-phase system was heated in an oil bath till reflux (60°C). The solution of **compound F** was added dropwise by a syringe in a few minutes. After addition was complete, the reaction mixture was refluxed for 2 h, and then allowed to cool to r.t.. Diethyl ether (50 ml) and $H_2O$ (50 ml) were added, and the organic layer was collected and dried ($Na_2SO_4$). After evaporation of the solvent the product was purified by column chromatography ($SiO_2$, hexane) to gave a yellowish solid (1.30 g, 76%). ($C_{27}H_{22}Br_2S_2$):

**A gelation experiment**

In a typical gelation experiment, a carefully weighed amount of the dithienylcyclopentene derivative under investigation and 1 ml of the solvent are placed in a test tube, which is sealed and then heated until the compound is dissolved. The solution is allowed to cool to room temperature. Gelation was considered to have occurred when a homogeneous solid substance was obtained, which exhibited no gravitational flow. Gelation did occur in the following non-limiting cases of solutions of Compound C in 1-phenyloctane, toluene, n-butylether, benzene, tetralin; Compound D in cyclohexane, 1-phenyloctane, toluene, n-butyl ether, benzene, tetralin; Compound E in hexadecane, cyclohexane, 1-phenyloctane, toluene, n-butylether, benzene, tetralin, 1,4-dioxane, and n-butyl acetate. The gelation process could be followed by UV-vis and CD spectroscopy as depicted in Figure 23. At low concentrations (0.35 mM) compound D is dissolved in toluene and this solution did not show a CD effect. Increasing the concentration to 1.8 mM causes gelation of the solvent, which is accompanied by a change of the UV-visible spectrum, together with the appearance of a strong CD signal at 323 nm (Figure 22 A and B). Similar changes in the UV-vis and CD spectrum are observed upon gelation of the 1.8 mM solution of D in toluene by cooling to a temperature below the sol-gel phase transition (from here on referred to as gel(o). From a plot of the increase of the CD signal versus the temperature the sol-gel phase transition temperature of gel(o) was determined to be 29°C for a 1.8 mM solution of D in toluene.

**Photoswitching of a gel of compound D**

In a typical photoswitching experiment a solution (1.8 mM) of Compound D in toluene was prepared by cooling from 70°C to the desired temperature (typically between 30°C and 60°C, i.e. above the gel-sol phase transition temperature of the open form of D) in a cuvette of 1 mm path length. This solution was then irradiated at $\lambda_1$ = 313 nm to convert the ring-open form of Compound D to the ring-closed form. By using a 150 W Xe lamp and a 313 nm bandpass filter, the photostationary state (PSS) was reached typically within 10 minutes, which consist of 60mol% of the closed form and 40mol% of the open form. During this irradiation process the solution of D was transformed into a deeply purple colored gel (from here referred to as gel(c,I), which could be monitored by UV-vis and CD spectroscopy (Figure 23 D and D). From a plot of the increase of the CD signal versus the temperature the sol-gel phase transition temperature of gel(c,I) was determined to be 62°C, showing that the thermal stability has increased by more than 30°C compared to that of gel(o). It should be noted that dissolving gel(c,I) by heating and subsequent cooling to again a temperature below the gel-sol phase transition, resulted in a CD spectrum different to that of gel(c,I), indicating that gels of the closed form of D can adopt an alternate structure, which is from hereon referred to as gel(c,II) (see Figure 23D).

Gels of the closed form of D can also be dissolved by converting the closed form to the open form by irradiation with light of wavelength □□.
Irradiation of gel(c,I) or gel(c,II) with □□ > 450 nm at while keeping the temperature between 30°C and 60°C causes dissolution of the gel to give a solution of the open form D, of which the UV-vis and CD spectra are identical to that of the starting solution. This process of gelation-dissolution by alternating irradiations with UV (313 nm) and Vis (> 450

nm) light can be repeated many times, showing that the photo-induced gelation process is fully reversible.
In another experiment a solution (1.8 mM) of Compound D in toluene was prepared by cooling from 70°C to a temperature below the gel-sol phase transition temperature of the open form of (typically between 20°C and 29°C) in a cuvette of 1 mm path length. This solution was then irradiated at $\lambda_1$ = 313 nm to convert the ring-open form of Compound D to the ring-closed form, whereas solutions which have been kept in the dark stay colorless and did not form a gel within 1 hour. This clearly shows that gelation of a solution of D can be accelerated by converting the open to the closed form by irradiation with light of wavelength $\lambda_1$.

Example 13

Some examples of suitable amphiphiles and a method for preparation thereof

**Preparation of 1-(9'-cis-octadecenyl)-4-(10"-cis-nonadecenyl) pyridinium Chloride**

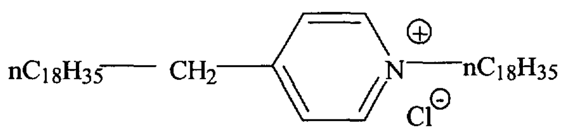

*4-(10'-cis)-nonadecenyl pyridine*

A solution of 5.05 g (49.9 mmol) diisopropylamine in 300 ml of dry THF was cooled to 0° C under a nitrogen atmosphere. Then, 31.3 ml of a n-butyl lithium/hexane solution (1.6 M in hexane's, 50.0 mmol) was added dropwise and the resulting reaction mixture was stirred for an additional 30 min at 0° C. Subsequently, 4.50 g of 4-picoline (48.5 mmol) was added dropwise at -50° C under careful temperature control. After completion, the orange-yellow solution was stirred for another 30 min at -50° C. Hereafter, 18.33 g of 1-Iodo-(9-cis)-octadecene (85% cis, 48.5 mmol) in 25 ml of dry THF was added dropwise at -50° C under careful temperature control. The bright red solution was stirred for an additional 60 min at -50° C and at RT overnight. The reaction was quenched by adding 300 ml of a saturated $NH_4Cl$ solution. The organic layer was separated and the waterlayer extracted with three portions of ether (50 ml). The combined organic fractions were washed twice with $H_2O$ (100 ml) and brine (200 ml), dried over $Na_2SO_4$, filtered and concentrated in vacuo to yield a viscous brown oil. The product was purified over a column of 300 g neutral $Al_2O_3$ (act. III, freshly prepared) using n-hexane/diethyl ether as gradient eluent mixture (100:0 > 80:20) yielding 4-(10'-cis)-nonadecenyl pyridine as a colourless viscous oil. Yield after purification 12.20 g, 36.9 mmol, 76%. The cis:trans ratio at the double bonds was unchanged 85:15 based upon $^1H$ NMR integration of the protons at δ 5.25 and 5.33 ppm, which are broad singlets when selectively homo nuclear decoupled at ~ δ 1.90 ppm.
$^1H$ NMR ($CDCl_3$): δ 0.81 (t, $^3J$= 6.95 Hz, 3H), 1.15-1.35 (br, m, 26H), 1.52-1.58 (br, m, 2H), 1.82-1.98 (br, m, 4H), 2.52 (t, $^3J$= 7.69 Hz, 2H), 5.21-5.38 (m, 2H),
7.02 (d, $^3J_{AB}$= 5.49 Hz, 2H), 8.41 (d, $^3J_{AB}$= 5.49 Hz, 2H).
$^{13}C$ NMR ($CDCl_3$): δ 12.60, 21.17, 21.78, 24.09, 25.69, 27.68, 27.75, 27.81, 27.99, 28.25, 28.79, 30.39, 31.09, 66.44, 122.36, 123.09, 128.28, 128.41, 128.76, 148.08.
Elemental analysis for $C_{24}H_{41}N$ (343.60) calcd. C 83.90%, H 12.03%, N 4.08%; found C 83.87%, H 12.07%, N 4.04%. EI-MS calcd. for $C_{24}H_{41}N$ 343.3 found 343.3.

*1-(9'-cis-octadecenyl)-4-(10"-cis-nonadecenyl) pyridinium Iodide*

A pre-mixed (1 min ultrasonic bath) amount of 4-(10'-cis)-nonadecenyl pyridine (0.50 g, 1.51 mmol) and 1-Iodo-(9-cis)-octadecene (85% cis, 0.75 g, 1.98 mmol) was placed in a MicroWave oven and carefully deoxygenated. The mixture was reacted using Pulsed Intervals [10 min 600 Watt (90%) followed by 10 min of 300 Watt (50%), sequence repeated three times], resulting in a deep red-brown very viscous oil. Based upon $^1H$ NMR, conversion had evolved to approximately 90%. The material was purified over a column of 50 g neutral $Al_2O_3$ (act. III, freshly prepared) using n-hexane as eluent to remove impurities and (excess) starting materials. The material was brought on the column dissolved in a minimum amount of $CHCl_3$. After changing the solvent to n-hexane/methanol (98:2), 1-(9-cis-octadecenyl)-4-(10'-cis-nonadecenyl) pyridinium iodide was obtained as a colourless viscous oil that colours quickly. The material solidifies completely if stored at temperatures below 4° C. Yield 0.83 g, 1.17 mmol, 78%. The cis:trans ratio at the double bonds was unchanged 85:15 based upon $^1$H NMR integration of the protons at δ 5.32 and 5.40 ppm, which are broad singlets when selectively homo nuclear decoupled at ~ δ 2.00 ppm.
$^1$H NMR ($CDCl_3$): δ 0.85 (t, $^3J$= 6.8 Hz, 6H), 1.20-1.45 (br, m, 46H), 1.50-1.80 (br, m, 4H), 1.90-2.10 (br, m, 8H), 2.87 (t, $^3J$= 8.12 Hz, 2H), 4.87 (t, $^3J$= 7.31 Hz, 2H), 5.28-5.45 (m, 4H), 7.83 (d, $^3J_{AB}$= 6.59 Hz, 2H), 9.23 (d, $^3J_{AB}$= 6.59 Hz, 2H).
$^{13}$C NMR ($CDCl_3$): δ 12.60, 21.25, 24.50, 25.68, 27.52, 27.73, 27.76-27.78 (several), 27.87, 28.09, 28,18, 28.23, 30.28, 30.37, 31.08, 34.47, 59.86, 126.40, 128.12, 128.23, 128.44, 128.50, 142.62, 161.81.
Elemental analysis for $C_{42}H_{76}NI$ (721.98) calcd. C 69.87%, H 10.61%, N 1.94%, I 17.58%; found C 69.74%, H 10.53%, N 1.98%, I 17.55%. EI-MS calcd. for $C_{42}H_{76}NI$ 721.5, found 721.3.

*1-(9'-cis-octadecenyl)-4-(10''-cis-nonadecenyl) pyridinium Chloride*

0.50 g (0.70 mmol) of 1-(9'-cis-octadecenyl)-4-(10''-cis-nonadecenyl) pyridinium iodide (purified) was dissolved in 5 ml of methanol and this solution was eluted over a DEAE Sephadex A-25 column (50 g, Chloride form) yielding 1-(9'-cis-octadecenyl)-4-(10''-cis-nonadecenyl) pyridinium chloride (0.40 g, 0.66 mmol, 94%) as a colourless viscous oil. The material solidifies completely if stored at temperatures below 4° C. The cis:trans ratio at the double bonds was unchanged 85:15 based upon $^1$H NMR integration of the protons at δ 5.30 and 5.38 ppm, which are broad singlets when selectively homo-nuclear decoupled at ~ δ 2.00 ppm.
$^1$H NMR ($CDCl_3$): δ 0.85 (t, $^3J$= 6.8 Hz, 6H), 1.20-1.45 (br, m, 46H), 1.50-1.80 (br, m, 4H), 1.90-2.10 (br, m, 8H), 2.87 (t, $^3J$= 8.1 Hz, 2H), 4.98 (t, $^3J$= 7.3Hz, 2H), 5.32-5.54 (m, 4H), 7.94 (d, $^3J_{AB}$= 6.22 Hz, 2H), 9.43 (d, $^3J_{AB}$= 6.22 Hz, 2H). $^{13}$C NMR ($CDCl_3$): δ 12.60, 21.25, 24.50, 25.68, 27.52, 27.73, 27.76-27.78 (several), 27.87, 28.09, 28,18, 28.23, 30.28, 30.37, 31.08, 34.47, 59.96, 126.40, 128.12, 128.23, 128.44, 128.50, 142.65, 161.92.
Elemental analysis for $C_{42}H_{76}NCl$ (630.53) calcd. C 80.01%, H 12.15%, N 2.22%, Cl 5.62%; found C 79.85%, H 12.08%, N 2.27%, Cl 5.58%. EI-MS calcd. for $C_{42}H_{76}NCl$ 629.6, found 629.6.

**1-(6''-Phenylhexyl)-4-(10'-cis-nonadecenyl) pyridinium Bromide**

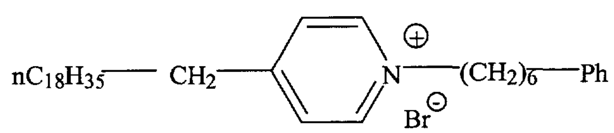

*1-(6''-Phenylhexyl)-4-(10'-cis-nonadecenyl)pyridinium Bromide*

A pre-mixed (1 min ultrasonic bath) amount of 0.50 g (1.46 mmol) 4-(10'-cis)-nonadecenyl pyridine and 1.67 g 1-bromo-6-phenylhexane (7.30 mmol) was placed in a MicroWave oven and carefully deoxygenated. The mixture was reacted using Pulsed Intervals [10 min 600 Watt (90%) followed by 10 min of 300 Watt (50%), sequence repeated three times], resulting in a deep brown material. The crude product mixture was purified by column chromatography (act. III, freshly prepared) using diethyl ether as eluent to remove impurities and (excess) starting materials. The material was brought on the column dissolved in a minimum amount of $CHCl_3$. After changing the solvent to diethyl ether/methanol (98:2) the product was obtained as a slightly coloured viscous oil. Yield 0.73 g, 1.26 mmol, 86%.
$^1$H NMR ($CD_3OD$): δ 0.81 (t, $^3J$= 6.02 Hz, 3H), 1.13-1.40 (br, m, 30H), 1.56 (t, $^3J$= 8.02 Hz, 2H), 1.64 (t, 3J= 7.04 Hz, 2H), 1.84-2.04 (br, m, 4H), 2.52 (t, $^3J$= 8.02 Hz, 2H), 2.86 (t, $^3J$= 7.94 Hz, 2H), 4.46 (t, $^3J$= 6.68 Hz, 2H), 5.22-5.36 (m, 2H), 7.01-7.18 (m, 5H), 7.86 (d, $^3J_{AB}$= 7.02 Hz, 2H), 8.73 (d, $^3J_{AB}$= 7.02 Hz).

**1-Methyl-4-(-carbo-(9'-cis-nonadecenyloxy)) pyridinium Chloride**

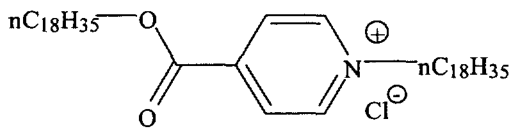

*4-carbo-(9'-cis-nonadecenyloxy)pyridine*

A solution of 2.0 g isonicotinoylchloride hydrochloride (11.24 mmol) and triethylamine (2.53 g, 25 mmol) in 50 ml of $CH_2Cl_2$ was cooled to 0° C. 1-hydroxy-(9-cis)-octadecene (3.01 g, 11.24 mmol, 85% cis) dissolved in 25 ml of $CH_2Cl_2$ was added dropwise and the reaction was allowed to react for 60 min. Subsequently, the reaction mixture was taken to reflux overnight. The mixture was cooled to 0° C and filtered, the filtrate was concentrated at reduced pressure yielding a yellow viscous oil. The crude product mixture was purified by column chromatography (act. III, freshly prepared, 75 g) using hexane as eluent to remove impurities and (excess) starting materials. The material was brought on the column dissolved in a minimum amount of $CHCl_3$. Changing the solvent to hexane:$CHCl_3$ (gradient, 100:0 to 80:20 and a few drops of triethylamine) afforded the product as a colourless oil that partly solidifies upon standing. Yield 2.09 g, 5.12 mmol, 45%. The cis:trans ratio at the double bonds was unchanged 85:15 based upon $^1$H NMR integration of the protons at δ 5.27 and 5.33 ppm, which are broad singlets when selectively homo nuclear decoupled at ~ δ 1.95 ppm.
$^1$H NMR ($CDCl_3$): δ 0.80 (t, $^3$J= 5.86 Hz, 3H), 1.15-1.42 (br, m, 22H), 1.71 (m, 2H), 1.87-2.01 (br, m, 4H), 4.28 (t, 3J= 6.65 Hz, 2H), 5.22-5.38 (br, m, 2H), 7.77 (d, $^3J_{AB}$= 5.86 Hz, 2H), 8.79 (d, $^3J_{AB}$= 5.86 Hz, 2H).
$^{13}$C NMR ($CDCl_3$): δ 13.98, 22.56, 25.84, 27.04, 27.09, 28.49, 29.10, 29.19, 29.27, 29.40, 29.58, 29.63, 31.78, 32.49, 65.82, 122.70, 129.60, 129.89, 137.52, 150.44, 165.02.

*1-(9'-cis-nonadecenyl)-4-(-carbo-(9"-cis-nonadecenyloxy)) pyridinium Iodide*

0.40 g 4-carbo-(9'-cis-nonadecenyloxy) pyridine (0.97 mmol) and 0.57 g 1-Iodo-(9-cis)-octadecene (85% cis, 1.50 mmol) were placed in a MicroWave oven and carefully deoxygenated. The mixture was reacted under continuous progress control (TLC) using Pulsed Intervals [10 min 600 Watt (50%) followed by 10 min of 300 Watt (15%)] until the conversion had evolved to approx. 85%. This process takes about 80 minutes, resulting in a reddish very viscous material. The crude product mixture was purified by column chromatography (act. III, freshly prepared, 25 g) using hexane as eluent to remove impurities and (excess) starting materials. The material was brought on the column dissolved in a minimum amount of $CHCl_3$. Changing the solvent to hexane:$CHCl_3$ (gradient, 100:0 to 80:20) and eventually to hexane:$CHCl_3$:methanol (75:20:5) afforded the product as a colourless oil, that partly solidifies upon standing. Yield 0.54 g, 0.69 mmol, 71%.The material was used immediately for ion exchange due to the relative low stability as Iodide salt. The cis:trans ratio at the double bonds was unchanged 85:15 based upon $^1$H NMR integration of the protons at δ 5.25 and 5.31 ppm, which are broad singlets when selectively homo nuclear decoupled at ~ δ 1.90 ppm.
$^1$H NMR ($CDCl_3$): δ 0.82 (dt, $^3$J= 6.96 Hz, 6H), 1.20-1.45 (br, m, 22H), 1.48-1.54 (br, m, 20H), 1.72-1.83 (m, 2H), 1.90-2.08 (br, m, 12 H), 4.40 (t, $^3$J= 6.95 Hz, 2H), 4.98 (br, t, $^3$J= 7.32 Hz, 2H), 5.20-5.35 (m, 4H), 8.48 (d, $^3J_{AB}$= 5.86 Hz, 2H), 9.41 (d, $^3J_{AB}$= 5.86 Hz, 2H).

*1-(9'-cis-nonadecenyl)-4-(-carbo-(9"-cis-nonadecenyloxy))pyridinium Chloride*

A solution of 0.25 g 1-(9'-cis-nonadecenyl)-4-(-carbo-(9"-cis-nonadecenyloxy)) pyridinium Iodide (0.32 mmol) and 0.46 g AgCl (3.2 mmol) in 25 ml of dry acetone was placed under a nitrogen atmosphere and taken to reflux. During reflux the solution de-coloured from yellow to white. The reaction mixture was cooled to approx. 0° C and filtered. The residue was washed with small amounts of dry acetone. The combined acetone layers were combined and concentrated in vacuo. The crude product mixture was purified by column chromatography (act. III, freshly prepared, 25 g) using hexane:$CHCl_3$ (85:15) to remove impurities. Changing the solvent to hexane:$CHCl_3$:methanol (75:20:5) afforded the product as a colourless oil/gel, that partly solidifies upon standing. Yield 0.16 g, 0.25 mmol, 78%. The cis: trans ratio at the double bonds was unchanged 85:15 based upon $^1$H NMR integration of the protons at δ 5.25 and 5.30 ppm, which are broad singlets when selectively homo nuclear decoupled at ~ δ 1.90 ppm.

$^1H$ NMR ($CDCl_3$): δ 0.82 (dt, $^3J$= 6.96 Hz, 6H), 1.20-1.45 (br, m, 22H), 1.48-1.54 (br, m, 20H), 1.72-1.83 (m, 2H), 1.90-2.08 (br, m, 12 H), 4.67 (t, $^3J$= 6.87 Hz, 2H), 5.09 (br, t, $^3J$= 7.24 Hz, 2H), 5.20-5.45 (m, 4H), 8.58 (d, $^3J_{AB}$= 5.81 Hz, 2H), 9.61 ((d, $^3J_{AB}$= 5.81 Hz, 2H).

According to the experimental procedures given above the following compounds have been synthesized:

*1-Benzyl-4-nonadecyl pyridinium Chloride*

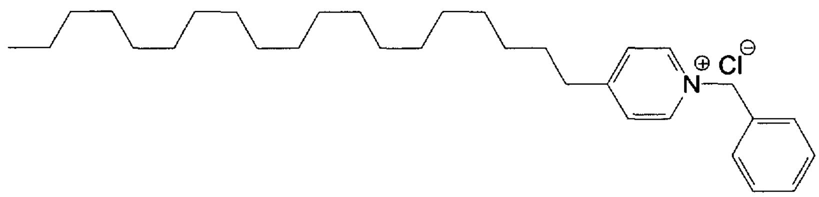

$^1H$ NMR ($CD_3OD$): δ 0.81 (t, $^3J$= 7.00 Hz, 3H), 1.13-1.35 (m, 32 H), 1.58-1.71 (m, 2H), 2.86 (t, $^3J$= 7.00 Hz, 2H), 5.68 (br, m, 2H), 7.34 (br, m, 5H), 7.87 (d, $^3J_{AB}$= 6.03 Hz, 2H), 8.79 (d, $^3J_{AB}$= 6.03 Hz, 2H).

*1-Benzyl-4-(cis-10')-nonadecenyl pyridinium Chloride*

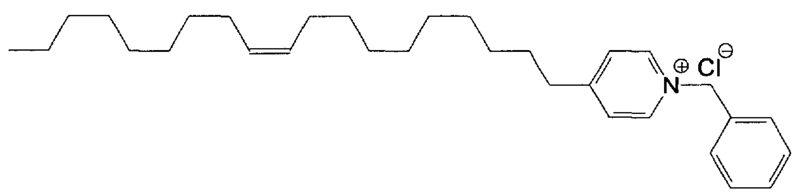

$^1H$ NMR ($CDCl_3$): δ 0.79 (t, $^3J$= 7.01 Hz, 3H), 1.09-1.36 (m, 24H), 1.55 (q, $^3J$= 7.01 Hz, 2H), 1.84-2.05 (m, 4H), 2.73 (t, $^3J$= 6.92 Hz, 2H), 5.23-5.36 (br, m, 2H), 6.08 (br, s, 2H), 7.28 (dd, $^3J_{AB}$= 7.04 Hz, $^4J$= 3.05 Hz, 3H), 7.56 (dd, 3JAB= 7.04 Hz, 4J= 3.05 Hz), 7.65 (d, $^3J_{AB}$= 7.09 Hz, 2H), 9.33 ($^3J_{AB}$= 7.09 Hz, 2H).

*1-(4'-Phenylbutyl)-4-nonadecyl pyridinium Chloride*

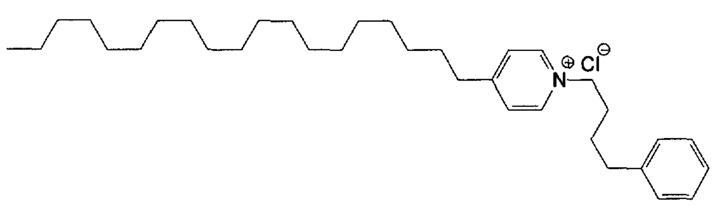

$^1H$ NMR (DMSO-$d_6$): δ 0.88 (t, $^3J$= 5.67 Hz, 3H), 1.09-1.40 (m, 32H), 1.63 (m, 4H), 1.97 (m, 2H), 2.61 (t, $^3J$= 7.02 Hz, 2H), 2.77 (t, $^3J$= 8.06 Hz, 2H), 4.91 (br, t, J=6.97 Hz, 2H), 7.04-7.27 (m, 5H), 7.69 (d, $^3J_{AB}$= 7.04 Hz, 2H), 9.27 (d, $^3J_{AB}$= 7.04 Hz, 2H).

*1-(4"-Phenylbutyl)-4-(cis-10)-nonadecenyl pyridinium Chloride*

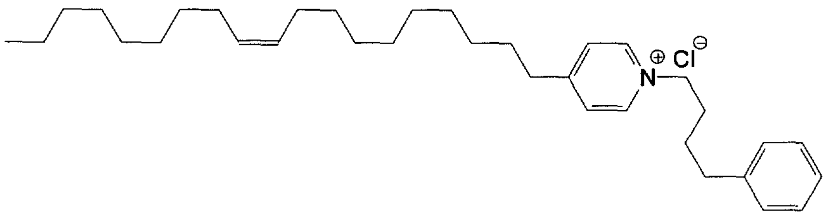

$^1$H NMR ($CD_3OD$): δ 0.89 (t, $^3J$= 7.01 Hz, 3H), 1.09-1.40 (m, 26H), 1.58-1.84 (m, 4H), 1.89-2.13 (m, 4H), 2.68 (t, 3J= 6.91 Hz, 2H), 2.97 (t, 3J= 7.99 Hz, 2H0, 4.56 (br, m, 2H), 5.23-5.36 (m, 2H), 7.09-7.36 (m, 5H), 7.92 (d, $^3J_{AB}$= 7.07 Hz, 2H), 8.79 (d, $^3J_{AB}$= 7.07 Hz, 2H).

*1-(6'-Phenylhexyl)-4-nonadecyl pyridinium Chloride*

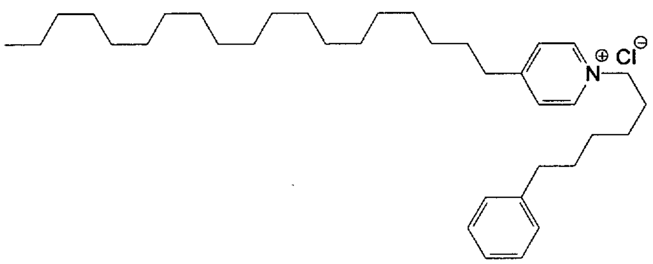

$^1$H NMR ($CD_3OD$): δ 0.87 (t, $^3J$= 6.03 Hz, 3H), 1.3-1.44 (m, 34H), 1.49-1.76 (m, 4H), 1.89-2.09 (m, 4H), 2.56 (t, $^3J$= 7.03 Hz, 2H), 2.84 (t, $^3J$= 7.01 Hz, 2H), 4.93 (br, m, 2H), 7.09-7.31 (m, 5H), 7.80 (d, $^3J_{AB}$= 7.05 Hz, 2H), 9.34 (d, $^3J_{AB}$= 7.05 Hz, 2H).

*1-(6"-Phenylhexyl)-4-(cis-10)-nonadecenyl pyridinium Bromide*

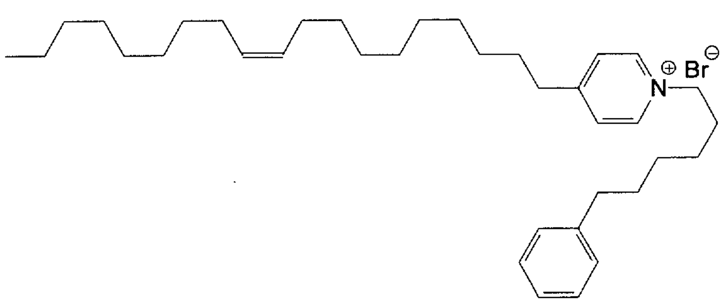

$^1$H NMR ($CD_3OD$): δ 0.81 (t, $^3J$= 6.02 Hz, 3H), 1.13-1.40 (br, m, 30H), 1.56 (t, $^3J$= 8.02 Hz, 2H), 1.64 (t, 3J= 7.04 Hz, 2H), 1.84-2.04 (br, m, 4H), 2.52 (t, $^3J$= 8.02 Hz, 2H), 2.86 (t, $^3J$= 7.94 Hz, 2H), 4.46 (t, $^3J$= 6.68 Hz, 2H), 5.22-5.36 (m, 2H), 7.01-7.18 (m, 5H), 7.86 (d, $^3J_{AB}$= 7.02 Hz, 2H), 8.73 (d, $^3J_{AB}$= 7.02 Hz).

*1,1'-bis-(1''',4''''-butyl)-4-(cis-10'')-nonadecenyl pyridinium Chloride*

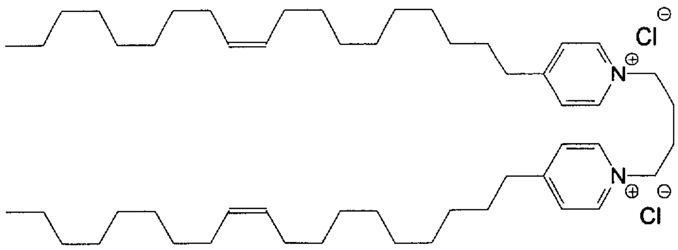

$^1$H NMR ($CDCl_3$): δ 0.83 (t, $^3J$= 7.00 Hz, 6H), 1.08-1.38 (m, 44H), 1.50-1.79 (m, 8H), 1.96-2.05 (m, 8H), 2.30-2.48 (m, 4H), 2.90 (m, 8H), 5.02 (br, m, 4H), 5.25-5.41 (m, 4H), 7.76 (d, $^3J_{AB}$= 8.67 Hz, 4H), 9.80 (d, $^3J_{AB}$= 8.67 Hz, 4H).

*1,1'-bis-(1''',6''''-hexyl)-4-(cis-10'')-nonadecenyl pyridinium Chloride*

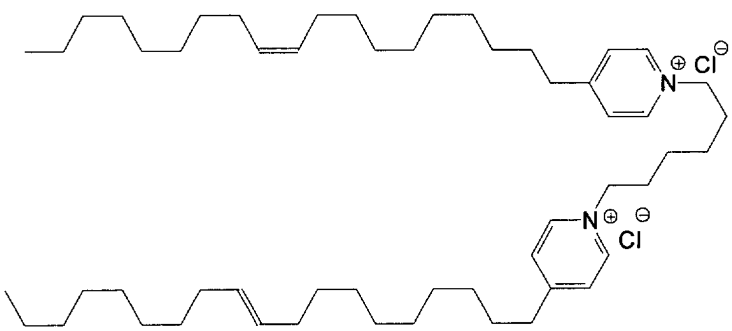

$^1$H NMR ($CDCl_3$): δ 0.83 (t, $^3J$= 7.01 Hz, 6H), 1.08-1.40 (m, 46H), 1.42-1.79 (m, 8H), 1.96-2.09 (m, 8H), 2.11-2.24 (m, 4H), 2.93 (m, 8H), 4.97 (br, m, 4H), 5.21-5.39 (m, 4H), 7.79 (d, $^3J_{AB}$= 6.35 Hz, 4H), 9.81 (d, $^3J_{AB}$= 6.35 Hz, 4H).

*1-Methyl-4-(10'-cis-nonadecenyl) pyridinium Chloride*

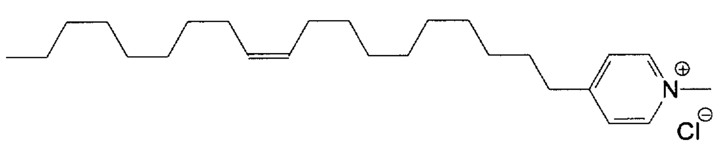

$^1$H NMR ($CDCl_3$): δ 0.82 (t, $^3J$= 7.81 Hz, 3H), 1.06-1.52 (m, 26H), 1.60-1.92 (m's, 4H), 4.89 (t, $^3J$= 6.62 Hz, 2H), 4.92 (br, m, 3H), 5.30-5.56 (m, 4H), 7.98 (d, $^3J_{AB}$= 6.87 Hz, 2H), 9.45 (d, $^3J_{AB}$= 6.87 Hz, 2H).

*1-(9'-cis-octadecenyl)-4-(10"-cis-nonadecenyl)pyridinium Chloride*

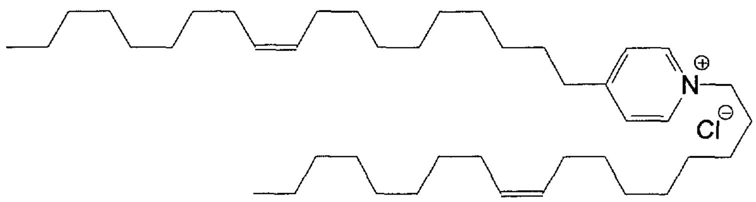

$^1$H NMR ($CDCl_3$): δ 0.85 (t, $^3$J= 6.80 Hz, 6H), 1.20-1.45 (br, m, 46H), 1.50-1.80 (br, m, 4H), 1.90-2.10 (br, m, 8H), 2.87 (t, $^3$J= 8.11 Hz, 2H), 4.98 (t, $^3$J= 7.31 Hz, 2H), 5.32-5.54 (m, 4H), 7.94 (d, $^3J_{AB}$= 6.22 Hz, 2H), 9.43 (d, $^3J_{AB}$= 6.22 Hz, 2H). *1-(hexadecyl)-4-(10"-cis-nonadecenyl)pyridinium Chloride*

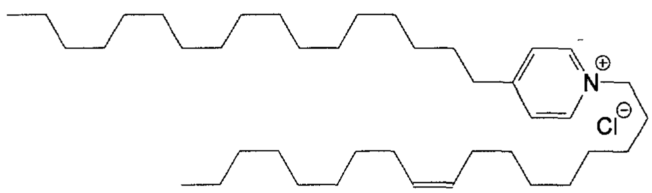

$^1$H NMR ($CDCl_3$): δ 0.82-0.94 (t's, 6H), 1.20-1.45 (br, m, 46H), 1.50-1.85 (br, m, 4H), 1.91-2.14 (br, m, 8H), 2.84 (t, 2H), 4.88 (t, $^3$J= 7.30 Hz, 2H), 5.30-5.45 (m, 2H), 7.87 (d, $^3J_{AB}$= 6.34 Hz, 2H), 9.56 (d, $^3J_{AB}$= 6.34 Hz, 2H).

**1-(9'-cis-nonadecenyl)-4-(-carbo-(9"-cis-nonadecenyloxy))pyridinium Chloride**

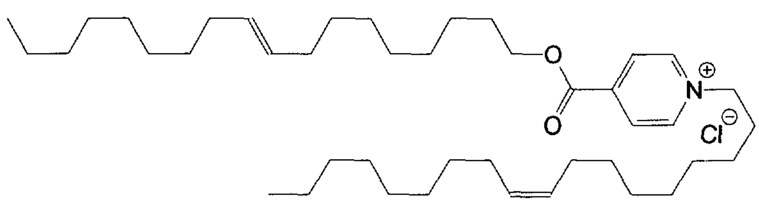

$^1$H NMR ($CDCl_3$): δ 0.82 (dt, $^3$J= 6.96 Hz, 6H), 1.20-1.45 (br, m, 22H), 1.48-1.54 (br, m, 20H), 1.72-1.83 (m, 2H), 1.90-2.08 (br, m, 12 H), 4.67 (t, $^3$J= 6.87 Hz, 2H), 5.09 (br, t, $^3$J= 7.24 Hz, 2H), 5.20-5.45 (m, 4H), 8.58 (d, $^3J_{AB}$= 5.81 Hz, 2H), 9.61 ((d, $^3J_{AB}$= 5.81 Hz, 2H).

**1-(9'-cis-nonadecenyl)-4-(-carbo-(hexadecyloxy)) pyridinium Chloride**

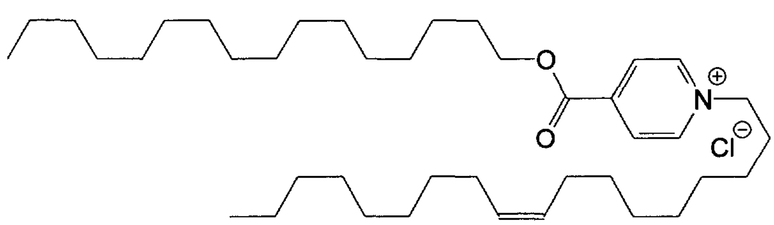

$^1H$ NMR ($CDCl_3$): δ 0.88-0.94 (t's, 6H), 1.20-1.45 (br, m, 26H), 1.48-1.54 (br, m, 16H), 1.70-1.90 (m, 4H), 1.92-2.10 (br, m, 12H), 4.64 (t, $^3J$= 6.81 Hz, 2H), 5.05 (br, t, $^3J$= 7.20 Hz, 2H), 8.52 (d, $^3J_{AB}$= 6.21 Hz, 2H), 9.56 (d, $^3J_{AB}$= 6.21 Hz, 2H).

*1-Methyl-4-(-carbo-(9'-cis-nonadecenyloxy)) pyridinium Chloride*

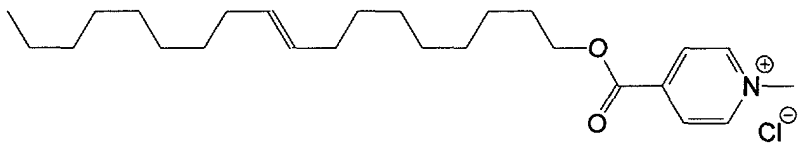

$^1H$ NMR ($CDCl_3$): δ 0.86 (t, $^3J$= 6.05 Hz, 3H), 1.08-1.37 (m, 22H), 1.52-1.76 (m, 2H), 1.82-2.04 (m, 4H), 4.45 (t, $^3J$= 6.89 Hz, 2H), 4.79 (br, m, 3H), 5.21-5.40 (m, 2H), 8.46 (d, $^3J_{AB}$= 6.68 Hz, 2H), 9.45 (d, $^3J_{AB}$= 6.68 Hz, 2H).

*1-Methyl-4-(-carbo-(hexadecyloxy))pyridinium Chloride*

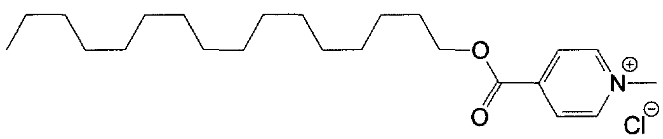

$^1H$ NMR ($CDCl_3$): δ 0.90 (t, $^3J$= 6.51 Hz, 3H), 1.18-1.51 (m, 22H), 1.60-1.82 (m, 6H), 4.67 (t, $^3J$= 6.48 Hz, 2H), 4.81 (br, m, 3H), 8.46 (d, $^3J_{AB}$= 5.61 Hz, 2H), 9.49 (d, $^3J_{AB}$= 5.61 Hz, 2H).

**Transfection protocol**

Transfections are carried out following standard protocols as is described in EP 0755924. In numerous publications variations of transfection protocols are given. A person acquainted with the field of non-viral transfections, in particular transfections using cationic amphiphiles will be able to use the cationic amphiphiles of the invention in transfection methods known per se. In standard experiments COS-7 cells are transfected with plasmid DNA containing the β-galactosidase reporter gene. Upon successful transfection the cell produces the enzyme β-galactosidase whose activity can be easily measured. Other plasmids comprising alternative reporter genes such as the gene encoding Green Fluoerscent Protein or Chloroamphenicol Acetyl Transferase are suited as well. For analyzing stable transfection a plasmid containing the neomycin resistance gene can be used. Toxicity of the cationic amphiphiles towards the cells can be monitored qualitatively and quantitatively by visual inspection of the cells and by a protein determination.

**Transfection with 1-(9'-cis-octadecenyl)-4-(10"-cis-nonadecenyl) pyridinium Chloride (cationic amphiphile of the formula 1 in which R1 = R2 = n$C_{18}H_{35}$, A = $CH_2$ and A is attached in the para position, X = $Cl^-$)**

A solution of cationic amphiphile in a 50/50 molar ratio with DOPE in chloroform was dried under a stream of nitrogen. The residual solvent was removed under vacuum. The lipid film was hydrated in water at room temperature and sonicated to clarity in a bath sonicator immediately before use.
A 7.1 kb pair plasmid containing the *E.coli* β-galactosidase gene under control of the cytomegalovirus immediate early gene promoter/enhancer (pCMV β-gal Clontech, Palo Alto, CA, USA). DNA was isolated from *E.coli* using a Qiagen Plasmid Kit (QIAGEN Inc, USA). The plasmid concentration was determined by measuring the absoption at 260 nm using the relation 1.0 OD = 50 μg/ml. The $OD_{260}/OD_{280}$ value was 1.95.
COS-7 cells were cultured in Dulbecco's Modified Eagle Medium (DMEM Gibco The Netherlands) containing 7% of fetal calf serum, 2 mM L-Glutamine, 100 units/ml of penicilin and 100 mg/ml of streptomycin at 37 °C in $CO_2$/air (1:19). Cells (1x $10^5$ cells/well) were seeded in 12-well plates and were allowed to grow overnight.

The complex of cationic amphiphile/DOPE (1:1) with pCMV β-gal was prepared at the charge ratio 2.5:1 (15 nmol lipid and 1 μg DNA), in 100μl 10 mM HEPES (pH=7.4, 150 mM NaCl). After 10-15 min incubation at room temperature, the resulting complex was diluted in 1 ml medium (DMEM) and 0.5 ml of the mixture was added to the cells and incubated for 4 h at 37°C. The β-galactosidase assay was performed 48 later using CPRG-substrate.
Control wells underwent identical steps except that no cationic amphiphile was added.
β-galactosidase activity was observed whereas in control wells no activity was detected. The β-galactosidase activity obtained using the cationic amphiphile according to the invention was substantially higher than the activity obtained with Lipofectin™ (Felgner et al. Proc. Natl. Acad. Sci USA, 1987, 84, 7413-7417). The cationic amphiphile displayed no toxicity towards the cells as was analyzed in a protein determination and compared with the reference value of control wells.

Transfection protocol in the absence of helper lipid with complexation of cationic amphiphile and DNA in small volume of HBS followed by dilution in medium
In a comparative experiment the following cationic amphiphiles were used for transfection:

A: a compound having the formula of claim 1 in which R2-A is $(C_{16}H_{33})_2CH$ and A is attached in the para position, X is $Cl^-$ and R1 is $CH_3$
B: a compound having the formula of claim 1 in which R2-A is $(C_{16}H_{33})_2CH$ and A is attached in the para position, X is $Cl^-$ and R1 is $(CH_2)_4NH_3^+$.

Solutions of A, B and plasmid DNA in HBS (10 mM HEPES, 150 mM NaCl, pH 7.4) were prepared. The concentrations used were for A 7.5 and 15 nmol per 50 μl, for B 7.5 nmol per 50 μl and for DNA 1 μg per 50 μl.
For each concentration of amphiphile 50 μl was mixed with 50 μl of DNA solution. After a short incubation time of 15 minutes this was diluted with medium to a volume of 1 ml.
COS-7 cells (1x $10^5$ cells/well) were seeded in 12-well plates and were allowed to grow overnight. 0.5 ml of the mixture obtained above was added to one well.
The cells were incubated at 37°C for 4 hours followed by a β-galactosidase activity determination after 48 hours.
In a control well no β-galactosidase activity was observed.
For A, β-galactosidase activity was observed in approximately equal amounts for both concentrations.
For B a β-galactosidase activity was observed which was approximately 25 to 30 times higher than for A.

**Table A. AcmA cell wall binding domain homologs and their calculated pI values.**
**(the pI values are indicated directly behind the amino acid sequences)**

| | | | | | | | |
|---|---|---|---|---|---|---|---|
| ***Lactococcus*** *lactis* | * *acmA* | YTVKSGDTLWGISQRYGISVAQIQSAN NLKST IIYIGQKLVLT | 9.75 | 245-287(33) | 437 | U17696 | muramidase |
| | | VKVKSGDTLWALSVKYKTSIAQLKSWN HLSSD TIYIGQNLIVS | 9.64 | 321-363(31) | | | |
| | | HKVVKGDTLWGLSQKSGSPIASIKAWN HLSSD TILIGQYLRIK | 10.06 | 395-437 | | | |
| | * *acmD* | YKVQEGDSLSAIAAQYGTTVDALVSAN SLENANDIHVGEVLQVA | 4.15 | 194-237 | | QGCI25 | |
| | | YTVKSGDSLYSIAEQYGMTVSSLMSANGIYDVNSMLQVGQVLQVTV | 3.78 | 258-303 | | | |
| | | YTIQNGDSIYSIATANGMTADQLAALNGFGIND MIHPGQTIRI | 4.15 | 319-361 | | | |
| ØTuc2009 | * *lys* | YVVKQGDTLSGIASNWGTNWQELARQN SLSNPNMIYAGQVISFT | 6.31 | 332-375(10) | 428 | L31364 | glycosidase (muramidase) |
| | | YTVQSGDNLSSIAILLGTTVQSLVSMN GISNPNLIYAGQTLNY | 3.45 | 386-428 | | | |
| Ø-LC3 | * *lysB* | YIVKQGDTLSGIASNLGTNWQELARQN SLSNPNMIYSGQVISLT | 6.31 | 333-376(10) | 429 | U04309 | muramidase |
| | | YTVQSGDNLSSIARRLGTTVQSLVSMN GISNPNLIYAGQTLNY | 8.79 | 387-429 | | | |
| ***Enterococcus*** *faecalis* | * autolysin | YTVKSGDTLNKIAAQYGVSVANLRSWN GISGD LIFVGQKLIVK | 9.74 | 363-405(25) | 671 | P37710 | muramidase |
| | | YTVKSGDTLNKIAAQYGVTVANLRSWN GISGD LIFVGQKLIVK | 9.74 | 431-473(25) | | | |
| | | YTIKSGDTLNKIAAQYGVSVANLRSWN GISGD LIFAGQKIIVK | 9.74 | 499-541(25) | | | |
| | | YTIKSGDTLNKISAQFGVSVANLRSWN GIKGD LIFAGQTIIVK | 9.85 | 567-609(19) | | | |
| | | HTVKSGDSLWGLSMQYGISIQKIKQLN GLSGD TIYIGQTLKVG | 9.35 | 629-671 | | | |
| *hirae* | * *mur2* | YTVKSGDSVWGISHSFGITMAQLIEWN NIKNN FIYPGQKLTIK | 9.35 | 257-299(38) | 666 | P39046 | muramidase |
| | | YTVKSGDSVWKIANDHGISMNQLIEWN NIKNN FVYPGQQLVVS | 7.14 | 338-380(33) | | | |
| | | YTVKAGESVWSVSNKFGISMNQLIQWN NIKNN FIYPGQKLIVK | 9.91 | 414-456(32) | | | |
| | | YTVKAGESVWGVANKNGISMNQLIEWN NIKNN FIYPGQKLIVK | 9.64 | 489-531(33) | | | |
| | | YTVKAGESVWGVANKHHITMDQLIEWN NIKNN FIYPGQEVIVK | 7.31 | 565-607(15) | | | |
| | | YTVKAGESVWGVADSHGITMNQLIEWN NIKNN FIYPGQQLIVK | 7.15 | 623-665 | | | |
| ***Listeria*** *monocytogenes* | * P60 | VVVEAGDTLWGIAQSKGTTVDAIKKAN NLTTD KIVPGQKLQVN | 8.61 | 30- 72(130) | 484 | P21171 | adherence and invasion protein P60 |
| | | HAVKSGDTIWALSVKYGVSVQDIMSWN NLSSS SIYVGQKLAIK | 9.35 | 203-245 | | | |
| *innocua* | * P60 | VVVEAGDTLWGIAQSKGTTVDAIKKAN NLTTD KIVPGQKLQVN | 8.61 | 30- 72(129) | 481 | Q01836 | adherence and invasion protein P60 |
| | | HNVKSGDTIWALSVKYGVSVQDIMSWN NLSSS SIYVGQKPAIK | 8.35 | 201-243 | | | |
| *ivanovii* | * P60 | VVVEAGDTLWGIAQDKGTTVDALKKAN NLTSD KIVPGQKLQIT | 6.35 | 30- 72(125) | 524 | Q01837 | adherence and invasion protein P60 |
| | | YTVKSGDTIWALSSKYGTSVQNIMSWN NLSSS SIYVGQVLAVK | 9.37 | 198-240(73) | | | |
| | | YTVKSGDTLSKIATTFGTTVSKIKALN GLNSD NLQVGQVLKVK | 9.89 | 314-356 | | | |
| *seeligeri* | * P60 | VVVEAGDTLWGIAQDNGTTVDALKKAN KLTTD KIVPGQKLQVT | 6.35 | 30- 72(127) | 523 | Q01838 | adherence and invasion protein P60 |
| | | HTVKSGDTIWALSVKYGASVQDLMSWN NLSSS SIYVGQNIAVK | 8.64 | 200-242(77) | | | |
| | | YTVKSGDTLGKIASTFGTTVSKIKALN GLTSD NLQVGDVLKVK | 9.62 | 320-362 | | | |
| *welshimeri* | * P60 | VVVEAGDTLWGIAQSKGTTVDALKKAN NLTSD KIVPGQKLQVT | 8.61 | 30- 72(125) | 524 | M80348 | adherence and invasion protein P60 |
| | | HTVKSGDTIWALSVKYGASVQDLMSWN NLSSS SIYVGQKIAVK | 9.35 | 198-240(75) | | | |
| | | YTVKSGDSLSKIANTFGTSVSKIKALN NLTSD NLQVGTVLKVK | 9.89 | 316-358 | | | |
| *grayi* | * P60 | VVVASGDTLWGIASKTGTTVDQLKQLN KLDSD RIVPGQKLTIK | 9.42 | 30- 72(104) | 511 | Q01835 | adherence and invasion protein P60 |
| | | YKVKSGDTIWALSVKYGVPVQKLIEWN NLSSS SIYVGQTIAVK | 9.57 | 177-219(79) | | | |
| | | YKVQNGDSLGKIASLFKVSVADLTNWN NLNATITIYAGQELSVK | 8.59 | 299-342 | | | |
| ***Haemophilus*** *influenzae* | * *amiB* | HIVKKGESLGSLSNKYHVKVSDIIKLN QLKRK TLWLNESIKIP | 10.11 | 294-336 | 432 | P44493 | *N*-acetylmuramoyl-L-alanine amidase |
| | | HKVTKNQTLYAISREYNIPVNILLSLNPHLKNG KVITGQKIKLR | 10.49 | 387-430 | | | |
| | * *yebA* | YTVTEGDTLKDVLVLSGLDDSSVQPLIALDPELAHLKAGQQFYWI | 3.87 | 131-174 | 475 | P44693 | homologous to endopeptidase of *Staphylococcus* |
| | *lppB* | YKVNKGDTMFLIAYLAGIDVKELAALNNLSEPNYNLSLGQVLKIS | 6.40 | 147-190 | 405 | P44833 | outer membrane lipoprotein |
| *somnus* | *lppB* | YKVRKGDTMFLIAYISGMDIKELATLN NMSEPYHLSIGQVLKIA | 8.56 | 120-164 | 279 | L10653 | outer membrane lipoprotein |
| ***Helicobacter*** *pylori* | *dniR* | HVVLPKETLSSIAKRYQVSISNIQLAN DLKDS NIFIHQRLIIR | 10.02 | 319-361 | 372 | AE000654 | regulatory protein DniR |
| ***Pseudomonas*** *aeruginosa* | *lppB* | YIVRRGDTLYSIAFRFGWDWKALAARN GIAPPYTIQVGQAIQFG | 10.06 | 69-113 | 297 | P45682 | Lipoprotein |
| *Putida* | *nlpD* | YIVKPGDTLFSIAFRYGWDYKELAARN GIPAPYTIRPGQPIRFS | 9.77 | 44- 87 | 244 | Y19122 | lipoprotein |
| ***Sinohizobium*** *meliloti* | *nlpD* | IMVRQGDTVTVLARRFGVPEKEILKAN GLKSASQVEPGQRLVIP | 10.27 | 166-209 | 512 | U81296 | Lipoprotein |
| ***Synechocystis*** sp. | *nlpD* | HQVKEGESLWQISQAFQVDAKAIALAN SISTDTELQAGQVLNIP | 4.38 | 87-130 | 715 | D90915 | Lipoprotein |
| | *slr0878* | HVVKAGETIDSIAAQYQLVPATLISVN NQLSSGQVTPGQTILIP | 5.41 | 4- 47 | 245 | D90907 | Hypothetical protein |
| ***Aquifex*** *aeolicus* | *nlpD1* | YKVKKGDSLWKIAKEYKTSIGKLLELNPKLKNRKYLRPGEKICLK | 10.08 | 26- 70(24) | 349 | AE000700 | Lipoprotein |
| | | YRVKRGDSLIKIAKKFGVSVKEIKRVN KLKGN RIYVGQKLKIP | 10.95 | 95-137(37) | | | |
| | | YRVRRGDTLIKIAKRFRTSVKEIKRIN RLKGN LIRVGQKLKIP | 12.11 | 174-216 | | | |
| ***Volvox carteri*** | | YTIQPGDTFWAIAQRRGTTVDVIQSLN PGVVPTRLQVGQVINVP | 9.03 | 42- 85 | 309 | AF058716 | chitinase |
| ***f. nagariensis*** | | YTIQPGDTFWAIAQRRGTTVDVIQSLN PGVNPARLQVGQVINVP | 9.03 | 106-149 | | | |
| ***Staphylococcus*** *aureus* | ProtA | HVVKPGDTVNDIAKANGTTADKIAADN KLADKNMIKPGQELVVD | 5.58 | 431-474 | 524 | A04512 | protein A |
| | *lytN* | YTVKKGDTLSAIALKYKTTVSNIQNTN NIANPNLIFIGQKLKVP | 10.03 | 177-220 | 383 | AF106851 | autolysin homolog |
| ***Colletotrichum*** | *cih1* | HKVKSGESLTTIAEKYDTGICNIAKLN NLADPNFIDLNQDLQIP | 4.76 | 110-153(31) | 230 | AJ001441 | glycoprotein |
| *lindemuthianum* | | YSVVSGDTLTSIAQALQITLQSLKDAN PGVVPEHLNVGQKLNVP | 5.46 | 185-228 | | | |
| *Chlamydophila* | *amiB* | IVYREGDSLSKIAKKYKLSVTELKKIN KLDSD AIYAGQRLCLQ | 9.46 | 159-201 | 205 | AE001659.1 | N-Acetylmuramoyl-L-Ala Amidase |

| | | | | | | | |
|---|---|---|---|---|---|---|---|
| Pneumoniae | CPn0593 | YVVQDGDSLWLIAKRFGIPMDKIIQKN GLNHH RLFPGKVLKLP | 10.01 | 316-358 | 362 | AE001643 | |
| | NlpD | VVVKKGDFLERIARANHTTVAKLMQIN DLTTT QLKIGQVIKVP | 10.17 | 124-166 | 233 | AE001670 | Muramidase |
| | | YIVQEGDSPWTIALRNHIRLDDLLKMNDLDEYKARRLKPGDQLRIR | 8.64 | 188-233 | | | |
| Chlamydia trachomatis | NlpD | VIVKKGDFLERIARSNHTTVSALMQLN DLSST QLQIGQVLRVP | 9.99 | 138-180 | 245 | AE001348 | Muramidase |
| | | YVVKEGDSPWAIALSNGIRLDELLKLNGLDEQKARRLRPGDRLRIR | 10.00 | 200-245 | | | |
| | papQ | HIVKQGETLSKIASKYNIPVVELKKLN KLNSD TIFTDQRIRLP | 9.89 | 155-197 | 200 | AE001330 | |
| Prevotella intermedia | phg | HTVRSNESLYDISQQYGVRLKNIMKAN RKIVKRGIKAGDRVVL | 10.72 | 266-309 | 309 | AF017417 | hemagglutinin |
| Leuconostoc oenos Ø10MC | lys | YTVQSGDTLGAIAAKYGTTYQKLASLN GIGSPYIIIPGEKLKVS | 9.23 | 335-378 | 432 | | endolysin |
| | | YKVASGDTLSAIASKYGTSVSKLVSLN GLKNANYIYVGENLKIK | 9.68 | 389-432 | | | |
| Oenococcus oeni ØfOg44 | Lys44 | YTVRSGDTLGAIAAKYGTTYQKLASLN GIGSPYIIIPGEKLKVS | 9.58 | 335-378 | 432 | AF047001 | Lysin |
| | | YKVASGDTLSAIASKYGTSVSKLVSLN GLKNANYIYVGQTLRIK | 9.95 | 389-432 | | | |
| Thermotoga maritima TM0409 | | YKVQKNDTLYSISLNFGISPSLLLDWN PGLDPHSLRVGQEIVIP | 5.49 | 26- 69 | 271 | AE001720 | |
| | | YTVKKGDTLDAIAKRFFTTATFIKEAN QLKSY TIYAGQKLFIP | 9.73 | 76-118 | | | |
| TM1686 | | HVVKRGETLWSIANQYGVRVGDIVLIN RLEDPDRIVAGQVLKIG | 8.76 | 212-255 | 395 | AE001809 | |
| Treponema pallidum | dniR | HTIRSGDTLYALARRYGLGVDTLKAHN RAHSATHLKIGQKLIIP | 10.58 | 607-650 | 779 | AE001237 | membrane-bound lytic murein transglycosylase D |
| | | HVVQQGDTLWSLAKRYGVSVENLAEEN NLAVDATLSLGMILKTP | 4.81 | 734-777 | | | |
| TP0155 | | YEVREGDVVGRIAQRYDISQDAIISLN KLRSTRALQVGQLLKIP | 9.58 | 87-130 | 371 | AE001200 | |
| | TP0444 | HVIAKGETLFSLSRRYGVPLSALAQAN NLANVHQLVPGQRIVVP | 10.98 | 67-110 | 342 | AE001221 | |
| Borrelia burgdorferi | BB0262 | HKIKPGETLSHVAARYQITSETLISFN EIKDVRNIKPNSVIKVP | 9.72 | 183-226(6) | 417 | AE001137 | Hypothetical protein |
| | | YIVKKNDSISSIASAYNVPKVDILDSN NLDNE VLFLGQKLFIP | 4.58 | 233-275 | | | |
| | * BB0625 | YKVVKGDTLFSIAIKYKVKVSDLKRIN KLNVD NIKAGQILIIP | 10.02 | 44- 86(28) | 697 | AE001164 | N-acetylmuramoyl-L-alanine amidase |
| | | YTAKEGDTIESISKLVGLSQEEIIAWN DLRSK DLKVGMKLVLT | 5.00 | 115-157(7) | | | |
| | | YMVRKGDSLSKLSQDFDISSKDILKFN FLNDD KLKIGQQLFLK | 9.20 | 165-207(8) | | | |
| | | HYVKRGETLGRIAYIYGVTAKDLVALN GNRAI NLKAGSLLNVL | 10.05 | 216-258(27) | | | |
| | | HSVAVGETLYSIARHYGVLIEDLKNWN NLSSN NIMHDQKLKIF | 7.41 | 286-328 | | | |
| | BB0761 | YKVKKGDTFFKIANKINGWQSGIATIN LLDSP AVSVGQEILIP | 9.34 | 59-102 | 295 | AE001176 | |
| Lactobacillus Øg1e | * lys | YTVVSGDSWWKIAQRNGLSMYTLASQN GKSIYSTIYPGNKLIIK | 9.83 | 399-442 | 442 | X90511 | lysin |
| Bacillus subtilis | * lytE | IKVKKGDTLWDLSRKYDTTISKIKSEN HLRSD IIYVGQTLSIN | 9.55 | 28- 70(17) | 334 | U38819.1 | D-Glutamate-M-diaminopimlate endopeptidase |
| | | YKVKSGDSLWKISKKYGMTINELKKLN GLKSD LLRVGQVLKLK | 10.16 | 88-130(20) | | | |
| | | YKVKSGDSLSKIASKYGTTVSKLKSLN GLKSD VIYVNQVLKVK | 10.03 | 151-193 | | | |
| | spoVID | CIVQQEDTIERLCERYEITSQQLIRMN SLALDDELKAGQILYIP | 4.20 | 525-568 | 575 | P37963 | stage VI sporulation protein D |
| | yaaH | MVKQGDTLSAIASQYRTTTNDITETN EIPNPDSLVVGQTIVIP | 3.89 | 1- 43(5) | 427 | P37531 | Hypothetical protein |
| | | YDVKRGDTLTSIARQFNTTAAELARVN RIQLNTVLQIGFRLYIP | 10.32 | 49- 92 | | | |
| | yhdD | IKVKSGDSLWKLAQTYNTSVAALTSAN HLSTT VLSIGQTLTIP | 9.56 | 29- 71(22) | 488 | Y14079 | Hypothetical protein |
| | | YTVKSGDSLWLIANEFKMTVQELKKLN GLSSD LIRAGQKLKVS | 9.62 | 94-136(39) | | | |
| | | YKVQLGDSLWKIANKVNMSIAELKVLN NLKSD TIYVNQVLKTK | 9.72 | 176-218(23) | | | |
| | | YTVKSGDSLWKIANNYNLTVQQIRNIN NLKSD VLYVGQVLKLT | 9.65 | 242-284(24) | | | |
| | | YTVKSGDSLWVIAQKFNVTAQQIREKN NLKTD VLGVGQKLVIS | 9.72 | 309-351 | | | |
| | yojL | IKVKSGDSLWKLSRQYDTTISALKSEN KLKST VLYVGQSLKVP | 9.93 | 29- 71(18) | 414 | Z99114 | similar to cell wall binding protein |
| | | YTVAYGDSLWMIAKNHKMSVSELKSLN SLSSD LIRPGQKLKIK | 9,81 | 90-132(26) | | | |
| | | YTVKLGDSLWKIANSLNMTVAELKTLN GLTSD TLYPKQVLKIG | 9.27 | 159-201(25) | | | |
| | | YKVKAGDSLWKIANRLGVTVQSIRDKN NLSSD VLQIGQVLTIS | 9.84 | 227-269 | | | |
| | yocH | ITVQKGDTLWGISQKNGVNLKDLKEWN KLTSD KIIAGEKLTIS | 9.25 | 28- 70(9) | 287 | AF027868 | similar to papQ |
| | | YTIKAGDTLSKIAQKFGTTVNNLKVWN NLSSD MIYAGSTLSVK | 9.64 | 80-122 | | | |
| | ykvP | HHVTPGETLSIIASKYNVSLQQLMELN HFKSD QIYAGQIIKIR | 8.65 | 345-387 | 399 | Z99111 | Hypothetical protein |
| | * xlyB | YHVKKGDTLSGIAASHGASVKTLQSIN HITDPNHIKIGQVIKLP | 9.72 | 179-222 | 317 | Z99110 | N-acetylmuramoyl-L-alanine amidase |
| | yrbA | HIVQKGDSLWKIAEKYGVDVEEVKKLNTQLSNPDLIMPGMKIKVP | 8.51 | 4- 48 | 387 | Z99118 | similar to spore coat protein |
| | ydhD | HIVGPGDSLFSIGRRYGASVDQIRGVN GLDET NIVPGQALLIP | 5.49 | 4- 46 | 439 | Z99107 | |
| | ykuD | YQVKQGDTLNSIAADFRISTAALLQAN PSLQA GLTAGQSIVIP | 6.10 | 4- 46 | 164 | Z99111 | |
| ØPBSX | * xlyA | YVVKQGDTLTSIARAFGVTVAQLQEWN NIEDPNLIRVGQVLIVS | 4.65 | 161-204 | 297 | P39800 | N-acetylmuramoyl-L-alanine amidase |
| Ø PZA | * orf15 | YKVKSGDNLTKIAKKHNTTVATLLKLNPSIKDPNMIRVGQTINVT | 10.23 | 163-207(6) | 258 | P11187 | lysozyme |
| (=Ø-29) | | HKVKSGDTLSKIAVDNKTTVSRLMSLNPEITNPNHIKVGQTIRLS | 10.17 | 214-258 | | P07540 | |
| Ø B103 | * orf15 | HVVKKGDTLSEIAKKIKTSTKTLLELNPTIKNPNKIYVGQRINVG | 10.11 | 165-209(9) | 263 | X99260 | lysozyme |
| | | YKIKRGETLTGIAKKNKTTVSQLMKLNPNIKNANNIYAGQTIRLK | 10.61 | 219-263 | | | |
| sphaericus | * Pep I | ILIRPGDSLWYFSDLFKIPLQLLLDSNRNINPQ LLQVGQRIQIP | 8.86 | 3- 46(6) | 396 | X69507 | carboxypeptidase I |
| | | YTITQGDSLWQIAQNKNLPLNAILLVNPEIQPS RLHIGQTIQVP | 7.15 | 53- 96 | | | |
| Salmonella dublin | nlpD | YTVKKGDTLFYIAWITGNDFRDLAQRN SISAPYSLNVGQTLQVG | 8.64 | 121-164 | 377 | AJ006131 | |
| Escherichia coli | * yebA | YVVSTGDTLSSILNQYGIDMGDISQLAAADKELRNLKIGQQLSWT | 4.13 | 77-121 | 419 | P24204 | homologous to endopeptidase of Staphylococcus |
| | mltD | YTVRSGDTLSSIASRLGVSTKDLQQWN KLRGS KLKPGQSLTIG | 10.58 | 343-385(16) | 452 | P23931 | MEMBRANE-BOUND LYTIC MUREIN TRANSGLYCOSYLASE D PRECURSOR |

| | | | | | | | |
|---|---|---|---|---|---|---|---|
| | | YRVRKGDSLSSIAKRHGVNIKDVMRWN SDTAN LQPGDKLTLF | 10.18 | 402-443 | | | |
| | UUG | YTVKRGDTLYRISRTTGTSVKELARLN GISPPYTIEVGQKLKLG | 10.16 | 50- 93 | 259 | U28375 | Hypothetical protein |
| | nlpD | YTVKKGDTLFYIAWITGNDFRDLAQRN NIQAPYALNVGQTLQVG | 8.64 | 123-166 | 379 | P33648 | Lipoprotein |
| *Drosophila melanogaste* | r L82 | YTVGNRDTLTSVAARFDTTPSELTHLN RLNSS FIYPGQQLLVP | 7.15 | 329-371 | 1270 | AF125384 | |
| *Caenorhabditis elegans* | F43G9.2 | RKVKNGDTLNKLAIKYQVNVAEIKRVN NMVSEQDFMALSKVKIP | 10.01 | 12- 55 | 179 | Z79755 | |
| *Caenorhabditis elegans* | F52E1.13 | YTITETDTLERVAASHDCTVGELMKLN KMASR MVFPGQKILVP | 7.08 | 24- 66 | 819 | U41109 | |
| *Caenorhabditis elegans* | | TEIKSGDSCWNIASNAKISVERLQQLN KGMKCDKLPLGDKLCLA | 8.32 | 23- 66(11) | 1614 | U64836 | PUTATIVE ENDOCHITINASE |
| | | LKLKAEDTCFKIWSSQKLSERQFLGMN EGMDCDKLKVGKEVCVA | 7.84 | 78-121(21) | | | |
| | | HKIQKGDTCFKIWTTNKISEKQFRNLN KGLDCDKLEIGKEVCIS | 8.65 | 143-186(21) | | | |
| | | LKIKEGDTCYNIWTSQKISEQEFMELN KGLDCDKLEIGKEVCVT | 4.54 | 208-251(19) | | | |
| | | YRFKKGDTCYKIWTSHKMSEKQFRALN RGIDCDRLVPGKELCVG | 9.35 | 271-314(20) | | | |
| | | ITVKPGDTCFSIWTSQKMTQQQFMDIN PELDCDKLEIGKEVCVT | 4.21 | 335-378(23) | | | |
| | | VKINPGDTCFNIWTSQRMTQQQFMDLN KRLDCDKLEVGKEVCVT | 6.30 | 402-445(21) | | | |
| | | VQINPGDTCFKIWSAQKLTEQQFMELN KGLDCDRLEVGKEVCIA | 4.60 | 467-510(37) | | | |
| | | TEVKEGDTCFKIWSAHKITEQQFMEMN RGLDCNRLEVGKEVCIV | 5.12 | 548-591(44) | | | |
| | | IKVKEGDTCFKIWSAQKMTEQQFMEMN RGLDCNKLMVGKEVCVS | 7,85 | 636-679(66) | | | |
| | | ATITPGNTCFNISVAYGINLT DLQ KTYDCKALEVGDTICVS | 3.99 | 746-786(8) | | | |
| | | IEVIKGDTCWFLENAFKTNQTEMERAN EGVKCDNLPIGRMMCVW | 4.67 | 795-838 | | | |
| *Caenorhabditis elegans* | | HTIKSGDTCWKIASEASISVQELEGLN SKKSCANLAVGLSEQEF | 5.01 | 23- 66(51) | 1484 | U70858 | PUTATIVE ENDOCHITINASE |
| | | IHVKEGDTCYTIWTSQHLTEKQFMDMN EELNCGMLEIGNEVCVD | 4.12 | 118-161(25) | | | |
| | | ATVTPGSSCYTISASYGLNLAELQTTY NCDALQVDDTICVS | 3.07 | 187-226(9) | | | |
| | | IEILNGDTCGFLENAFQTNNTEMEIAN EGVKCDNLPIGRMMCVW | 3.85 | 236-279 | | | |
| *Bacillus subtilis* | # ypbE | HTVQKKETLYRISMKYYKSRTGEEKIRAYNHLNGNDVYTGQVLDIP | 9.45 | 191-136 | 240 | L47648 | |
| *Citrobacter fruendii* | # eae | YTLKTGESVAQLSKSQGISVPVIWSLNKHLYSSESEMMKASPGQQIILP | 8.59 | 65-113 | 936 | Q07591 | |
| *Escherichia coli* | # eae | YTLKTGETVADLSKSQDINLSTIWSLNKHLYSSESEMMKAAPGQQIILP | 5.65 | 65-113 | 934 | P43261 | NECESSARY FOR CLOSE (INTIMATE) ATTACHMENT OF BACTERIA |
| ***Micrococcus luteus*** | # rpf | *IVVKSGDSLWTLANEYEVEGGWTALYEANKGAVSDAAVIYVGQELVL* | 3.85 | 171-218 | 220 | Z96935 | BACTERIAL CYTOKINE |
| *Bacillus subtilis* | # yneA | IEVQQGDTLWSIADQVADTKKINKNDFIEWVADKNQLQTSDIQPGDELVIP | 3.81 | 40- 90 | 105 | Z73234 | |
| *Streptococcus pyogenes* | # | YTVKYGDTLSTIAEAMGIDVHVLGDINHIANIDLIFPDTILTANYNQHGQATTLT | 4.23 | 47-103 | 393 | U09352 | |
| *Bacillus subtilis* | # xkdP | YTVKKGDTLWDIAGRFYGNSTQWRKIWNANKTAMIKRSKRNIRQPGHWIFPGQKLKIP | 11.23 | 176-234 | 235 | P54335 | |
| *Bacillus subtilis* | # yqbP | YTVKKGDTLWDIAGRFYGNSTQWRKIWNANKTAMIKRSKRNIRQPGHWIFPGQKLKIP | 11.23 | 177-234 | 235 | G1225954 | |
| *Bacillus subtilis* | # | YTVKKGDTLWDLAGKFYGDSTKWRKIWKVNKKAMIKRSKRNIRQPGHWIFPGQKLKIP | 10.75 | 161-218 | 219 | P45932 | |

a) Proteins listed were obtained by a homology search in the SWISSPROT, PIR, and Genbank databases with the repeats of AcmA using the BLAST program.
b) * ; genes encoding cell wall hydrolases.
# ; proteins containing repeats that are longer than the consensus sequence.
c) The number of aa residues between the repeats are given between brackets.
d) Number of aa of the primary translation product.
e) Genbank accession number.

Table B.

| Calculated pI's of individual repeat sequences of the AcmA and AcmD protein anchors. | | | |
|---|---|---|---|
| AcmA anchor domain | | AcmD anchor domain | |
| Repeat | Calculated pI | Repeat | Calculated pI |
| A1 | 9.75 | D1 | 4.15 |
| A2 | 9.81 | D2 | 3.78 |
| A3 | 10.02 | D3 | 4.15 |
| A1A2A3 | 10.03 | D1D2D3 | 3.85 |

Table C.

| Hybrid protein anchors composed of different AcmA and AcmD repeat sequences and their calculated pI's. | | |
|---|---|---|
| **Composition** | | |
| AcmA-repeat sequence | AcmD-repeat sequence | Calculated pI |
| A1A2A3 | - | 10.03 |
| A1A2 | D1 | 9.53 |
| A1A2A3 | D1D2D3 | 8.66 |
| A1 | D2 | 8.45 |
| A3 | D1D2 | 7.39 |
| A1A2 | D1D2D3 | 6.08 |
| A3 | D1D2D3 | 5.07 |
| A1 | D1D2D3 | 4.37 |
| - | D1D2D3 | 3.85 |

Table D.

| Adherence of *Lactococcus lactis* NZ9000 carrying plasmids pNG304, pNG3041, pCWS1a, pCWS2a, and pCWS2a with human intestine 407 cells. | | | |
|---|---|---|---|
| Strain | 407 cells [a)] | Bacteria [b)] | Mean value |
| NZ9000(pNG304) | 54 | 255 | 4.7 |
| NZ9000(pNG304 | 78 | 447 | 5.7 |
| NZ9000(pCWS1a) | 73 | 1342 | 18.4 |
| NZ9000(pCWS2a) | 80 | 2131 | 27.4 |
| NZ9000(pCWS3a) | 64 | 1970 | 30.8 |

a) Number of whole epithelial cells in 10 randomly chosen fields of view in light microscopy

b) Number of lactococcal cells in contact with Henle cells

FIGURE LEGENDS

Figure 1. SDS-PAGE stained with Coomassie Brilliant Blue (molecular weight markers indicated in kDa on the left of lane A, and purified detergent-solubilized MscL in lane B), and the Western blot (lane C).

Figure 2. Electrospray ionization mass spectrometry of G22C-MscL-6His and its MTSES conjugate. (solid line) Spectrum of G22C- MscL-6His. Not all peptides that are present in the sample are indicated in the spectrum. (broken line) Spectrum of MTSES conjugated G22C-MscL-6His. The masses are indicated at the peaks and show that all proteins are conjugated

Figure 3. Equilibrium centrifugation on sucrose gradients of proteoliposomes. 6His-MscL purified with Triton X-100 and incorporated in liposomes titrated with 4.0 mM Triton X-100 (Rsat; open squares) and liposomes titrated with 10.0 mM Triton X-100 (Rsol; closed squares). After centrifugation, the gradients were fractionated (0.5 mL) and assayed for the presence of lipids and protein. All protein, as determined by Western blotting as shown in the inset, is shown to be associated with the lipids, as determined by measuring fluorescence (AU) of $R_{18}$.

Figure 4. Freeze-fracture image of proteoliposome showing the MscL channel protein as a transmembrane vesicle (white box).

Figure 5. Patch-clamp recordings of channel activities at -20 mV from MscL reconstituted into liposomes of different lipid compositions as indicated in the figures. Pressure in the pipette, relative to atmospheric, is shown in the lower traces, and recording of the current through a patch of membrane excised from a blister is shown in the upper traces.

Figure 6. Pressure dependence of the MscL channel reconstituted in liposomes of different lipid composition. Open probability in the patch of a membrane with a lipid composition of PC: PS, 90:10 m/m (A) and PC: PE, 70:30, m/m (B) versus the applied pressure. Smooth curves are Boltzman fits.

Figure 7. Calcein efflux from liposomes with (closed circles) and without (closed squares) MscL as a function of a decrease in Osmolality of the external medium. A small volume (typically 20 µL) containing (proteo)liposomes in iso-osmotic buffer, is rapidly diluted with buffer of decreasing osmolality and calcein release was determined by dividing the fluorescence at 100 sec after dilution by the total fluorescence obtained after Triton X-100 lysis.

Figure 8. Calcein release under iso-osmotic condition mediated by conjugated G22C-MscL-6His. Calcein release of MTSES conjugated G22C-MscL-6His channel protein reconstituted into liposomes (PC:Chol, 60:40, m/m) (closed circles). Liposomes with the same lipid composition and sample treatment as above but without MscL (closed squares).

Figure 9. Effect of 5 mol% DGPE-PEG(2000) on the calcein release from liposomes (PC:Chol, 60:40, m/m). Calcein release from PC:Chol:DGPE-PEG(2000) liposomes in the presence of buffer (closed triangles), rat plasma (closed circles), human plasma (closed squares). Calcein release from liposomes without DGPE-PEG(2000) (closed diamond).

Figure 10. Structure of DTCP1 in the open conformation (A) and in the closed conformation (B). The molecule can reversibly isomerize depending on the wavelength of the absorbed light.

Figure 11. ESI-MS analysis of the DTCP1 conjugation to a single cysteine mutant of MscL at position 22. Unconjugated G22C-MscL with expected mass of 15697 Da (light grey line). BI conjugated G22C-MscL with a 156 Da mass increase (thick black line). DTCP1 conjugated G22C-MscL with a 344 Da mass increase (thin black line).

Figure 12. Absorption spectroscopy of DTCP1 switching after conjugation to G22C-MscL. DTCP1 switch in the open conformation (Fig. 10-A) shown in light grey and switch in closed conformation (Fig. 10-B) shown in black. Inset: biphasic switching between closed and open conformation of DTCP1 in membrane-reconstituted modified G22C-MscL.

Figure 13. Structure of 2-Bromo-3-(5-imidazolyl)propionic acid (BI). BI reacts with the only free sulfhydryl of G22C-MscL, supposedly mimicking the G22H-MscL pH-dependent properties.

Figure 14. Dependence of the amount of membrane tension needed to open the MscL channel on the lipid composition of the membrane. Increase in DOPE content results in a decrease in the membrane tension needed to open the channel.

Figure 15. Western blot that shows effect of pH supernatant on binding of MSA2::cD to TCA-pretreated *L. lactis* cells. As before, the Western blot shows the amount of MSA2::cD that was bound by the cells. In addition, the amount of MSA2::cD that was not bound and remained in the medium after binding is shown. The arrow indicates the expected position for pro-MSA2::cD and the asterisk the position of mature MSA2::cD.

1. pH during binding 6.2, cells.
2. pH during binding 6.2, supernatant after binding.
3. pH during binding 3.2, cells.

4. pH during binding 3.2, supernatant after binding.
5. Positive control: *L. lactis*, TCA-pretreated with bound MSA2::cA at pH6.2.

It is clearly visible that MSA2::cD binds better at pH 3.2 than at pH 6.2 (compare lanes 1 and 3).

Figure 16. Schematic presentation of the CWS domain containing protein hybrids used for adherence. SS: signal sequence of *L. lactis* PrtP; PS: prosequence of *L. lactis* PrtP; MSA2: merozoite surface antigen 2 of *P. falciparum* (reporter protein); CWS: cell wall spanning domain *of L. lactis* PrtP; CWA: cell-wall anchor (covalent) of *L. lactis* PrtP.

Figure 17. Adherence *of L. lactis* NZ9000 cells expressing (A) MSA2 fused to AcmA protein anchor and (B) MSA2 fused to three CWS domains (M::C3a) to human intestine 407 Henle cells.

Figure 18. Gelators, compounds A, B, C, D, E, Z.

Figure 19 Reaction scheme

Figure 20 Compounds Z, F, G, H, I

Figure 21 Compounds Z, F, J

Figure 22 Effect of irradiation

Figure 23

(a) UV-VIS spectra of D in toluene of a solution (0.35 mM) (—)and of a gel (1.8mM) (---). (b) UV-Vis spectra of D in toluene after irradiation at $\lambda$ = 313nm of a solution of (0.35 mM) (—) and of a gel (---). (c) CD spectra at 15 °C of a toluene gel of D (1.8 mM) (—) and of a solution (0.35 mM) (---). (d) CD spectra at 15 °C of a toluene gel of D (1.8 mM) after irradiation at $\lambda$ = 313 nm (—), and after heating and cooling (---) and of a diluted solution after irradiation at $\lambda$ = 313 nm (···) (PSS)

**Claims**

**1.** A delivery vehicle for delivering a substance of interest to a predetermined site, said vehicle comprising said substance and a means for inducing availability of at least one compartment of said vehicle toward the exterior, thereby allowing access of said substance to the exterior of said vehicle at said predetermined site.

**2.** A delivery vehicle according to claim 1, wherein inducing availability is achieved by inducing opening of said at least one compartment.

**3.** A delivery vehicle according to claim 1 or claim 2, wherein said induction allows passage of said substance to the exterior of said vehicle.

**4.** A delivery vehicle according to any one of claims 1-3, further comprising a targeting means for directing said vehicle to said predetermined site.

**5.** A delivery vehicle according to any one of claims 1-4, wherein said vehicle comprises a binding molecule capable of specifically binding a binding partner.

**6.** A delivery vehicle according to claim 5, wherein said binding molecule comprises PrtP, AcmA, AcmD or a functional part, derivative and/or analogue thereof.

**7.** A delivery vehicle according to any one of claims 1-6, wherein said means for inducing availability of at least one compartment comprises a proteinaceous channel.

**8.** A delivery vehicle according to claim 7, wherein said proteinaceous channel comprises a solute channel.

**9.** A delivery vehicle according to claim 7 or claim 8, wherein said proteinaceous channel comprises a mechanosensitive channel.

**10.** A delivery vehicle according to any one of claims 7-9, wherein said proteinaceous channel comprises a mechanosensitive channel of large conductance (MscL) or a functional equivalent thereof.

**11.** A delivery vehicle according to any one of claims 1-10, wherein said vehicle comprises a film enclosing said at least one compartment.

**12.** A delivery vehicle according to claim 11, wherein said vehicle comprises a liposome.

**13.** A delivery vehicle according to claim 11 or claim 12, wherein said proteinaceous channel is located in said film.

**14.** A delivery vehicle according to claim 13, wherein said film is formulated to allow preferential opening of said channel at said predetermined site.

**15.** A delivery vehicle according to any one of claims 11-14, wherein said film comprises positively and/or neutrally charged lipids.

**16.** A delivery vehicle according to claim 15, wherein said film consists predominantly of positively and/or neutrally charged lipids.

**17.** A delivery vehicle according to any one of claims 11-16, wherein said vehicle comprises an asymmetrical bilayer.

**18.** A delivery vehicle according to any one of claims 7-17, wherein said vehicle comprises a light sensitive lipid and/or MscL.

**19.** A delivery vehicle according to claim 10 or 18, wherein said MscL is a mutant MscL.

**20.** A delivery vehicle according to claim 19, wherein said mutant allows preferential availability of said compartment at, or in the vicinity of, said predetermined site.

**21.** A delivery vehicle according to any one of claim 1-20, wherein said means for inducing availability of said at least one compartment toward the exterior comprises light.

**22.** A delivery vehicle according to any one of claim 1-21, wherein said means for inducing availability of said at least one compartment toward the exterior comprises an pH, salt concentration and/or temperature.

**23.** A delivery vehicle according to any one of claims 1-22, wherein said vehicle is formulated and prepared for use in a human.

**24.** A delivery vehicle according to any one of claims 1-23, wherein vehicle comprises a gelator or a thickening agent.

**25.** A delivery vehicle according to claim 24, wherein said gelator and/or thickening agent comprises an organogel, a hydrogel or a combination thereof.

**26.** A delivery vehicle according to claim 25, comprising a N,N'-disubstituted aldaramides, a N,N'-disubstituted pentaramides or a derivative and/or combination thereof.

**27.** A delivery vehicle according to claim 26, wherein said vehicle comprises a compound of formula II

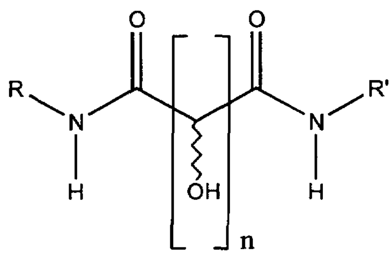

(II),

wherein n is 3 or 4, and wherein R and R' represent the same or different substituents chosen from the group of substituted or unsubstituted, branched, possibly aromatic groups containing, cyclic or linear alkyl, alkenyl, alkynyl groups having from 1 to 40 carbon atoms.

**28.** A delivery vehicle according to any one of claims 24-27, wherein said gelling and or thickener agent comprises a light-switchable gelator.

**29.** A delivery vehicle according to claim 28, wherein said light-switchable gelator comprises the formula (III):

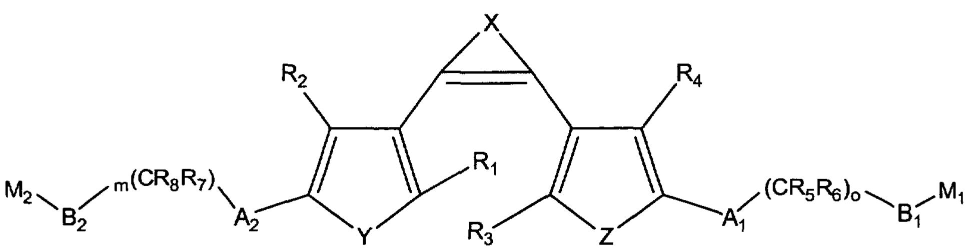

(III)

wherein

- X is chosen from the group of the moieties $-(CH_2)_n-$, $-(CF_2)_n-$, -C(=O)-O-C(=O)-and -C(=O)-NR-C(=O)-, wherein n is 3 or 4 and wherein R is hydrogen, a (cyclo)alkyl group or an aryl group;
- Y and Z each are nitrogen or sulfur;
- $R_1$ and $R_3$ each are an alkyl group;
- $R_2$ and $R_4$ each are hydrogen or an alkyl group;
- $A_1$ and $A_2$ each are absent or are an aryl group;
- $R_5$, $R_6$, $R_7$, and $R_8$ each are hydrogen, an alkyl group or an aryl group;
- m and o each are integers chosen from the group of 0, 1, 2, 3, and 4;
- $B_1$ and $B_2$ are hydrogen bonding moieties; and
- $M_1$ and $M_2$ each are an aryl group, a (cyclo)alkyl group, or $-CR_9R_{10}R_{11}$, wherein $R_9$, $R_{10}$ and $R_{11}$ each are hydrogen, a (cyclo)alkyl group, an aralkyl group or an aryl group. It is to be noted that all symbols defined above may be chosen to have a meaning as defined, independent on the meaning of any of the other symbols, unless otherwise indicated herein.

**30.** A delivery vehicle according to any one of claims 24-29, wherein said means for inducing availability of said at least one compartment comprises a viscous material that upon applying a signal becomes less viscous.

**31.** A delivery vehicle according to claim 30, wherein said signal comprises radiation and/or an electrical field.

**32.** A delivery vehicle according to any one of claims 11-31, wherein said membrane comprises hydrophobin or a functional equivalent thereof.

**33.** A delivery vehicle according to any one of claims 11-32, wherein said vehicle comprises a cationic amphiphile.

**34.** A delivery vehicle according to claim 33, wherein said cationic amphiphile comprises an aromatic ring comprising a nitrogen atom according to the following formula (I)

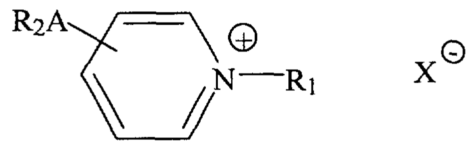

in which:

R1 is selected from the group consisting of:

a branched or linear (C6-C24) carbon chain optionally interrupted by one or more heteroatoms, optionally containing one or more functional groups, optionally containing one or more double or triple carbon-carbon bonds or combinations of double and triple carbon-carbon bonds, optionally being substituted or combinations thereof
and

R3-Ar-A-R2 in which R3 is a C2-C10 carbon chain, Ar is an aromatic or a heteroaromatic ring optionally to which relative to R3 in the ortho-, meta-, or para-position A-R2 is attached,
A is attached to the aromatic ring in the ortho-, meta- or para-position relative to the nitrogen atom in the aromatic ring,
A is selected from the group consisting of $CH_2$, O, S, ,S-S, NH, OC=O, O=CO, SC=O, O=CS, NHC=O, O=CNH, CH=N, N=CH, phosphate, alkylphosphate and phosphonate,
R2 represents a branched or linear (C6-C24) carbon chain optionally interrupted by one or more heteroatoms, optionally containing one or more functional groups, optionally containing one or more double or triple carbon-carbon bonds or combinations of double and triple carbon-carbon bonds, optionally being substituted or combinations thereof,
$X^-$ is a physiologically acceptable anion.

**35.** A delivery vehicle according to any one of claims 1-34, wherein said substance comprises nucleic acid (nog uit te breiden aan de hand van aan te leveren informatie.)

**36.** A delivery vehicle according to any one of claims 1-34, wherein said substance comprises a small hydrophilic molecule capable of passing through an activated MscL.

**37.** A delivery vehicle according to claim 36, wherein said small molecule comprises a peptide.

**38.** A delivery vehicle according to claim 36, wherein said small molecule comprises a diameter smaller than 60 Å.

**39.** Use of a delivery vehicle according to any one of claims 1-38, for the preparation of a medicament.

**40.** Use of a delivery vehicle according to any one of claims 1-38, for controlling availability of said substance.

**41.** Use of a delivery vehicle according to any one of claims 1-38, for making said substance specifically available to the exterior of said vehicle at said predetermined site.

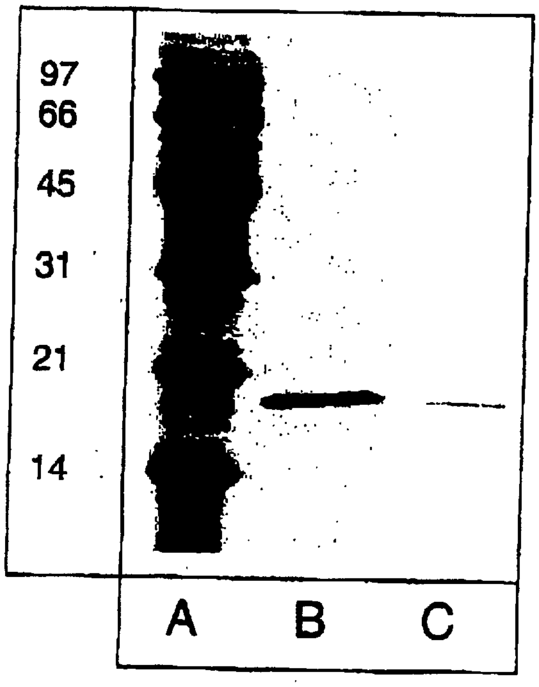


Figure 1

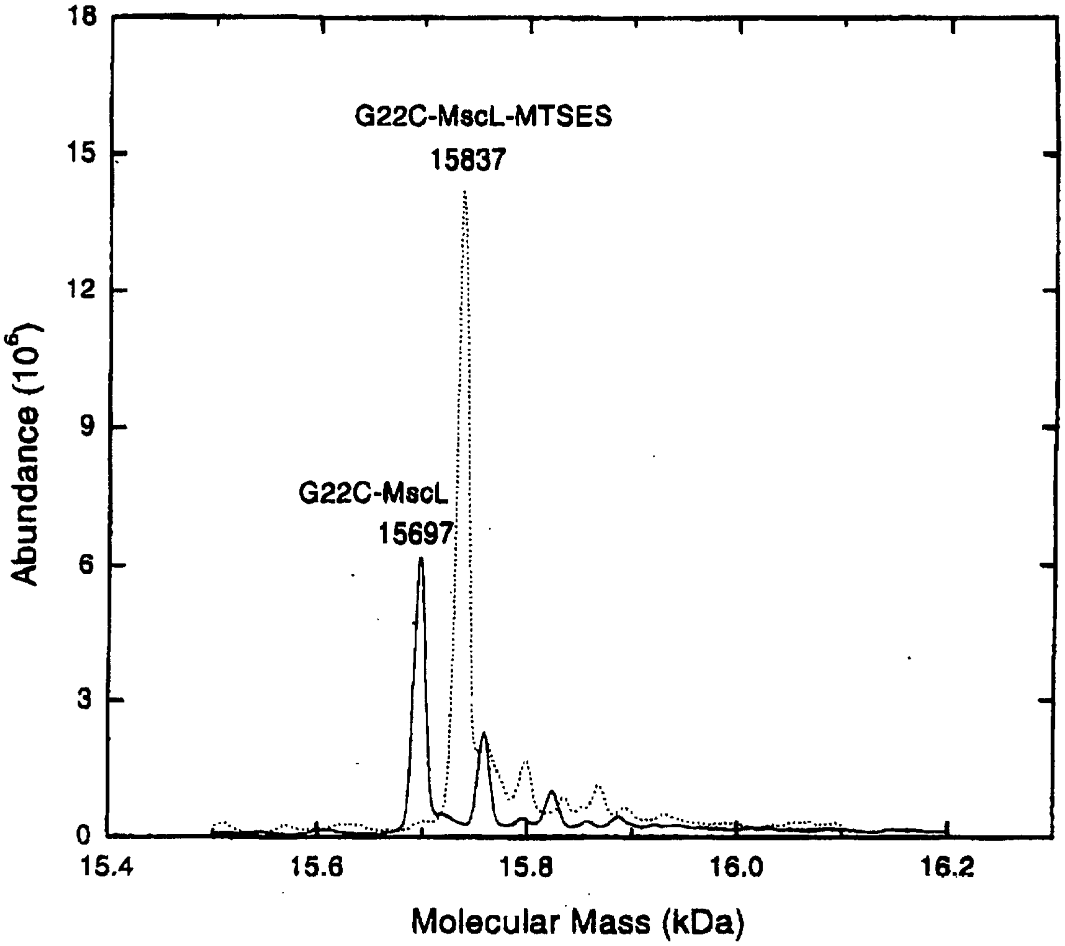
G22C-MscL-MTSES
15837
G22C-MscL
15697
Abundance (10^6)
18
15
12
9
6
3
0
15.4
15.6
15.8
16.0
16.2
Molecular Mass (kDa)

Figure 2

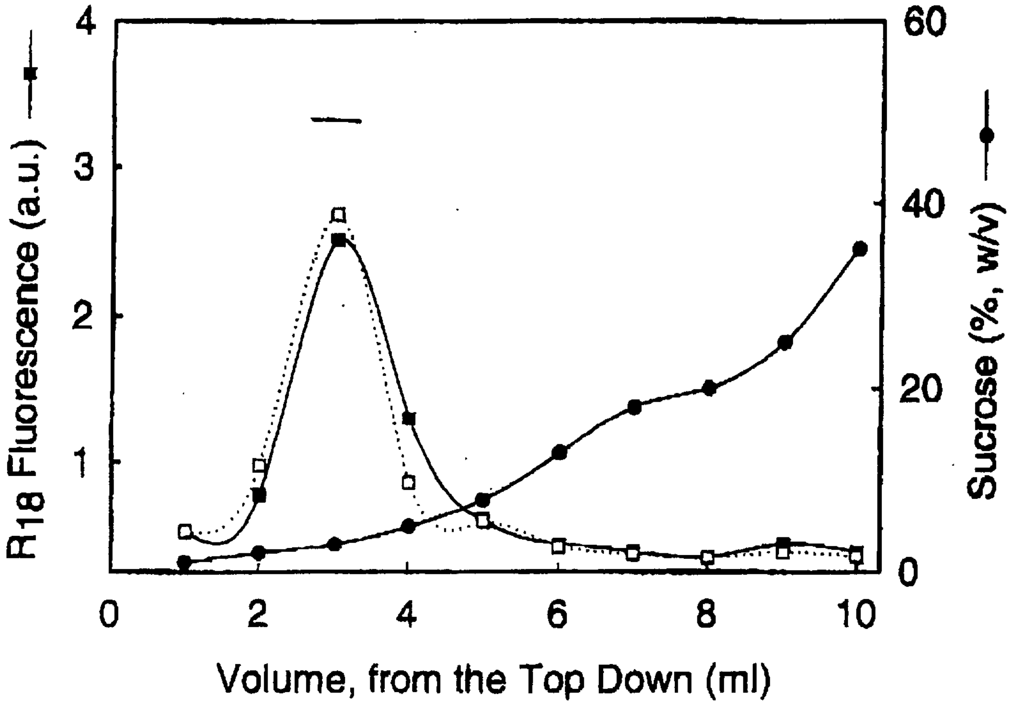
R18 Fluorescence (a.u.)
Sucrose (%, w/v)
Volume, from the Top Down (ml)
0
1
2
3
4
0
20
40
60
0
2
4
6
8
10

Figure 3

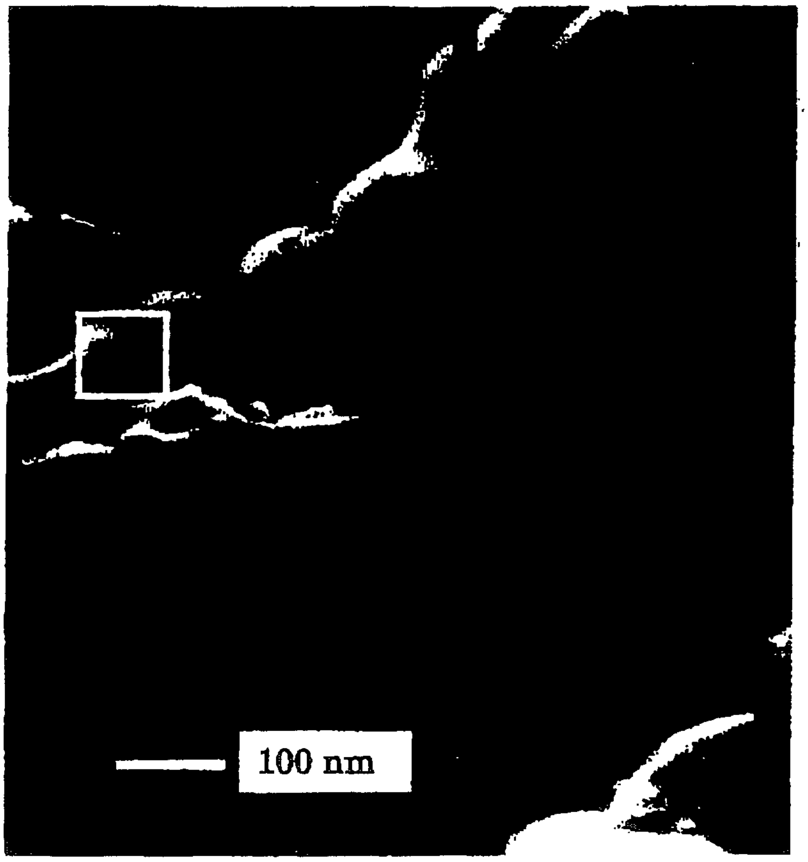


Figure 4

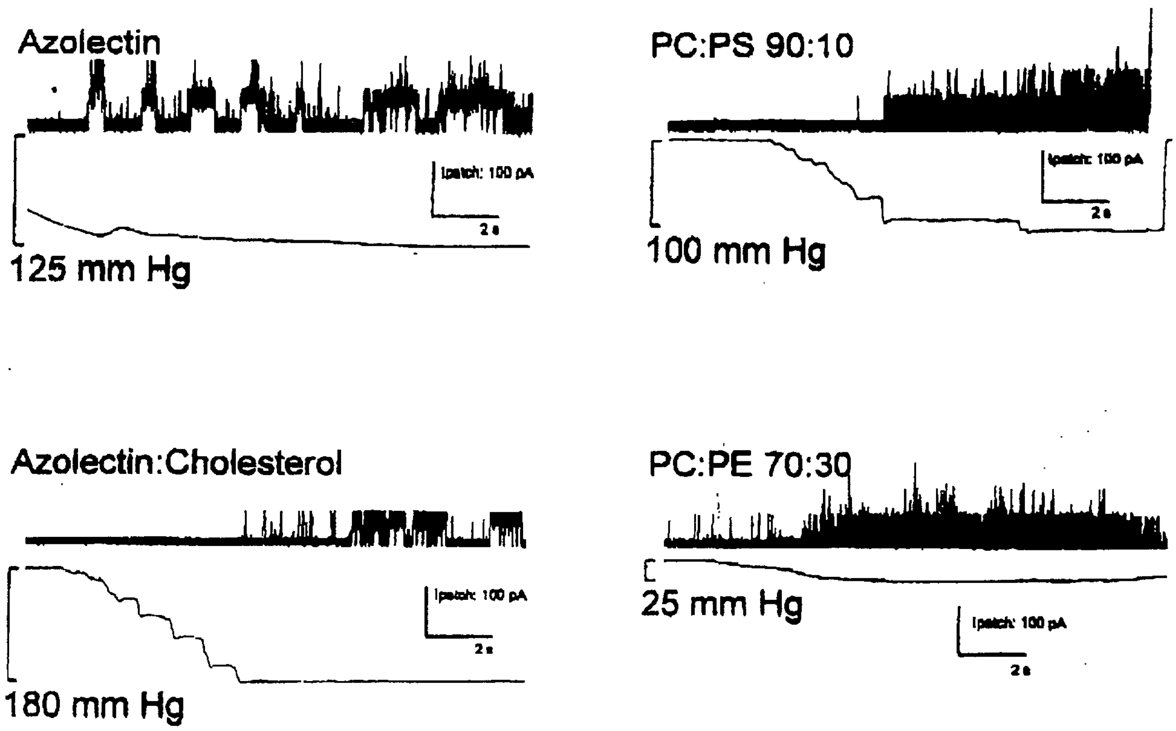
Azolectin
Ipatch: 100 pA
2 s
125 mm Hg
PC:PS 90:10
Ipatch: 100 pA
2 s
100 mm Hg
Azolectin:Cholesterol
Ipatch: 100 pA
2 s
180 mm Hg
PC:PE 70:30
25 mm Hg
Ipatch: 100 pA
2 s

Figure 5

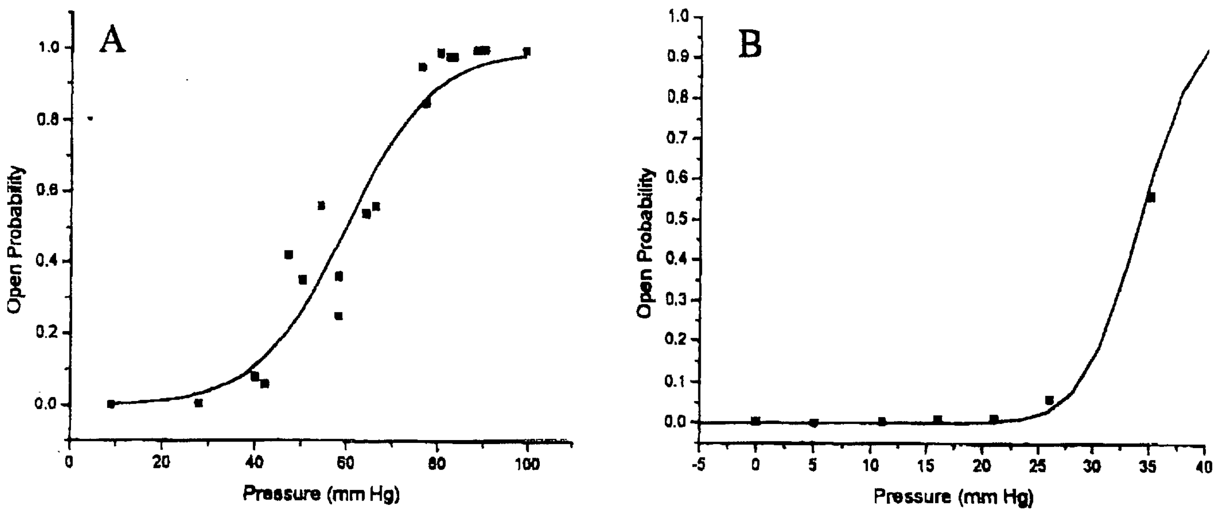
A
Open Probability
1.0
0.8
0.6
0.4
0.2
0.0
0
20
40
60
80
100
Pressure (mm Hg)
B
Open Probability
1.0
0.9
0.8
0.7
0.6
0.5
0.4
0.3
0.2
0.1
0.0
-5
0
5
10
15
20
25
30
35
40
Pressure (mm Hg)

Figure 6

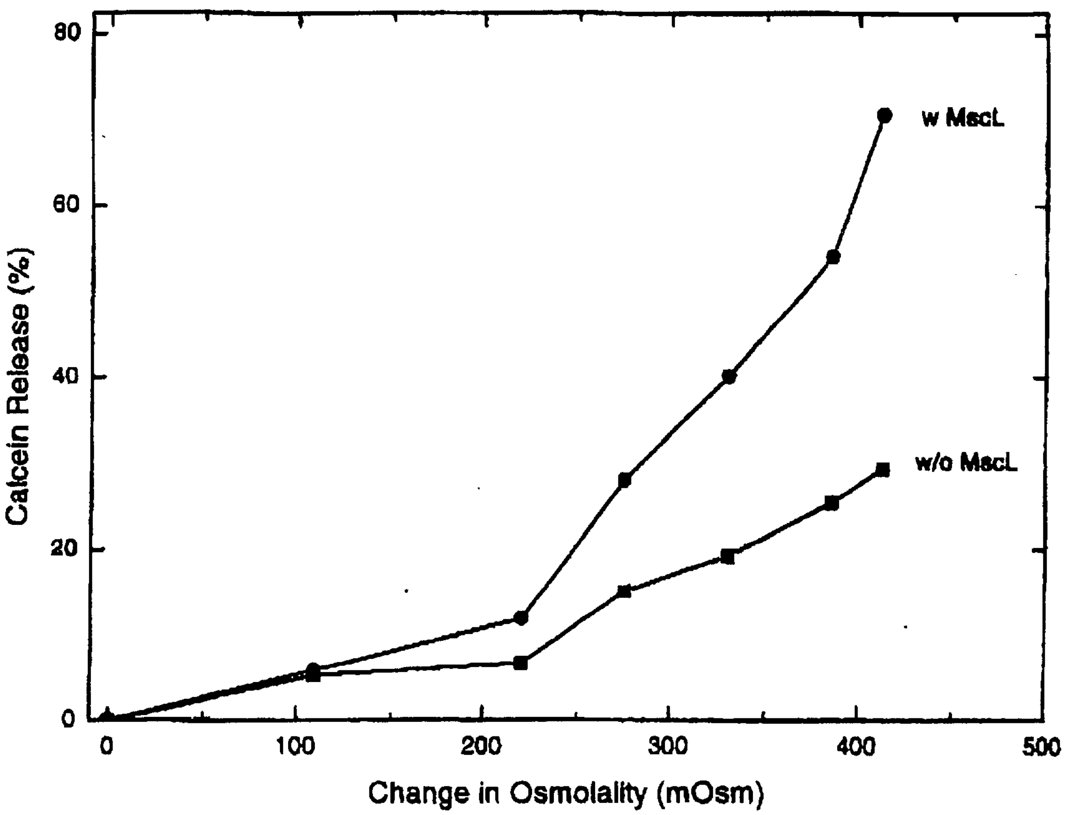
80
60
40
20
0
Calcein Release (%)
0
100
200
300
400
500
Change in Osmolality (mOsm)
w MscL
w/o MscL

Figure 7

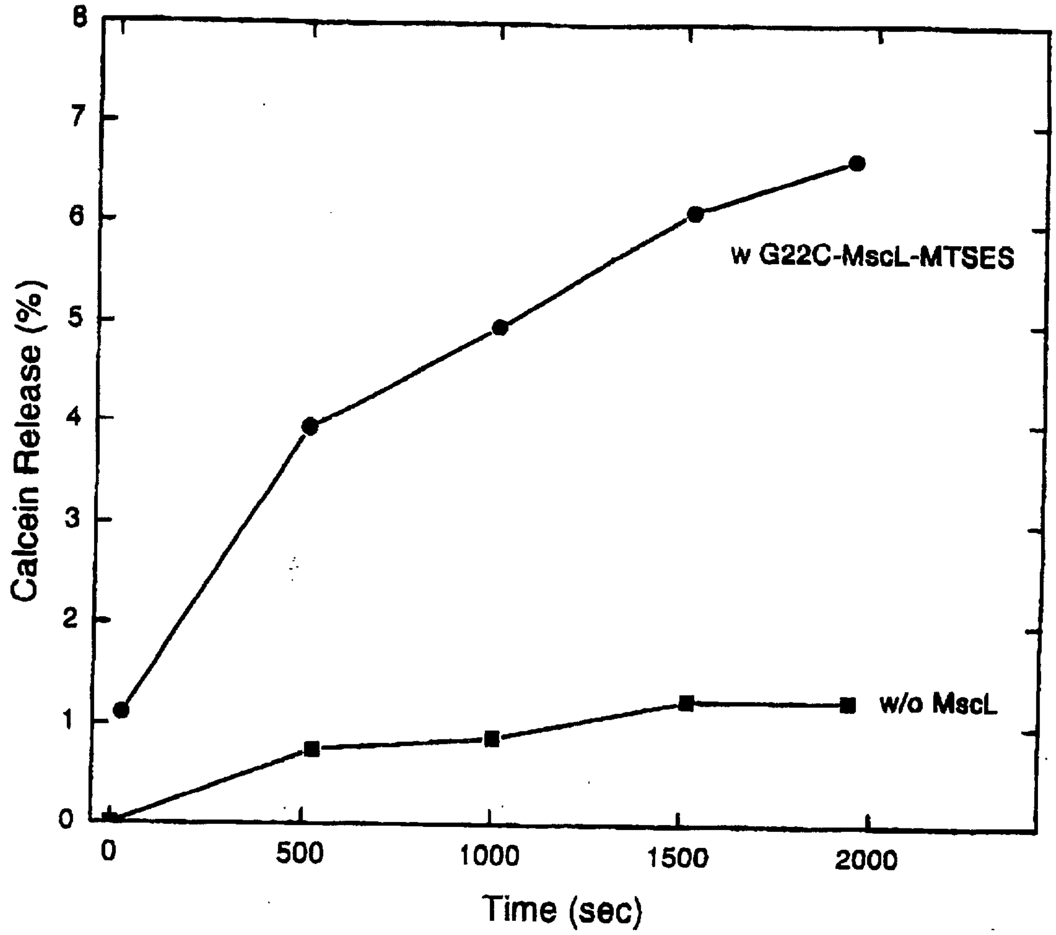
8
7
6
5
4
3
2
1
0
Calcein Release (%)
w G22C-MscL-MTSES
w/o MscL
0
500
1000
1500
2000
Time (sec)

Figure 8

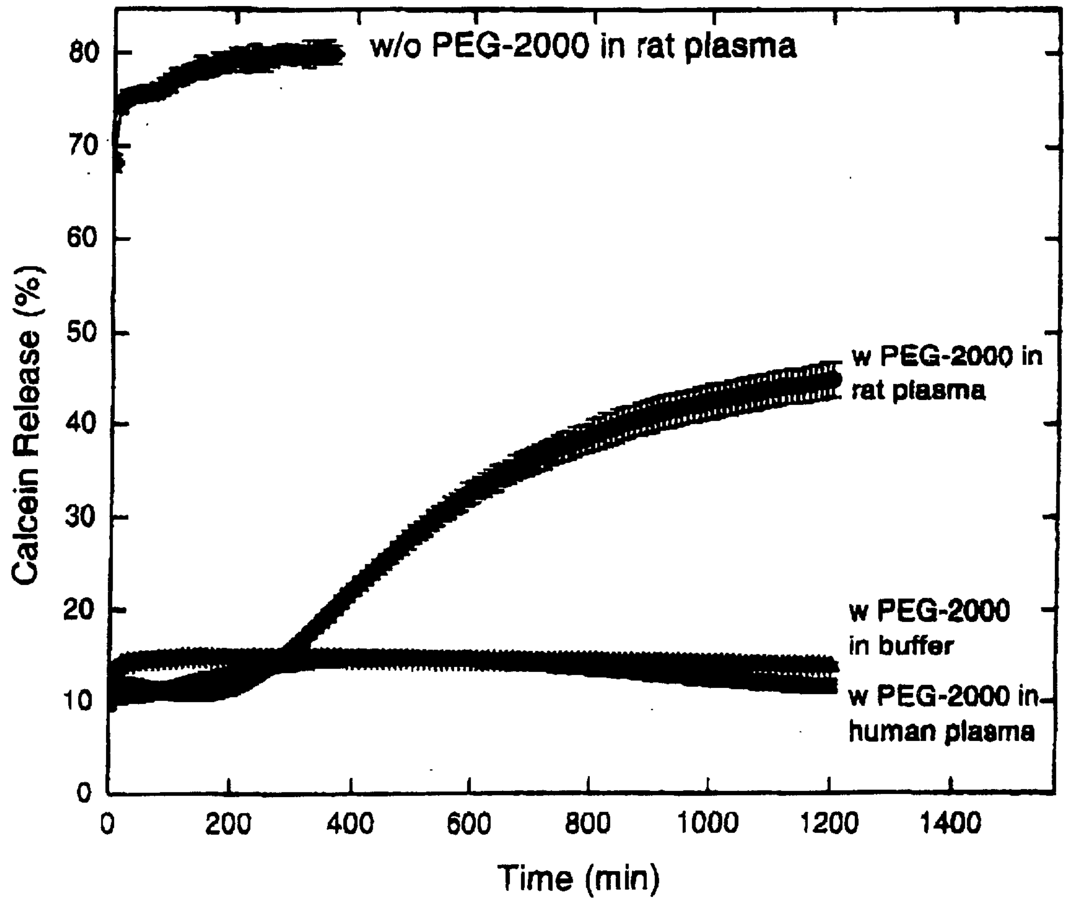
w/o PEG-2000 in rat plasma
w PEG-2000 in rat plasma
w PEG-2000 in buffer
w PEG-2000 in human plasma
Calcein Release (%)
0
10
20
30
40
50
60
70
80
Time (min)
0
200
400
600
800
1000
1200
1400

Figure 9

A

B

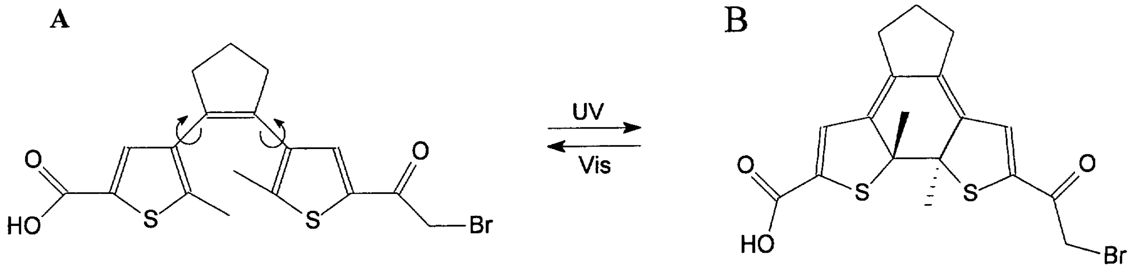


Figure 10

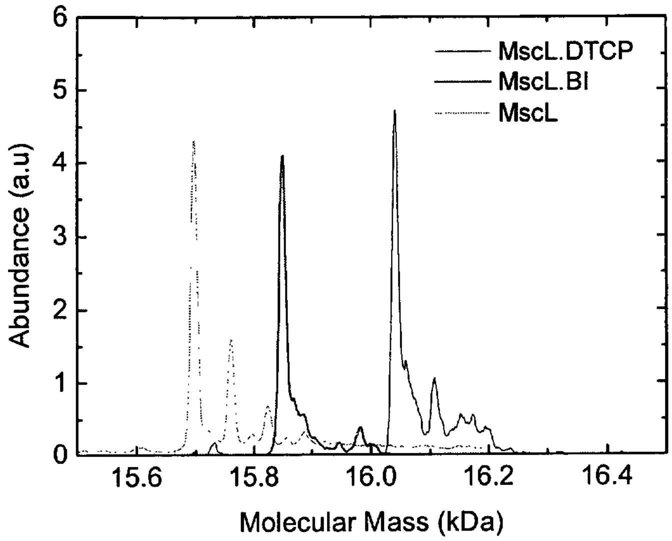
MscL.DTCP
MscL.BI
MscL
Abundance (a.u)
Molecular Mass (kDa)
0
1
2
3
4
5
6
15.6
15.8
16.0
16.2
16.4

Figure 11

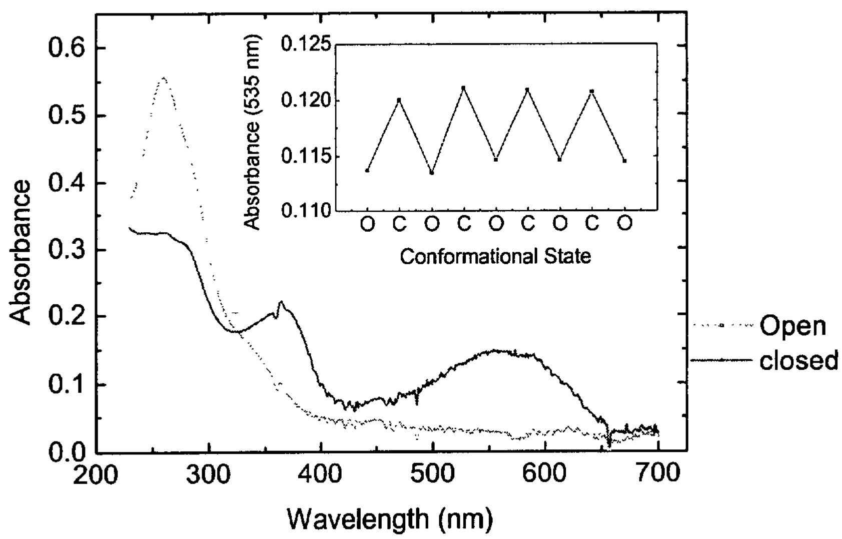
0.6
0.5
0.4
0.3
0.2
0.1
0.0
Absorbance
200
300
400
500
600
700
Wavelength (nm)
Absorbance (535 nm)
0.125
0.120
0.115
0.110
O C O C O C O C O
Conformational State
Open
closed

Figure 12

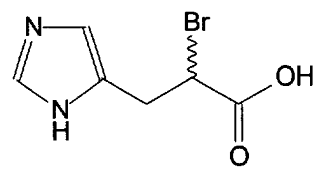

Figure 13

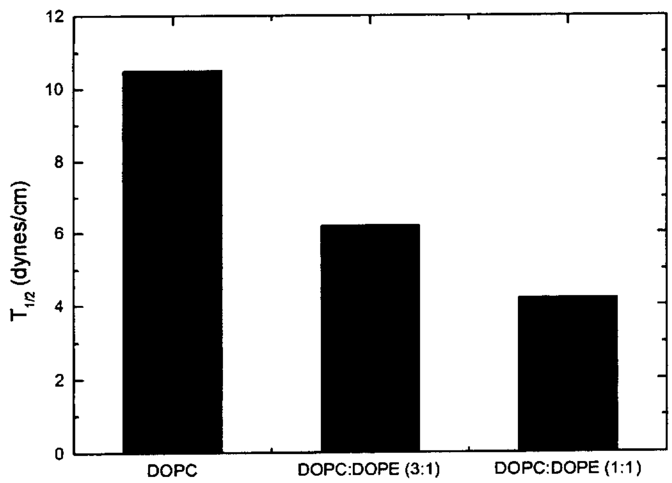


Figure 14

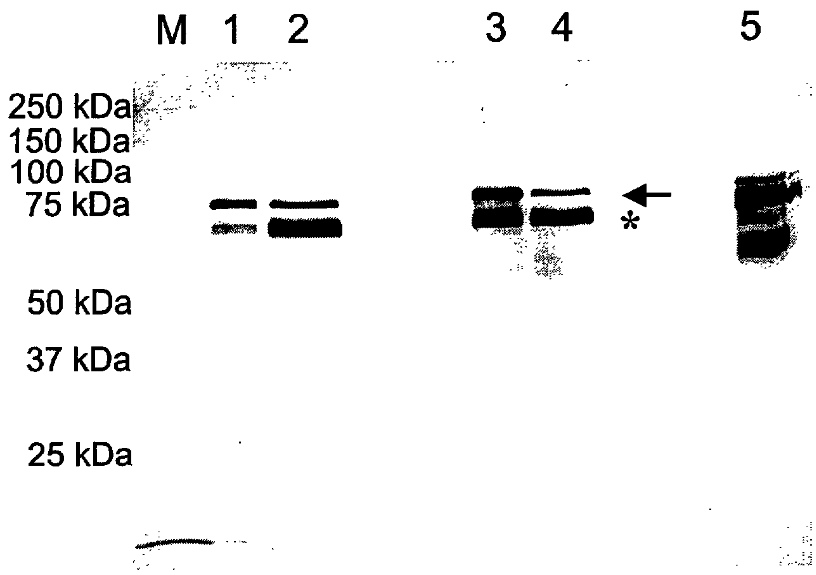


Figure 15

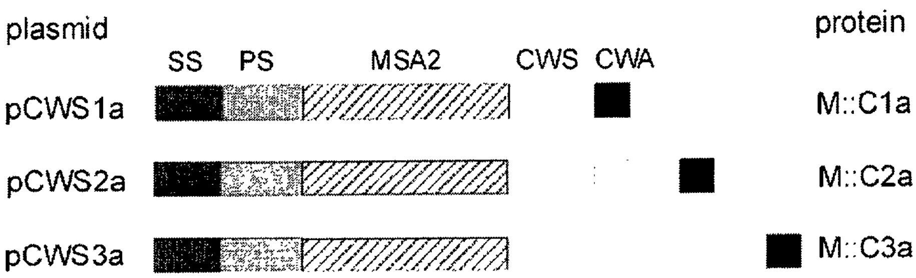
plasmid
protein
SS
PS
MSA2
CWS
CWA
pCWS1a
M::C1a
pCWS2a
M::C2a
pCWS3a
M::C3a

Figure 16

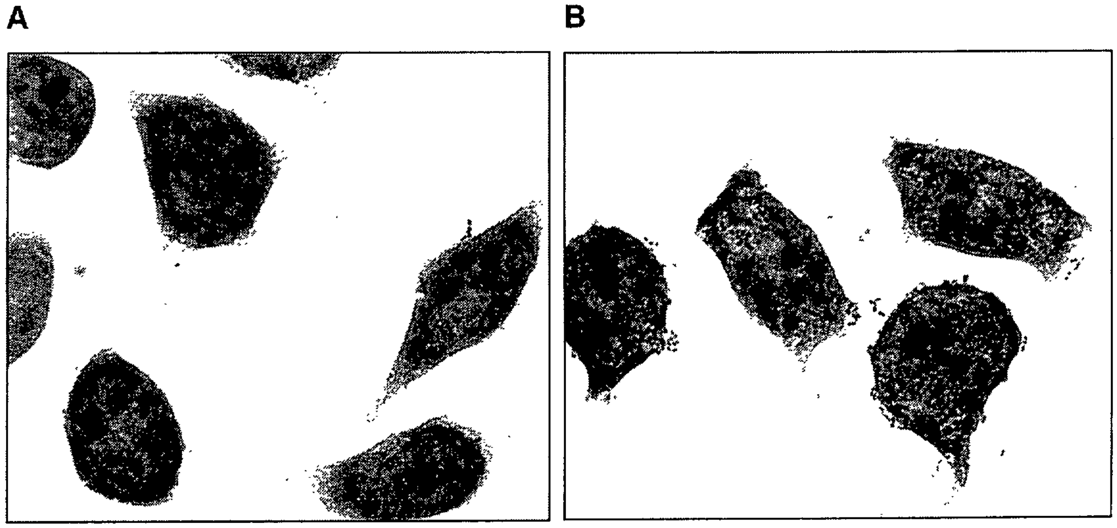


Figure 17

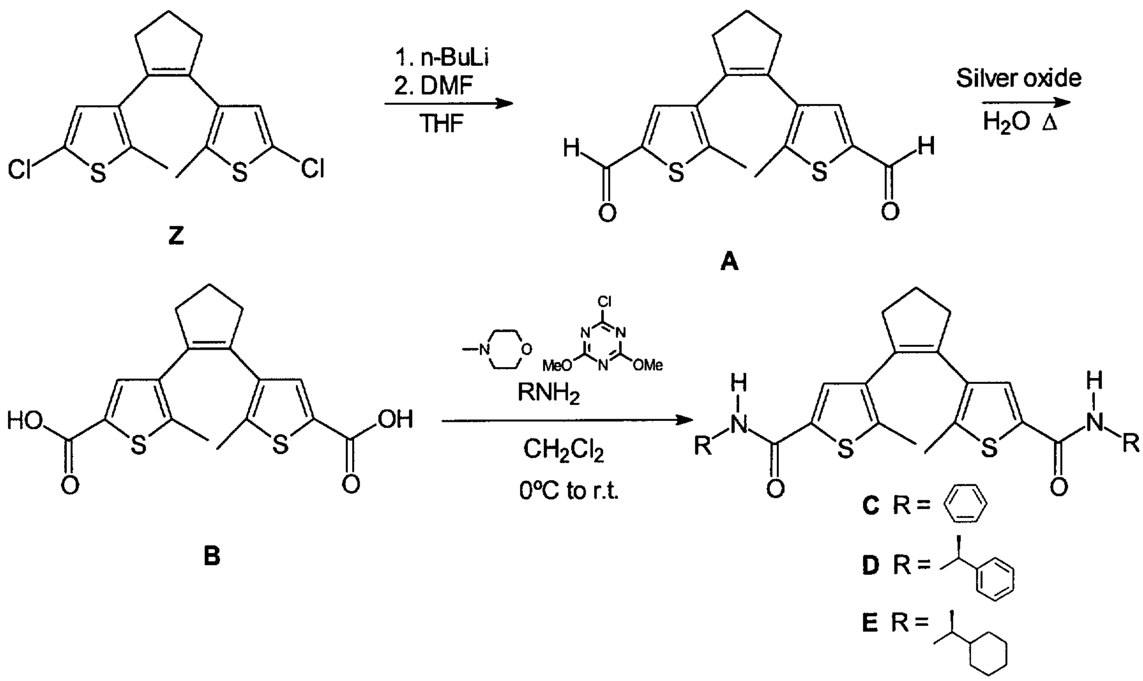
1. n-BuLi
2. DMF
THF
Z
A
Silver oxide
$H_2O$ Δ
B
$RNH_2$
$CH_2Cl_2$
0°C to r.t.
C R =
D R =
E R =

Figure 18

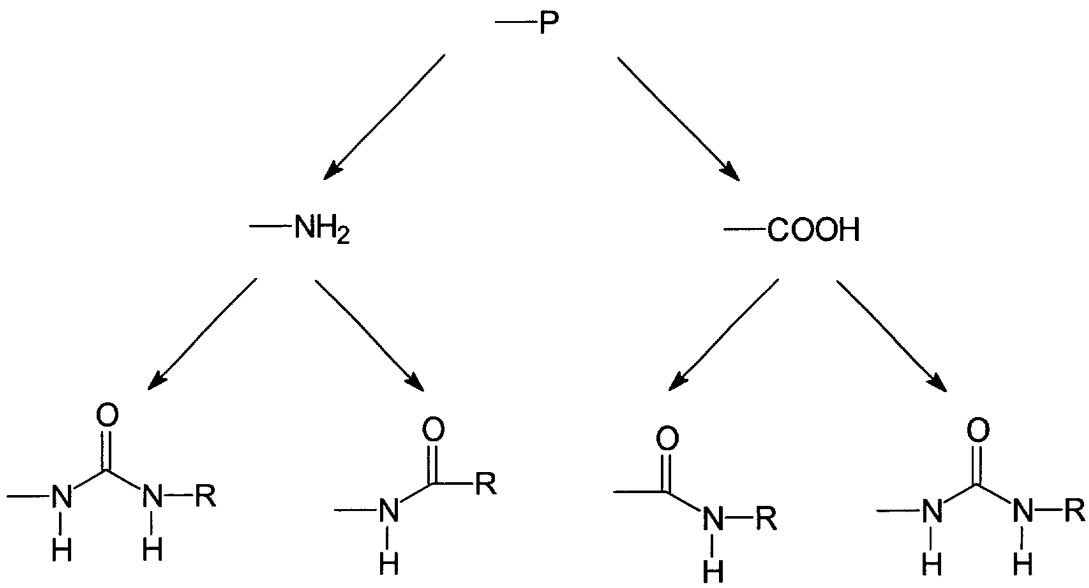

Figure 19

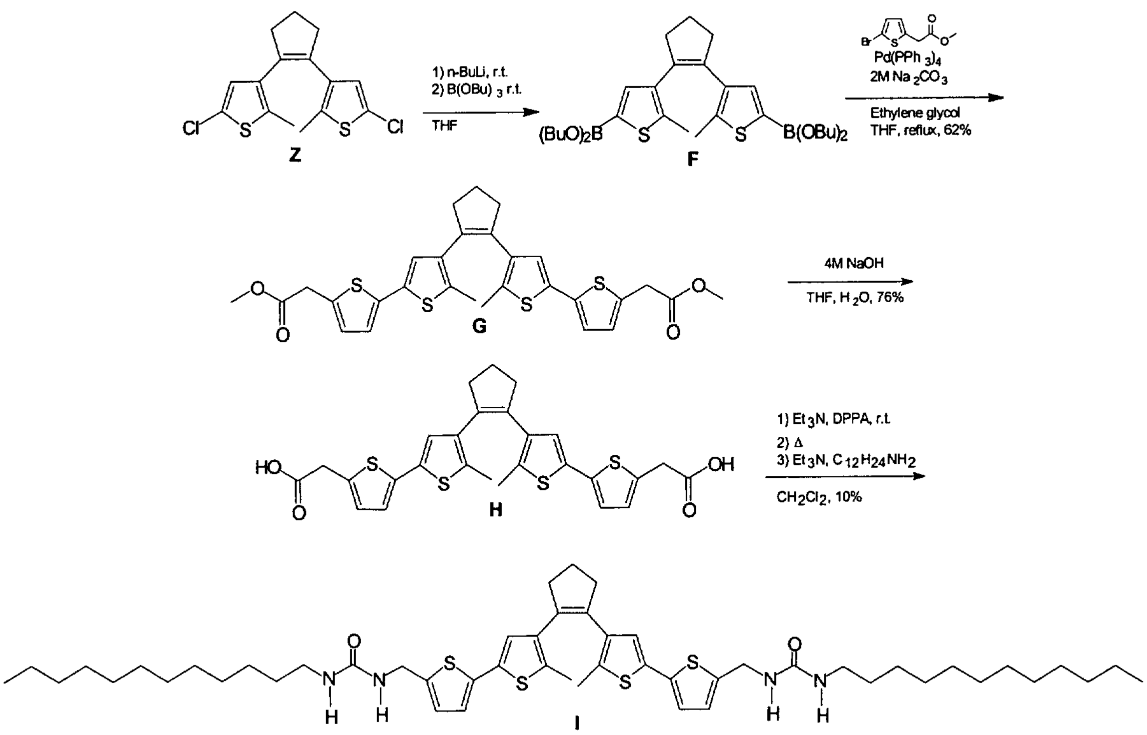


Z F G H I

Figure 20

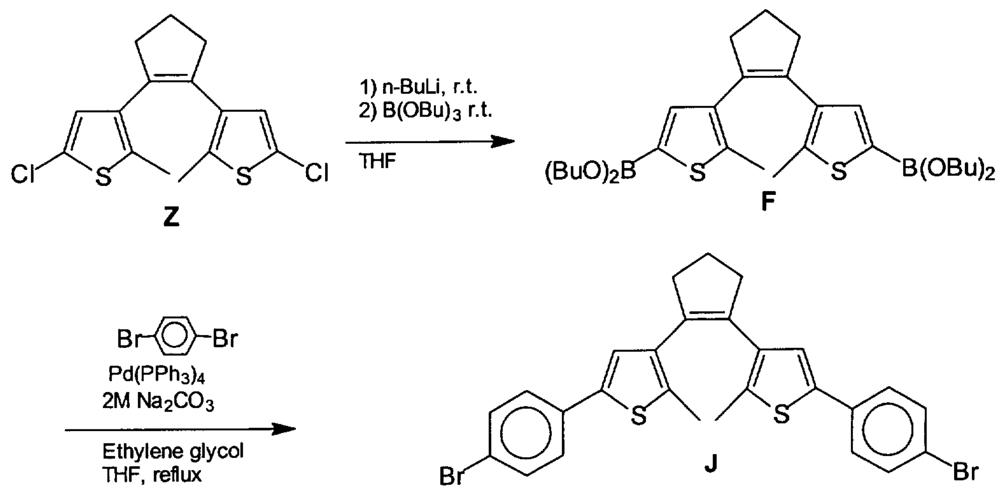
Z
1) n-BuLi, r.t.
2) B(OBu)3 r.t.
THF
F
Pd(PPh3)4
2M Na2CO3
Ethylene glycol
THF, reflux
J

Figure 21

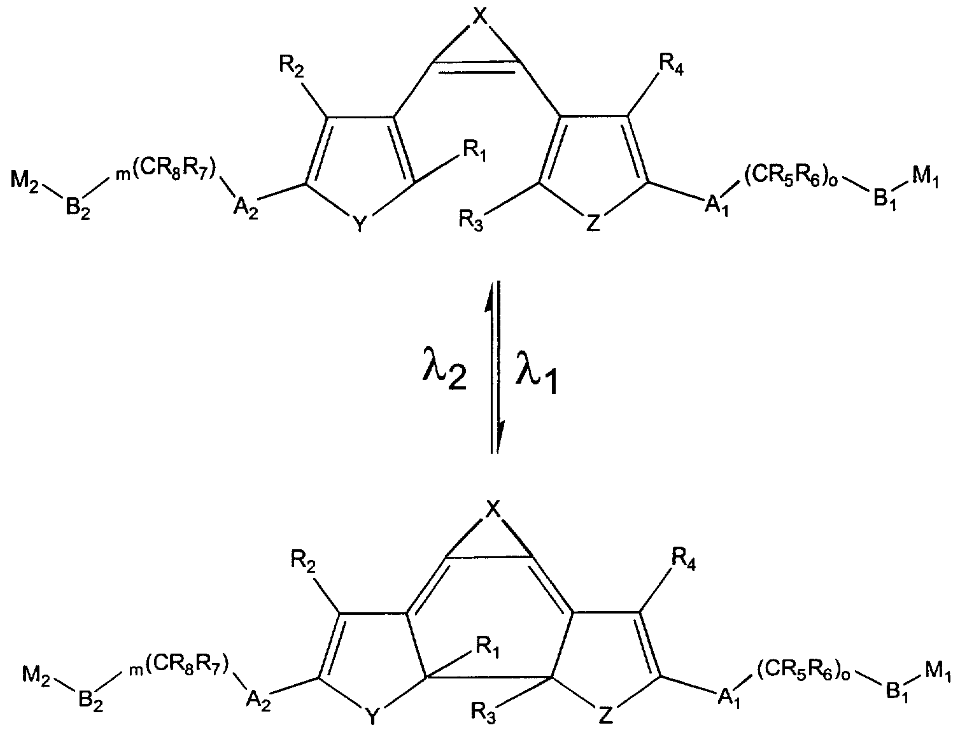

Figure 22

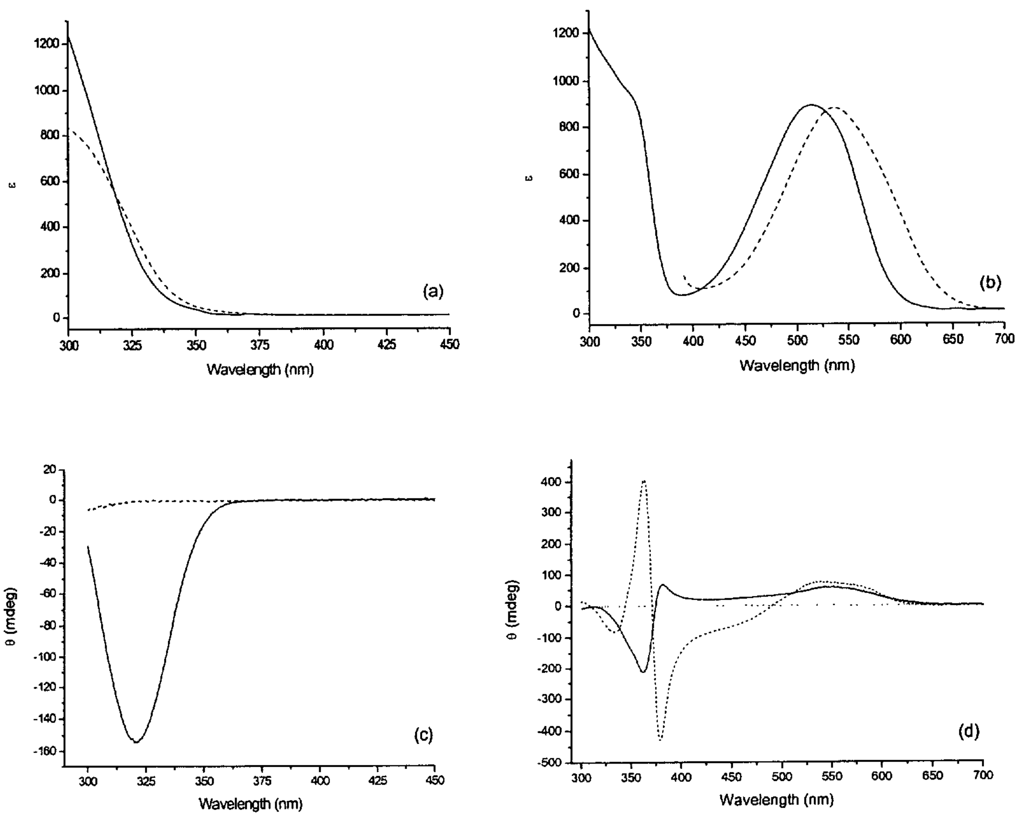


Figure 23

European Patent Office

# EUROPEAN SEARCH REPORT

Application Number
EP 02 07 6316

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | SIMÕES S ET AL: "Enhancement of cationic liposome-mediated gene delivery by transferrin and fusogenic peptides" PROCEEDINGS OF THE 24TH. INTERNATIONAL SYMPOSIUM ON CONTROLLED RELEASE OF BIOACTIVE MATERIALS, vol. SYMP. 24, 1997, pages 659-660, XP002098090 ISSN: 1022-0178<br>* the whole document *<br>--- | 1-6,23, 35,36, 38-41 | A61K9/127<br>A61K9/107<br>A61K9/50<br>A61K47/18<br>A61K47/22 |
| X | US 5 820 879 A (FERNANDEZ JULIO M ET AL) 13 October 1998 (1998-10-13)<br>* column 2, line 14 - line 38 *<br>* column 2, line 63 - line 67 *<br>* column 19, line 18 - column 21, line 53 *<br>* column 26, line 1 - column 27, line 20 *<br>* figures 13-15 *<br>--- | 1-6,23, 36-41 | |
| X | WO 00 42987 A (PANACEA BIOTEC LTD (IN)) 27 July 2000 (2000-07-27)<br>* page 4, paragraph 2 - page 5, paragraph 2 *<br>* page 8, paragraph 2 - paragraph 3 *<br>* page 12, paragraph 3 - page 13 *<br>* examples *<br>--- | 1-6,23, 36,38-41 | **TECHNICAL FIELDS SEARCHED (Int.Cl.7)**<br>A61K |
| X | WO 97 05899 A (UNIV GUELPH (CA)) 20 February 1997 (1997-02-20)<br>* page 4, line 16 - line 27 *<br>* example 5 *<br>--- | 1-6,23, 36,38-41 | |
| X | WO 00 18377 A (WARNER LAMBERT CO (US)) 6 April 2000 (2000-04-06)<br>* page 3, line 14 - page 4, line 3 *<br>* page 6, line 10 - line 19 *<br>* examples *<br>---<br>-/-- | 1-4,23, 36-41 | |

~~The present search report has been drawn up for all claims~~

| Place of search | Date of completion of the search | Examiner |
|---|---|---|
| THE HAGUE | 21 August 2002 | Epskamp, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 07 6316

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GUTOWSKA A ET AL: "Thermosensitive polymers for drug delivery" POLYMER PREPRINTS, AMERICAN CHEMICAL SOCIETY, vol. 37, no. 2, 1996, pages 115-116, XP000874751 ISSN: 0032-3934 * the whole document * --- | 1-4,23, 36-41 | |
| X | ANONYMOUS: "National refining photo album - tank trucks" INTERNET ARTICLE, [Online] 1 January 2002 (2002-01-01), XP002210455 Retrieved from the Internet: <URL:http://www.enarco.com/trucks.htm> [retrieved on 2002-08-19] * figures * --- | 1-4,36, 38,40,41 | |
| D,A | WO 99 25836 A (RIJKSUNIVERSITEIT GRONINGEN (NL)) 27 May 1999 (1999-05-27) * page 4, line 1 - line 29 * * page 9, line 22 - page 11, line 7 * ----- | 1-6 | TECHNICAL FIELDS SEARCHED (Int.Cl.7) |

~~The present search report has been drawn up for all claims~~

| Place of search | Date of completion of the search | Examiner |
|---|---|---|
| THE HAGUE | 21 August 2002 | Epskamp, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

**Application Number**
EP 02 07 6316

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

see annex

European Patent Office

**LACK OF UNITY OF INVENTION**
**SHEET B**

Application Number
EP 02 07 6316

The Search Division considers that the present European patent application does not comply with the requirements of unity of invention and relates to several inventions or groups of inventions, namely:

1. Claims: 1-3 (partially), 4-6, 23 (p.), 35-41 (p.)

   A delivery vehicle according to any one of claims 1-3, further comprising a targeting means for directing said vehicle to said predetermined site.

2. Claims: 1-3 (p.), 7-10, 11 (p.), 12-20, 23 (p.), 35-41 (p.)

   A delivery vehicle according to any one of claims 1-3, wherein said means for inducing availability of at least one compartment comprises a proteinaceous channel.

3. Claims: 1-3 (p.), 21, 23 (p.), 35-41 (p.)

   A delivery vehicle according to any one of claims 1-3, wherein said means for inducing availability of said at least one compartment toward the exterior comprises light.

4. Claims: 1-3 (p.), 22 (p.), 23 (p.), 35-41 (p.)

   A delivery vehicle according to any one of claims 1-3, wherein said means for inducing availability of said compartment toward the exterior comprises an pH.

5. Claims: 1-3 (p.), 22 (p.), 23 (p.), 35-41 (p.)

   A delivery vehicle according to any one of claims 1-3, wherein said means for inducing availability of said compartment toward the exterior comprises salt concentration.

6. Claims: 1-3 (p.), 22 (p.), 23 (p.), 35-41 (p.)

   A delivery vehicle according to any one of claims 1-3, wherein said means for inducing availability of said compartment toward the exterior comprises temperature.

7. Claims: 1-3 (p.), 23-25 (p.), 26, 27, 30 (p.), 31 (p.), 35-41 (p.)

   A delivery vehicle according to any one of claims 1-3, wherein vehicle comprises a gelator or a thickening agent, wherein said gelator and/or thickening agent comprises an organogel, a hydrogel or a combination thereof, comprising a

European Patent Office

**LACK OF UNITY OF INVENTION SHEET B**

Application Number
EP 02 07 6316

The Search Division considers that the present European patent application does not comply with the requirements of unity of invention and relates to several inventions or groups of inventions, namely:

N,N'-disubstituted aldaramides, a N,N'-disubstituted pentaramides or a derivative and/or combination thereof.

8. Claims: 1-3 (p.), 23-25 (p.), 28, 29, 30 (p.), 31 (p.), 35-41 (p.)

A delivery vehicle according to any one of claims 1-3, wherein vehicle comprises a gelator or a thickening agent, wherein said gelator and/or thickening agent comprises an organogel, a hydrogel or a combination thereof, wherein said gelling and or thickening agent comprises a light-switchable gelator.

9. Claims: 1-3 (p.), 11 (p.), 23 (p.), 32, 35-41 (p.)

A delivery vehicle according to any one of claims 1-3, wherein said vehicle comprises a film enclosing said at least one compartment, wherein said membrane comprises hydrophobin or a functional equivalent thereof.

10. Claims: 1-3 (p.), 11 (p.), 23 (p.), 33, 34, 35-41 (p.)

A delivery vehicle according to any one of claims 1-3, wherein said vehicle comprises a film enclosing said at least one compartment, wherein said vehicle comprises a cationic amphiphile.

## ANNEX TO THE EUROPEAN SEARCH REPORT ON EUROPEAN PATENT APPLICATION NO. EP 02 07 6316

This annex lists the patent family members relating to the patent documents cited in the above-mentioned European search report.
The members are as contained in the European Patent Office EDP file on
The European Patent Office is in no way liable for these particulars which are merely given for the purpose of information.

21-08-2002

| Patent document cited in search report | Publication date | Patent family member(s) | Publication date |
|---|---|---|---|
| US 5820879 A | 13-10-1998 | US 5654006 A | 05-08-1997 |
| | | US 5811124 A | 22-09-1998 |
| | | AT 162397 T | 15-02-1998 |
| | | AU 676543 B | 13-03-1997 |
| | | AU 6247994 A | 29-08-1994 |
| | | CA 2155648 A | 18-08-1994 |
| | | DE 69408122 D | 26-02-1998 |
| | | DE 69408122 T | 03-09-1998 |
| | | DK 684812 T | 21-09-1998 |
| | | EP 0684812 A | 06-12-1995 |
| | | ES 2114184 T | 16-05-1998 |
| | | JP 8509956 T | 22-10-1996 |
| | | WO 9417786 A | 18-08-1994 |
| | | US 5753261 A | 19-05-1998 |
| WO 0042987 A | 27-07-2000 | AU 3322100 A | 07-08-2000 |
| | | BR 0004813 A | 21-11-2000 |
| | | EP 1105097 A | 13-06-2001 |
| | | JP 2002535266 T | 22-10-2002 |
| | | US 6306838 B | 23-10-2001 |
| WO 9705899 A | 20-02-1997 | AU 707131 B | 01-07-1999 |
| | | AU 6609596 A | 05-03-1997 |
| | | BR 9609882 A | 27-07-1999 |
| | | CA 2182637 A | 05-02-1997 |
| | | EP 0841944 A | 20-05-1998 |
| WO 0018377 A | 06-04-2000 | AU 6261399 A | 17-04-2000 |
| | | CA 2344680 A | 06-04-2000 |
| | | CN 1321085 T | 07-11-2001 |
| | | EP 1117386 A | 25-07-2001 |
| | | JP 2002525314 T | 13-08-2002 |
| | | US 2001036473 A | 01-11-2001 |
| WO 9925836 A | 27-05-1999 | EP 0916726 A | 19-05-1999 |
| | | AU 752666 B | 26-09-2002 |
| | | AU 1179999 A | 07-06-1999 |
| | | CA 2310228 A | 27-05-1999 |
| | | EP 1032675 A | 06-09-2000 |
| | | JP 2002514381 T | 21-05-2002 |

EPO FORM P0459

For more details about this annex : see Official Journal of the European Patent Office, No. 12/82